# EUROPEAN PATENT APPLICATION

(11) **EP 1 987 827 A1**
(43) Date of publication of application: **05.11.2008**
(21) Application number: 07714510.0
(22) Date of filing: 19.02.2007
(51) Int. Cl.: A61K 31/40, A61K 31/045, A61K 31/275, A61K 31/277, A61K 31/336, A61K 31/401, A61K 31/4015, A61K 31/402, A61K 31/4025, A61K 31/4035, A61K 31/4152, A61K 31/42, A61K 31/438, A61K 31/445, A61K 31/4453, A61K 31/45, A61K 31/4535, A61K 31/472, A61K 31/495, A61K 31/535, A61K 31/5375

(54) **NOVEL PHARMACEUTICAL**

(30) Priority: 20.02.2006 JP 2006043141
(71) Applicant: Takeda Pharmaceutical Company Limited, Osaka-shi, Osaka 541-0045 (JP)
(72) Inventor: YAMAOKA, Masuo, Tsukuba-shi, Ibaraki 300-4293 (JP); HARA, Takahito, Ibaraki, 300-4293 (JP); YAMAMOTO, Satoshi, Osaka-shi, Osaka 532-8686 (JP)
(74) Representative: Weber, Thomas
(86) International application number: PCT/JP2007/052984
(87) International publication number: WO 2007/097289

(57) **Abstract**

A tissue-selective androgen receptor modulator containing a compound represented by the formula 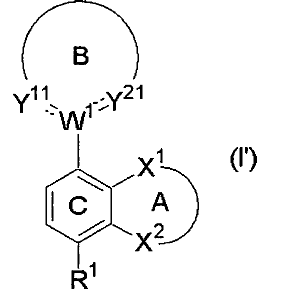 wherein Ring A represents an optionally substituted 5- to 8-membered ring, Ring B represents an optionally further substituted 4- to 10-membered ring, Ring C represents an optionally further substituted benzene ring, X¹ represents an optionally substituted carbon atom, X² represents an optionally substituted carbon atom, an oxygen atom and the like, W¹ represents a nitrogen atom and the like, Y¹¹ represents a group represented by the formula CR²R^{3'} (wherein R² represents a hydrogen atom, a cyano group, a nitro group and the like, and R^{3'} represents a bond, a hydrogen atom, a cyano group, a nitro group and the like, respectively), Y²¹ represents a group represented by the formula CR⁴R^{5'} (wherein R⁴ represents a hydrogen atom, a cyano group, a nitro group and the like, and R^{5'} represents a bond, a hydrogen atom, a cyano group, a nitro group and the like, respectively) and the like, R¹ represents an electron-withdrawing group, and the formula
-̅ -̅ -̅
represents a single bond or a double bond, or a salt thereof or a prodrug thereof.

## Description

### Technical Field

The present invention relates to a tissue-specific androgen receptor modulator containing a fused benzene derivative and the like.

### Background Art

Androgens are synthesized in the testis and the adrenal cortex, bind to an androgen receptor at the target organ, and exert various physiological activities. Natural androgens all belong to C19 steroid chemically. The most major androgen among them is testosterone, which is mainly synthesized at testis, taken up by target cells and has more potent physiological activity. For females, the adrenal cortex is a major source for androgens.
Androgens have actions of developing and maintaining the functions of reproductive organs (prostate, seminal vesicle, epididymis, vas deferens, etc.), sexual differentiation at fetal stage, formation of sperm, expression of secondary sexual characteristics (induction of masculinization for muscle/backbone, voice, fat distribution, etc.), promoting protein anabolism at muscle, etc., and actions for bone metabolism, etc. Therefore, insufficiency of androgen such as androgen deficiency by testis function disorders and castration, etc. is linked to various pathological state and decrease of QOL (quality of life). For this, androgens replacement therapy is usually carried out. In addition to testosterone, synthetic androgens having different balance of androgenic actions have been investigated, and applied in clinical practice.
On the other hand, in the case that androgens are associated with the progression of diseases, androgen deprivation therapy is carried out. For example, for androgen-dependent prostate cancer, testosterone level is lowered by surgical or medical castration or GnRH agonist administration, to achieve therapeutic effect.
Patent reference 1 (W02004/16576) describes that the compound represented by the formula:

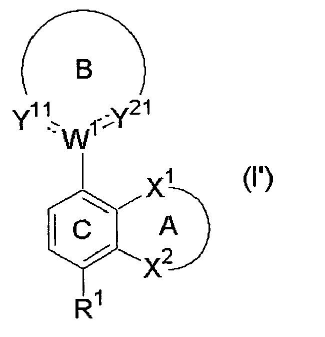

wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), W¹ represents a nitrogen atom, or a group represented by the formula CR^{a} (wherein R^{a} represents a bond, a hydrogen atom, a hydroxy group or an optionally substituted alkoxy group), Y¹¹ represents a group represented by the formula cR²R^{3'} (wherein R² represents a hydrogen atom, a cyano group, a nitro group, an optionally substituted acyl group, an optionally esterified or amidated carboxyl group or an optionally substituted hydrocarbon group, and R^{3'} represents a bond, a hydrogen atom, a cyano group, a nitro group, an optionally substituted acyl group, an optionally esterified or amidated carboxyl group or an optionally substituted hydrocarbon group), and Y²¹ represents a group represented by the formula CR⁴R^{5'} (wherein R⁴ represents a hydrogen atom, a cyano group, a nitro group, an optionally substituted acyl group, an optionally esterified or amidated carboxyl group or an optionally substituted hydrocarbon group, and R^{5'} represents a bond, a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and when Ring B is a bicyclic ring optionally further substituted, CR² for Y¹¹ or CR⁴ or the nitrogen atom for Y²¹ may constitute a part of Ring B, R¹ represents an electron-withdrawing group, and the formula

-̅ -̅ -̅

represents a single bond or a double bond, or a salt thereof, or a prodrug thereof has a superior androgen receptor modulator action, and is useful as an agent for the prophylaxis or treatment of, for example, hypogonadism, osteoporosis, hormone refractory cancer, climacteric disorder, anemia, arteriosclerosis, Alzheimer's disease, erectile dysfunction, depression, wasting disease and the like.
patent reference 1: WO2004/16576

### Disclosure of the Invention

### Problems to be Solved by the Invention

The present invention aims to provide a novel use of the compound described in W02004/16576, which has a superior androgen receptor regulating action.

### Means of Solving the Problems

The present inventors have conducted intensive studies in view of the above-mentioned problems, and found that a compound represented by the formula (I') unexpectedly has an organ selective androgen receptor regulating action, and that the compound is applicable to diseases and the like, for which it has not been applied heretofore, which resulted in the completion of the present invention.
Accordingly, the present invention provides
[1] a tissue-selective androgen receptor modulator comprising a compound represented by the formula

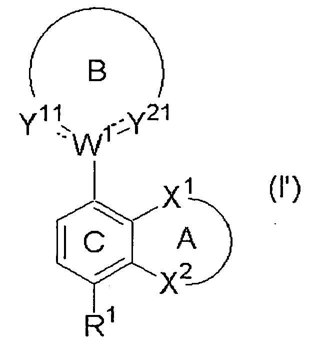

wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), W¹ represents a nitrogen atom, or a group represented by the formula CR^{a} (wherein R^{a} represents a bond, a hydrogen atom, a hydroxy group or an optionally substituted alkoxy group), Y¹¹ represents a group represented by the formula CR²R^{3'} (wherein R² represents a hydrogen atom, a cyano group, a nitro group, an optionally substituted acyl group, an optionally esterified or amidated carboxyl group or an optionally substituted hydrocarbon group, and R^{3'} represents a bond, a hydrogen atom, a cyano group, a nitro group, an optionally substituted acyl group, an optionally esterified or amidated carboxyl group or an optionally substituted hydrocarbon group), and Y²¹ represents a group represented by the formula CR⁴R^{5'} (wherein R⁴ represents a hydrogen atom, a cyano group, a nitro group, an optionally substituted acyl group, an optionally esterified or amidated carboxyl group or an optionally substituted hydrocarbon group, and R^{5'} represents a bond, a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and when Ring B is a bicyclic ring optionally further substituted, CR² for Y¹¹ or CR⁴ or the nitrogen atom for Y²¹ may constitute a part of Ring B, R¹ represents an electron-withdrawing group, and the formula

-̅ -̅ -̅

represents a single bond or a double bond, or a salt thereof, or a prodrug thereof;
[2] the modulator of the above-mentioned [1], which increases prostate weight by not more than about 10% (preferably not more than 0%) with a dose that increases levator ani muscle weight by about 20% (about 20% - about 50%);
[3] the modulator of the above-mentioned [1], which is a frailty suppressant, a muscular strength enhancer, a muscle increasing agent, a cachexia suppressant, a body weight decrease suppressant, an agent for the prophylaxis or treatment of prostate hypertrophy, an agent for the prophylaxis or treatment of amyotrophy, an agent for the prophylaxis or treatment of sarcopenia caused by a disease, an agent for reducing prostate weight, an agent for the prophylaxis or treatment of hypertriglyceridemia (hyperlipidemia), a cholesterol-lowering agent or an agent for the prophylaxis or treatment of metabolic syndrome;
[4] a method of tissue-selective modulation of androgen receptor, which comprises administering, to a mammal, an effective amount of a compound represented by the formula

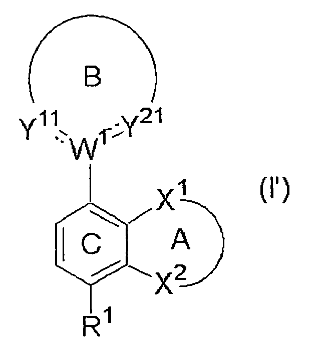

wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), W¹ represents a nitrogen atom, or a group represented by the formula CR^{a} (wherein R^{a} represents a bond, a hydrogen atom, a hydroxy group or an optionally substituted alkoxy group), Y¹¹ represents a group represented by the formula CR²R^{3'} (wherein R² represents a hydrogen atom, a cyano group, a nitro group, an optionally substituted acyl group, an optionally esterified or amidated carboxyl group or an optionally substituted hydrocarbon group, and R^{3'} represents a bond, a hydrogen atom, a cyano group, a nitro group, an optionally substituted acyl group, an optionally esterified or amidated carboxyl group or an optionally substituted hydrocarbon group), and Y²¹ represents a group represented by the formula CR⁴R^{5'} (wherein R⁴ represents a hydrogen atom, a cyano group, a nitro group, an optionally substituted acyl group, an optionally esterified or amidated carboxyl group or an optionally substituted hydrocarbon group, and R^{5'} represents a bond, a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and when Ring B is a bicyclic ring optionally further substituted, CR² for Y¹¹ or CR⁴ or the nitrogen atom for Y²¹ may constitute a part of Ring B, R¹ represents an electron-withdrawing group, and the formula

-̅ -̅ -̅

represents a single bond or a double bond, or a salt thereof, or a prodrug thereof;
[5] use of a compound represented by the formula

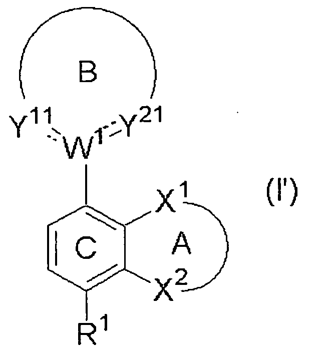

wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), W¹ represents a nitrogen atom, or a group represented by the formula CR^{a} (wherein R^{a} represents a bond, a hydrogen atom, a hydroxy group or an optionally substituted alkoxy group), Y¹¹ represents a group represented by the formula CR²R^{3'} (wherein R² represents a hydrogen atom, a cyano group, a nitro group, an optionally substituted acyl group, an optionally esterified or amidated carboxyl group or an optionally substituted hydrocarbon group, and R^{3'} represents a bond, a hydrogen atom, a cyano group, a nitro group, an optionally substituted acyl group, an optionally esterified or amidated carboxyl group or an optionally substituted hydrocarbon group), and Y²¹ represents a group represented by the formula CR⁴R^{5'} (wherein R⁴ represents a hydrogen atom, a cyano group, a nitro group, an optionally substituted acyl group, an optionally esterified or amidated carboxyl group or an optionally substituted hydrocarbon group, and R^{5'} represents a bond, a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and when Ring B is a bicyclic ring optionally further substituted, CR² for Y¹¹ or CR⁴ or the nitrogen atom for Y²¹ may constitute a part of Ring B, R¹ represents an electron-withdrawing group, the formula

-̅ -̅ -̅

represents a single bond or a double bond, or a salt thereof, or a prodrug thereof for production of a tissue-selective androgen receptor modulator.

Furthermore, the present invention provides
[6] the modulator of the aforementioned [1], wherein compound (I') is a compound represented by the formula

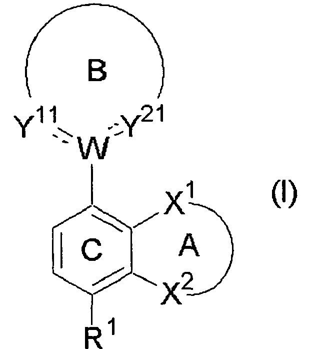

wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), W represents a nitrogen atom, or when Ring A is an optionally substituted benzene ring, a group represented by the formula CR^{a} (wherein R^{a} represents a bond, a hydrogen atom, a hydroxy group or an optionally substituted alkoxy group). Y¹¹ represents a group represented by the formula CR²R^{3'} (wherein R² represents a hydrogen atom, a cyano group, a nitro group, an optionally substituted acyl group, an optionally esterified or amidated carboxyl group or an optionally substituted hydrocarbon group, and R^{3'} represents a bond, a hydrogen atom, a cyano group, a nitro group, an optionally substituted acyl group, an optionally esterified or amidated carboxyl group or an optionally substituted hydrocarbon group), and Y²¹ represents 1) when W is a nitrogen atom, a group represented by the formula CR⁴R^{5'} (wherein R⁴ represents a hydrogen atom, a cyano group, a nitro group, an optionally substituted acyl group, an optionally esterified or amidated carboxyl group or an optionally substituted hydrocarbon group, and R^{5'} represents a bond, a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and 2) when W is a group represented by the formula CR^{a} (wherein the symbol is as defined above), a group represented by the formula CR⁴R^{5'} (wherein each symbol is as defined above) or a nitrogen atom (provided that when Y²¹ is a nitrogen atom and W is a group represented by the formula CR^{a} (wherein the symbol is as defined above), the bond between CR^{a} and Y²¹ is a double bond), and when Ring B is a bicyclic ring optionally further substituted, CR² for Y¹¹ or CR⁴ or the nitrogen atom for Y²¹ may constitute a part of Ring B, R¹ represents an electron-withdrawing group, and the formula

-̅ -̅ -̅

represents a single bond or a double bond, provided that
1) a compound wherein W is a nitrogen atom and Ring B is an optionally substituted piperazine ring,
2) a compound wherein Ring A is an optionally substituted benzene ring, R¹ is a nitro group or an optionally substituted sulfamoyl group, W is a nitrogen atom, Ring B is an octahydro[1,2-a]pyrazine ring, a homopiperazine ring in which the nitrogen atom is optionally substituted with an alkyl group or a 2,5-diazabicyclo[2,2,1]heptane ring in which the nitrogen atom is optionally substituted with an alkyl group,
3) a compound wherein Ring A is an optionally substituted, optionally saturated furan ring or pyran ring, R¹ is a halogen atom, W is a nitrogen atom, and Ring B is a pyrrolidine ring substituted with an optionally substituted amino group at the position 3,
4) a compound wherein W is a group represented by the formula CR^{a} (wherein the symbol is as defined above), and Ring B is an optionally substituted piperidine ring bonded to Ring C at the 4-position or an optionally substituted 1,2,5,6-tetrahydropyridine ring bonded to Ring C at the 4-position, and 5) 1-[4-(1-piperidinyl)-1-naphthyl]ethanone, 4-(1-piperidinyl)-1-nitronaphthalene, 4-(1-piperidinyl)-1-naphthonitrile and 4-(1-pyrrolidinyl)-1-nitronaphthalene, are excluded;
   [7] the modulator of the aforementioned [1], wherein compound (I') is a compound represented by the formula

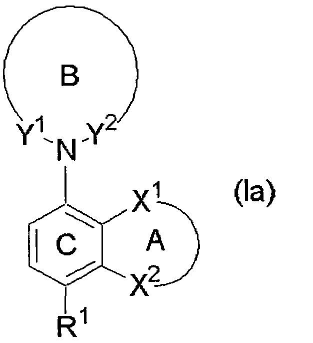

wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), Y¹ represents a group represented by the formula CR²R³ (wherein R² and R³ are the same or different and each represents a hydrogen atom, a cyano group, a nitro group, an optionally substituted acyl group, an optionally esterified or amidated carboxyl group or an optionally substituted hydrocarbon group), and Y² represents a group represented by the formula CR⁴R⁵ (wherein R⁴ and R⁵ are the same or different and each represents a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and when Ring B is a bicyclic ring optionally further substituted, CR² for Y¹ or CR⁴ or the nitrogen atom for Y² may constitute a part of Ring B, and R¹ represents an electron-withdrawing group, provided that
1) a compound wherein Ring B is an optionally substituted piperazine ring,
2) a compound wherein Ring A is an optionally substituted benzene ring, R¹ is a nitro group or an optionally substituted sulfamoyl group, Ring B is an octahydro[1,2-a]pyrazine ring, a homopiperazine ring in which the nitrogen atom is optionally substituted with an alkyl group or a 2,5-diazabicyclo[2,2,1]heptane ring in which the nitrogen atom is optionally substituted with an alkyl group,
3) a compound wherein Ring A is an optionally substituted, optionally saturated furan ring or pyran ring, R¹ is a halogen atom, and Ring B is a pyrrolidine ring substituted with an optionally substituted amino group at the 3-position, and
4) 1-[4-(1-piperidinyl)-1-naphthyl]ethanone, 4-(1-piperidinyl)-1-nitronaphthalene, 4-(1-piperidinyl)-1-naphthonitrile and 4-(1-pyrrolidinyl)-1-nitronaphthalene, are excluded;
   [8] the modulator of the aforementioned [6], wherein Ring A is an optionally substituted benzene ring, an optionally substituted thiophene ring or an optionally substituted furan ring;
   [9] the modulator of the aforementioned [6], wherein Ring B is an optionally substituted pyrrolidine ring, an optionally substituted piperidine ring, an optionally substituted morpholine ring, an optionally substituted thiomorpholine ring, an optionally substituted pyrazoline ring, an optionally substituted pyrazolidine ring, an optionally substituted isoxazoline ring, an optionally substituted cyclopentane ring, an optionally substituted cyclopentene ring or an optionally substituted perhydroazepine ring;
   [10] the modulator of the aforementioned [6], wherein R¹ is a cyano group, a nitro group, a halogen atom, an optionally substituted acyl group, an optionally esterified or amidated carboxyl group or a C₁₋₆ alkyl group substituted with 1 to 5 halogen atoms;
   [11] the modulator of the aforementioned [6], wherein the substituent on Ring A or Ring B except for R^{a}, R², R^{3'}, R⁴ and R^{5'} is 1 to 6 groups selected from the group consisting of (1) a hydrogen atom, (2) a halogen atom, (3) a cyano group, (4) a nitro group, (5) a hydroxy group, (6) an optionally substituted amino group, (7) an optionally esterified or amidated carboxyl group, (8) an optionally substituted C₁₋₆ alkyl group, (9) an optionally substituted C₁₋₆ acyl group, (10) an optionally substituted C₁₋₆ alkoxy group, (11) a group represented by the formula R⁶S(O)ₚ (wherein R⁶ represents an optionally substituted C₁₋₆ alkyl group, and p represents 0, 1 or 2), (12) an oxo group, (13) a hydroxyimino group, (14) an optionally substituted C₁₋₆ alkoxyimino group and (15) an optionally substituted C₁₋₄ alkylenedioxy group;
   [12] the modulator of the aforementioned [1], wherein compound (I') is a compound represented by the formula

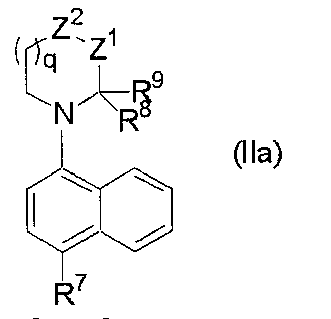

wherein R⁷ represents a cyano group, a nitro group, a halogen atom, an optionally substituted acyl group, an optionally esterified or amidated carboxyl group or a C₁₋₆ alkyl group substituted with 1 to 5 halogen atoms, R⁸ and R⁹ are the same or different and each represents (1) a hydrogen atom, (2) a cyano group, (3) a nitro group, (4) a C₁₋₆ alkyl group optionally substituted with a halogen atom, a hydroxy group or a C₁₋₆ alkoxy group, (5) a C₁₋₆ acyl group optionally substituted with a halogen atom, a hydroxy group or a C₁₋₆ alkoxy group, (6) a C₁₋₆ alkoxy group optionally substituted with a halogen atom, a hydroxy group or a C₁₋₆ alkoxy group or (7) an optionally esterified or amidated carboxyl group, q represents 0, 1 or 2, Z¹ represents a carbonyl group, a carbon atom substituted with a hydroxyimino group or an optionally substituted C₁₋₆ alkoxyimino group, a carbon atom substituted with a C₁₋₄ alkylenedioxy group or a group represented by the formula:

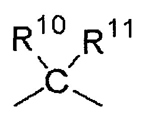

(wherein R¹⁰ and R¹¹ are the same or different and each represents
(1) a hydrogen atom, (2) a halogen atom, (3) a cyano group, (4) a nitro group, (5) a hydroxy group, (6) a C₁₋₆ alkyl group optionally substituted with a halogen atom, a hydroxy group or a C₁₋₆ alkoxy group, (7) a C₁₋₆ acyl group optionally substituted with a halogen atom, a hydroxy group or a C₁₋₆ alkoxy group, (8) a C₁₋₆ alkoxy group optionally substituted with a halogen atom, a hydroxy group or a C₁₋₆ alkoxy group, (9) an amino group optionally substituted with a C₁₋₆ alkyl group and/or a C₁₋₆ acyl group or (10) an optionally esterified or amidated carboxyl group), and Z² represents an oxygen atom, a sulfur atom, SO, SO₂, a carbonyl group, a carbon atom substituted with a hydroxyimino group or an optionally substituted C₁₋₆ alkoxyimino group, an amino group optionally substituted with a C₁₋₆ alkyl group or a C₁₋₆ acyl group, a carbon atom substituted with a C₁₋₄ alkylenedioxy group or a group represented by the formula:

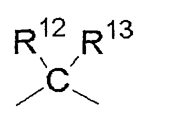

(wherein R¹² and R¹³ are the same or different and each represents
(1) a hydrogen atom, (2) a halogen atom, (3) a cyano group, (4) a nitro group, (5) a hydroxy group, (6) a C₁₋₆ alkyl group optionally substituted with a halogen atom, a hydroxy group or a C₁₋₆ alkoxy group, (7) a C₁₋₆ acyl group optionally substituted with a halogen atom, a hydroxy group or a C₁₋₆ alkoxy group, (8) a C₁₋₆ alkoxy group optionally substituted with a halogen atom, a hydroxy group or a C₁₋₆ alkoxy group, (9) an amino group optionally substituted with a C₁₋₆ alkyl group and/or a C₁₋₆ acyl group or (10) an optionally esterified or amidated carboxyl group), provided that
   1-[4-(1-piperidinyl)-1-naphthyl]ethanone, 4-(1-piperidinyl)-1-nitronaphthalene, 4-(1-piperidinyl)-1-naphthonitrile and 4-(1-pyrrolidinyl)-1-nitronaphthalene are excluded;
   [13] the modulator of the aforementioned [1], wherein compound (I') is a compound represented by the formula:

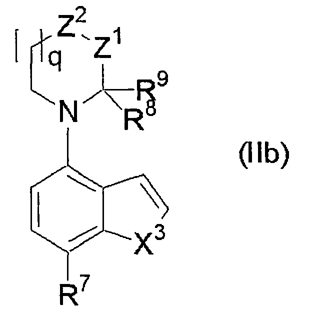

wherein X³ represents a sulfur atom or an oxygen atom, R⁷ represents a cyano group, a nitro group, a halogen atom, an optionally substituted acyl group, an optionally esterified or amidated carboxyl group or a C₁₋₆ alkyl group substituted with 1 to 5 halogen atoms, R⁸ and R⁹ are the same or different and each represents (1) a hydrogen atom, (2) a cyano group, (3) a nitro group, (4) a C₁₋₆ alkyl group optionally substituted with a halogen atom, a hydroxy group or a C₁₋₆ alkoxy group, (5) a C₁₋₆ acyl group optionally substituted with a halogen atom, a hydroxy group or a C₁₋₆ alkoxy group, (6) a C₁₋₆ alkoxy group optionally substituted with a halogen atom, a hydroxy group or a C₁₋₆ alkoxy group or (7) an optionally esterified or amidated carboxyl group, q represents 0, 1 or 2, Z¹ represents a carbonyl group, a carbon atom substituted with a hydroxyimino group or an optionally substituted C₁₋₆ alkoxyimino group, a carbon atom substituted with a C₁₋₄ alkylenedioxy group or a group represented by the formula:

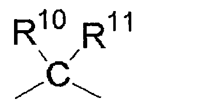

(wherein R¹⁰ and R¹¹ are the same or different and each represents
(1) a hydrogen atom, (2) a halogen atom, (3) a cyano group, (4) a nitro group, (5) a hydroxy group, (6) a C₁₋₆ alkyl group optionally substituted with a halogen atom, a hydroxy group or a C₁₋₆ alkoxy group, (7) a C₁₋₆ acyl group optionally substituted with a halogen atom, a hydroxy group or a C₁₋₆ alkoxy group, (8) a C₁₋₆ alkoxy group optionally substituted with a halogen atom, a hydroxy group or a C₁₋₆ alkoxy group, (9) an amino group optionally substituted with a C₁₋₆ alkyl group and/or a C₁₋₆ acyl group or (10) an optionally esterified or amidated carboxyl group), and Z² represents an oxygen atom, a sulfur atom, SO, SO₂, a carbonyl group, a carbon atom substituted with a hydroxyimino group or an optionally substituted C₁₋₆ alkoxyimino group, an amino group optionally substituted with a C₁₋₆ alkyl group or a C₁₋₆ acyl group, a carbon atom substituted with a C₁₋₄ alkylenedioxy group or a group represented by the formula:

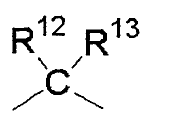

(wherein R¹² and R¹³ are the same or different and each represents
(1) a hydrogen atom, (2) a halogen atom, (3) a cyano group, (4) a nitro group, (5) a hydroxy group, (6) a C₁₋₆ alkyl group optionally substituted with a halogen atom, a hydroxy group or a C₁₋₆ alkoxy group, (7) a C₁₋₆ acyl group optionally substituted with a halogen atom, a hydroxy group or a C₁₋₆ alkoxy group, (8) a C₁₋₆ alkoxy group optionally substituted with a halogen atom, a hydroxy group or a C₁₋₆ alkoxy group, (9) an amino group optionally substituted with a C₁₋₆ alkyl group and/or a C₁₋₆ acyl group or (10) an optionally esterified or amidated carboxyl group), provided that a compound wherein
   X³ is an oxygen atom, R⁷ is a halogen atom, q is 0, R⁸ and R⁹ are each a hydrogen atom, Z¹ is a group represented by the formula:

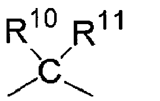

(wherein one of R¹⁰ and R¹¹ represents a hydrogen atom and the other represents an amino group optionally substituted with a C₁₋₆ alkyl group and/or a C₁₋₆ acyl group), and Z² is a methylene group is excluded;
[14] the modulator of the aforementioned [1], wherein compound (I') is
   4-[4-(hydroxymethyl)-1-piperidinyl]-1-naphthonitrile, 4-[3-(hydroxymethyl)-1-piperidinyl]-1-naphthonitrile, 4-[3-(hydroxymethyl)-3-methyl-1-piperidinyl]-1-naphthonitrile, 4-(2-methyl-1-pyrrolidinyl)-1-naphthonitrile, 4-(2-ethyl-1-pyrrolidinyl)-1-naphthonitrile, 4-(2-vinyl-1-pyrrolidinyl)-1-naphthonitrile, 4-(2-isopropyl-1-pyrrolidinyl)-1-naphthonitrile, 4-(3-hydroxy-2-methyl-1-pyrrolidinyl)-1-naphthonitrile, 4-(3-methoxy-2-methyl-1-pyrrolidinyl)-1-naphthonitrile, 4-(4-methoxy-2-methyl-1-pyrrolidinyl)-1-naphthonitrile, 4-[3-(hydroxymethyl)-2-methyl-1-pyrrolidinyl]-1-naphthonitrile, 4-[3-(1-hydroxy-1-methylethyl)-2-methyl-1-pyrrolidinyl]-1-naphthonitrile, 1-(4-cyano-1-naphthyl)-2-methylpyrrolidine-3-carboxamide, 1-(4-cyano-1-naphthyl)-2-methylpyrrolidine-3-carbonitrile, 4-(2-methyl-1-pyrrolidinyl)-1-benzothiophene-7-carbonitrile, 4-(3-hydroxy-2-methyl-1-pyrrolidinyl)-1-benzothiophene-7-carbonitrile, 4-(4-hydroxy-2-methyl-1-pyrrolidinyl)-1-benzothiophene-7-carbonitrile or an optically active form;
[15] the modulator of the aforementioned [10], wherein Ring A is an optionally substituted benzene ring;
[16] the modulator of the aforementioned [7], wherein Ring B is an optionally substituted pyrrolidine ring, an optionally substituted piperidine ring, an optionally substituted morpholine ring, an optionally substituted thiomorpholine ring or an optionally substituted perhydroazepine ring;
[17] the modulator of the aforementioned [1], which is combined with an anti-cancer agent;
[18] the modulator of the aforementioned [1], which is combined with a hormonal therapeutic agent;
[19] the modulator of the aforementioned [18], wherein the hormonal therapeutic agent is an LH-RH modulator;
[20] the modulator of the aforementioned [19], wherein the LH-RH modulator is an LH-RH agonist;
[21] the modulator of the aforementioned [20], wherein the LH-RH agonist is leuprorelin or a salt thereof;
[22] the method of the aforementioned [4], which comprises administration in combination with an effective amount of other anti-cancer agent;
[23] the method of the aforementioned [4], which comprises administration in combination with an effective amount of a hormonal therapeutic agent;
[24] the method of the aforementioned [23], wherein the hormonal therapeutic agent is an LH-RH modulator;
[25] the method of the aforementioned [24], wherein the LH-RH modulator is an LH-RH agonist;
[26] the method of the aforementioned [25], wherein the LH-RH agonist is leuprorelin or a salt thereof;
[27] the method of the aforementioned [4], which comprises administering an effective amount of the compound of the aforementioned [4] or a salt thereof or a prodrug thereof after administration of other anti-cancer agent;
[28] the method of the aforementioned [4], which comprises administering, to a mammal, an effective amount of the compound of the aforementioned [4] or a salt thereof or a prodrug thereof before applying an operation, a radiotherapy, a gene therapy, a thermotherapy, a cryotherapy and/or a laser ablation;
[29] the method of the aforementioned [4], which comprises administering, to a mammal, an effective amount of the compound of the aforementioned [4] or a salt thereof or a prodrug thereof after applying an operation, a radiotherapy, a gene therapy, a thermotherapy, a cryotherapy and/or a laser ablation;
[30] the modulator of the aforementioned [1], wherein Ring A represents
   (i) a C₅₋₈ alicyclic hydrocarbon,
   (ii) a C₆₋₈ aromatic hydrocarbon,
   (iii) a 5- or 6-membered aromatic monocyclic heterocycle containing, as ring-constituting atom, 1 to 4 of 1 to 3 kinds of hetero atoms selected from an oxygen atom, a sulfur atom and a nitrogen atom, or
   (iv) a 5- to 8-membered saturated non-aromatic heterocycle containing, as ring-constituting atom, 1 to 4 of 1 to 3 kinds of hetero atoms selected from an oxygen atom, a sulfur atom and a nitrogen atom,
      each of which is optionally substituted by substituent(s) selected from the group consisting of (1) a hydrogen atom, (2) a C₁₋₆ alkyl group, (3) a hydroxyl group and (4) an oxo group, Ring B represents

   (i) a 5- to 8-membered saturated or unsaturated non-aromatic heterocycle containing, as ring-constituting atom, 1 to 4 of 1 to 3 kinds of hetero atoms selected from an oxygen atom, a sulfur atom and a nitrogen atom,
   (ii) a non-aromatic heterocycle formed by the fusion of a 5- or 6-membered non-aromatic monocyclic heterocyclic group containing, as ring-constituting atom, 1 to 4 of 1 to 3 kinds of hetero atoms selected from an oxygen atom, a sulfur atom and a nitrogen atom, and a benzene ring, or
   (iii) a C₅₋₈ alicyclic hydrocarbon,
      each of which is, except for R^{a}, R², R^{3'}, R⁴ and R^{5'}, optionally further substituted by 1 to 6 groups selected from the group consisting of (1) a hydrogen atom, (2) a C₁₋₆ alkyl group optionally substituted by substituent(s) selected from (i) a hydroxyl group optionally substituted by substituent(s) selected from a C₆₋₁₀ aryl optionally substituted by a cyano and a carbamoyl, (ii) a C₁₋₆ alkoxy group, (iii) a C₁₋₆ alkoxycarbonyl group, (iv) a carboxyl group and (v) an amino group optionally substituted by substituent(s) selected from a C₁₋₆ alkylsulfonyl and a C₁₋₆ alkoxycarbonyl group, (3) a C₂₋₆ alkenyl group, (4) a C₆₋₁₄ aryl group, (5) an amino group optionally substituted by substituent(s) selected from (i) a C₁₋₆ alkyl, (ii) a C₁₋₆ alkanoyl, (iii) a C₁₋₆ alkoxycarbonyl and (iv) a C₁₋₆ alkylsulfonyl, (6) a hydroxyl group optionally substituted by a substituent selected from (i) a C₁₋₆ alkyl, (ii) a C₆₋₁₀ aryl and
   (iii) a C₇₋₁₀ aralkyl, (7) a carboxyl, (8) a C₁₋₆ alkoxycarbonyl,
   (9) a carbamoyl, (10) a halogen atom, (11) a cyano group, (12) a hydroxyimino group, (13) an oxo group and (14) a C₁₋₄ alkylenedioxy group,
   Ring C represents a benzene ring optionally further substituted by substituent(s) selected from a hydrogen atom and a halogen atom,
   X¹ represents a carbon atom optionally substituted by substituent(s) selected from the group consisting of (1) a hydrogen atom, (2) a hydroxyl group and (3) an oxo groups group,
   X² represents a carbon atom, an oxygen atom or a group represented by the formula S(O)ₖ (wherein k is 0, 1 or 2),
   W¹ represents a nitrogen atom or a group represented by the formula CR^{a} (wherein R^{a} is a bond, a hydrogen atom or a hydroxyl group),
   Y¹¹ represents CH₂,
   Y²¹ represents

1) when W¹ is a nitrogen atom,
   (i) a group represented by the formula CR⁴R^{5'} (wherein R⁴ represents (1) a hydrogen atom, (2) a cyano group, (3) a carbamoyl group, (4) a C₁₋₆ alkyl group optionally substituted by substituent(s) selected from (i) a hydroxy, (ii) a C₁₋₆ alkoxy and (iii) a C₆₋₁₀ aryloxy optionally substituted by a cyano group, or (5) a C₂₋₆ alkenyl group, and R^{5'} represents a hydrogen atom or a C₁₋₆ alkyl group, respectively),
   (ii) a nitrogen atom optionally substituted by a hydrogen atom or a C₁₋₆ alkyl group, or
   (iii) an oxygen atom, and
2) when W¹ is a group represented by the formula CR^{a} (wherein the symbol is as defined above),
   a group represented by the formula CR⁴R^{5'} (wherein R⁴ represents a C₁₋₆ alkyl group, and R^{5'} represents a bond or a hydrogen atom) or a nitrogen atom (provided that when Y²¹ is a nitrogen atom and W¹ is a group represented by the formula CR^{a} (wherein the symbol is as defined above), the bond between CR^{a} and Y²¹ is a double bond), and when Ring B is a optionally further substituted non-aromatic heterocycle formed by the fusion of a 5- or 6-membered non-aromatic monocyclic heterocyclic group and a benzene ring, CR² for Y¹¹ or CR⁴ or the nitrogen atom for Y²¹ may constitute a part of Ring B,
   R¹ represents a cyano group, a nitro group, a halogen atom, a C₁₋₆ alkanoyl group optionally substituted by a halogen atom, a carboxyl group or a C₁₋₆ alkyl group substituted by 1 to 5 halogen atoms, and
   the formula

-̅ -̅ -̅
represents a single bond or a double bond;
[31] the modulator of the aforementioned [6], wherein Ring A represents
   (i) a C₅₋₈ alicyclic hydrocarbon,
   (ii) a C₆₋₈ aromatic hydrocarbon,
   (iii) a 5- or 6-membered aromatic monocyclic heterocycle containing, as ring-constituting atom, 1 to 4 of 1 to 3 kinds of hetero atoms selected from an oxygen atom, a sulfur atom and a nitrogen atom, or
   (iv) a 5- to 8-membered saturated non-aromatic heterocycle containing, as ring-constituting atom, 1 to 4 of 1 to 3 kinds of hetero atoms selected from an oxygen atom, a sulfur atom and a nitrogen atom,
each of which is optionally substituted by substituent(s) selected from the group consisting of (1) a hydrogen atom, (2) a C₁₋₆ alkyl group, (3) a hydroxyl group and (4) an oxo group,
Ring B represents
(i) a 5- to 8-membered saturated or unsaturated non-aromatic heterocycle containing, as ring-constituting atom, 1 to 4 of 1 to 3 kinds of hetero atoms selected from an oxygen atom, a sulfur atom and a nitrogen atom,
(ii) a non-aromatic heterocycle formed by the fusion of a 5- or 6-membered non-aromatic monocyclic heterocyclic group containing, as ring-constituting atom, 1 to 4 of 1 to 3 kinds of hetero atoms selected from an oxygen atom, a sulfur atom and a nitrogen atom, and a benzene ring, or
(iii) a C₅₋₈ alicyclic hydrocarbon,
   each of which is, except for R^{a}, R, R^{3'}, R⁴ and R^{5'}, optionally further substituted by 1 to 6 groups selected from the group consisting of (1) a hydrogen atom, (2) a C₁₋₆ alkyl group optionally substituted by substituent(s) selected from (i) a hydroxyl group optionally substituted by substituent(s) selected from a C₆₋₁₀ aryl optionally substituted by a cyano and a carbamoyl, (ii) a C₁₋₆ alkoxy group, (iii) a C₁₋₆ alkoxycarbonyl group, (iv) a carboxyl group and (v) an amino group optionally substituted by substituent(s) selected from a C₁₋₆ alkylsulfonyl and a C₁₋₆ alkoxycarbonyl group, (3) a C₂₋₆ alkenyl group, (4) a C₆₋₁₄ aryl group, (5) an amino group optionally substituted by substituent(s) selected from (i) a C₁₋₆ alkyl, (ii) a C₁₋₆ alkanoyl, (iii) a C₁₋₆ alkoxycarbonyl and (iv) a C₁₋₆ alkylsulfonyl, (6) a hydroxyl group optionally substituted by a substituent selected from (i) a C₁₋₆ alkyl, (ii) a C₆₋₁₀ aryl and
(iii) a C₇₋₁₀ aralkyl, (7) a carboxyl, (8) a C₁₋₆ alkoxycarbonyl, (9) a carbamoyl, (10) a halogen atom, (11) a cyano group, (12) a hydroxyimino group, (13) an oxo group and (14) a C₁₋₄ alkylenedioxy group,
   Ring C represents a benzene ring optionally further substituted by substituent(s) selected from a hydrogen atom and a halogen atom,
   X¹ represents a carbon atom optionally substituted by substituent(s) selected from the group consisting of (1) a hydrogen atom, (2) a hydroxyl group and (3) an oxo groups group, X² represents a carbon atom, an oxygen atom or a group represented by the formula S(O)ₖ (wherein k is 0, 1 or 2), respectively.
   W represents a nitrogen atom, or
   when Ring A is an optionally substituted benzene ring, a group represented by the formula CR^{a} (wherein R^{a} is a bond, a hydrogen atom or a hydroxyl group),
   Y¹¹ represents CH₂,
   Y²¹ represents

1) when W is a nitrogen atom,
   (i) a group represented by the formula CR⁴R^{5'} (wherein R⁴ represents (1) a hydrogen atom, (2) a cyano group, (3) a carbamoyl group, (4) a C₁₋₆ alkyl group optionally substituted by substituent(s) selected from (i) a hydroxy, (ii) a C₁₋₆ alkoxy and (iii) a C₆₋₁₀ aryloxy optionally substituted by a cyano group, or (5) a C₂₋₆ alkenyl group, and R^{5'} represents a hydrogen atom or a C₁₋₆ alkyl group, respectively),
   (ii) a nitrogen atom optionally substituted by a hydrogen atom or a C₁₋₆ alkyl group, or
   (iii) an oxygen atom, and
2) when W is a group represented by the formula CR^{a} (wherein the symbol is as defined above),
   a group represented by the formula CR⁴R⁵' (wherein R⁴ represents a C₁₋₆ alkyl group, and R^{5'} represents a bond or a hydrogen atom) or a nitrogen atom (provided that when Y²¹ is a nitrogen atom and W is a group represented by the formula CR^{a} (wherein the symbol is as defined above), the bond between CR^{a} and Y²¹ is a double bond), and when Ring B is a optionally further substituted non-aromatic heterocycle formed by the fusion of a 5- or 6-membered non-aromatic monocyclic heterocyclic group and a benzene ring, CR² for Y¹¹- or CR⁴ or the nitrogen atom for Y²¹ may constitute a part of Ring B,
   R¹ represents a cyano group, a nitro group, a halogen atom, a C₁₋₆ alkanoyl group optionally substituted by a halogen atom, a carboxyl group or a C₁₋₆ alkyl group substituted by 1 to 5 halogen atoms, and
   the formula

-̅ -̅ -̅
represents a single bond or a double bond;
[32] the modulator of the aforementioned [12], wherein R⁷ represents a cyano group, a nitro group, a halogen atom, a C₁₋₆ alkanoyl group optionally substituted by a halogen atom, a carboxyl group or a C₁₋₆ alkyl group substituted by 1 to 5 halogen atoms,
   R⁸ and R⁹ are the same or different and each is
   (1) a hydrogen atom,
   (2) a cyano group,
   (3) a C₁₋₆ alkyl group optionally substituted by a hydroxyl group or a C₁₋₆ alkoxy group, or
   (4) a carbamoyl group,
      q represents 0, 1 or 2,
      Z¹ represents (1) a carbonyl group, (2) a carbon atom substituted by a hydroxyimino group, or
(3) a group represented by the formula

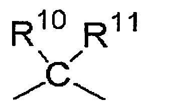

(wherein R¹⁰and R¹¹ are the same or different and each is
(1) a hydrogen atom,
(2) a halogen atom,
(3) a cyano group,
(4) a hydroxyl group,
(5) a C₁₋₆ alkyl group optionally substituted by substituent(s) selected from (i) a hydroxyl group and (ii) a C₁₋₆ alkoxy group,
(6) a C₁₋₆ alkoxy group,
(7) an amino group optionally substituted by substituent(s) selected from a C₁₋₆ alkyl group and a C₁₋₆ acyl group,
(8) a carboxyl group, or
(9) a C₁₋₆ alkoxycarbonyl group),
Z² represents
(1) an oxygen atom,
(2) a sulfur atom,
(3) SO,
(4) SO₂,
(5) a carbonyl group,
(6) a carbon atom substituted by a hydroxyimino group,
(7) a nitrogen atom substituted by a hydrogen atom or a C₁₋₆ alkyl group,
(8) a carbon atom substituted by a C₁₋₄ alkylenedioxy group, or
(9) a group represented by the formula

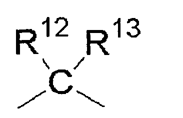

(wherein R¹² and R¹³ are the same or different and each is
(1) a hydrogen atom,
(2) a hydroxyl group,
(3) a C₁₋₆ alkyl group optionally substituted by substituent(s) selected from a hydroxyl group and a C₁₋₆ alkoxy group,
(4) a C₁₋₆ alkoxy group, or
(5) a carbamoyl group); and
[33] the modulator of the aforementioned [13], wherein X³ represents a sulfur atom or an oxygen atom,
R⁷ represents a cyano group,
R⁸ and R⁹ are the same or different and each is (1) a hydrogen atom or (2) a C₁₋₆ alkyl group,
q represents 0 or 1,
Z¹ represents a group represented by the formula

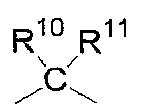

(wherein R¹⁰ and R¹¹ are the same or different and each is
(1) a hydrogen atom,
(2) a halogen atom,
(3) a hydroxyl group or
(4) a C₁₋₆ alkyl group optionally substituted by substituent(s) selected from a hydroxyl group and a C₁₋₆ alkoxy group),
   Z² represents a group represented by the formula

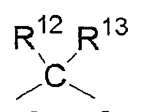

(wherein R¹² and R¹³ are the same or different and each is
(1) a hydrogen atom,
(2) a halogen atom,
(3) a hydroxyl group or
(4) a C₁₋₆ alkyl group optionally substituted by substituent(s) selected from a hydroxyl group and a C₁₋₆ alkoxy group); and the like.

### [Best Mode for Carrying out the Invention]

The compounds (I'), (I), (Ia), (IIa), (IIb), salts thereof and prodrugs thereof to be used in the present invention are known compounds described in W02004/16576, and can be obtained according to the production method described in the publication.

In the present specification, the formula

-̅ -̅ -̅

is also represented by

-̅ -̅ -̅

and both show a single bond or a double bond.

As the "hydrocarbon group" of the "optionally substituted hydrocarbon group" for R², R³, R^{3'}, R⁴, R⁵ or R^{5'}, for example, an "aliphatic linear hydrocarbon group", an "alicyclic hydrocarbon group" and an "aromatic hydrocarbon group" can be used.
As the "aliphatic linear hydrocarbon group" as an example of the hydrocarbon group, for example, a straight or branched aliphatic hydrocarbon group such as an alkyl group, an alkenyl group and an alkynyl group can be used.
As the "alkyl group", for example, a C₁₋₁₀ alkyl group such as methyl, ethyl, n-propyl, isopropyl, n-butyl, isobutyl, sec-butyl, tert-butyl, n-pentyl, isopentyl, neopentyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methylheptyl, nonyl, etc. can be used, and preferred are a C₁₋₆ alkyl (e.g., methyl, ethyl, n-propyl, isopropyl, n-butyl, sec-butyl, tert-butyl, etc.), etc.
As the "alkenyl group", for example, a C₂₋₁₀ alkenyl group such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc. can be used, and preferred are a C₂₋₆ alkenyl group, etc.
As the alkynyl group, for example, a C₂₋₁₀ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl can be used, and preferred are C₂₋₆ alkynyl group, etc.

As the "alicyclic hydrocarbon group" as an example of the hydrocarbon group, for example, a saturated or unsaturated, monocyclic or fused polycyclic alicyclic hydrocarbon group such as a cycloalkyl group, a cycloalkenyl group, cycloalkanedienyl group and a bicyclic or tricyclic fused ring of these groups and a C₆₋₁₄ aryl group (e.g., benzene, etc.), etc can be used.
As the "cycloalkyl group", for example, a C₃₋₁₀ cycloalkyl such as cyclopropyl, cyclobutyl, cyclopentyl, cyclohexyl, cycloheptyl, cyclooctyl, cyclononyl, etc can be used.
As the "cycloalkenyl group", for example, a C₃₋₁₀ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, etc can be used.
As the "cycloalkanedienyl group", for example, a C₄₋₆ cycloalkanedienyl group such as 2,4-cyclopentadien-1-yl, 2,4-cyclohexadien-1-yl, 2,5-cyclohexanedien-1-yl, etc can be used.

As the "aromatic hydrocarbon group" as an example of the hydrocarbon group, monocyclic or fused polycyclic aromatic hydrocarbon group can be used, and is not particularly limited. Preferred is a C₆₋₂₂ aromatic hydrocarbon group, more preferred is a C₆₋₁₈ aromatic hydrocarbon group, and further preferred are a C₆₋₁₀ aromatic hydrocarbon group, etc. Specific examples include phenyl, o-tolyl, m-tolyl, p-tolyl, 2,3-xylyl, 2,4-xylyl, mesityl, o-cumenyl, m-cumenyl, p-cumenyl, α-methylbenzyl, benzhydryl, o-biphenyl, m-biphenyl, p-biphenyl, 1-naphthyl, 2-naphthyl, 2-indenyl, 2-anthryl, azulenyl, phenantholyl, fluorenyl, etc. and, among these, phenyl, 1-naphthyl, 2-naphthyl, 2-anthryl, etc. are preferable.

While the "electron-withdrawing group" for R¹ is not particularly limited as long as it has tendency to attract electrons of others generally on the basis of hydrogen in the molecule, and is used in organic chemistry, for example, a cyano group, a nitro group, a halogen atom, an optionally substituted acyl group, an optionally esterified or amidated carboxyl group or a C₁₋₆ alkyl group substituted with 1 to 5 halogen atoms, etc. can be used.

As the "C₁₋₆ alkyl group" of the "optionally substituted C₁₋₆ alkyl group" for R⁶ and the "substituent on Ring A or Ring B except for R^{a}, R², R^{3'}, R⁴ and R^{5'}", those similar to the ones defined above can be used.
As the "C₁₋₆ alkoxy group" of the "optionally substituted C₁₋₆ alkoxy group" for the "substituent on Ring A or Ring B except for R^{a}, R², R^{3'}, R⁴ and R^{5'}", for example, methoxy, ethoxy, n-propoxy, isopropyloxy, n-butoxy, isobutyloxy, sec-butyloxy, tert-butyloxy, n-pentyloxy, isopentyloxy, neopentyloxy, n-hexyloxy, isohexyloxy, 1,1-dimethylbutyloxy, 2,2-dimethylbutyloxy, 3,3-dimethylbutyloxy and 2-ethylbutyloxy, etc. can be used, and methoxy, ethoxy, n-propoxy, isopropyloxy, n-butoxy, etc. are preferable.
As the "alkoxy group" of the "optionally substituted alkoxy group" for R^{a}, a C₁₋₆ alkoxy group is preferable, and, for example, methoxy, ethoxy, n-propoxy, isopropyloxy, n-butoxy, isobutyloxy, sec-butyloxy, tert-butyloxy, n-pentyloxy, isopentyloxy, neopentyloxy, n-hexyloxy, isohexyloxy, 1,1-dimethylbutyloxy, 2,2-dimethylbutyloxy, 3,3-dimethylbutyloxy and 2-ethylbutyloxy, etc. can be used, with preference given to methoxy, ethoxy, n-propoxy, isopropyloxy, n-butoxy, etc.
As the "halogen atom" for R¹, R⁷, R¹⁰, R¹¹, R¹², R¹³ and the "substituent on Ring A or Ring B except for R^{a}, R², R^{3'}, R⁴ and R^{5'}", a fluorine atom, a chlorine atom, a bromine atom, an iodine atom, etc. can be used, and a fluorine atom, a chlorine atom, etc. are preferable.

As the "acyl group" of the "optionally substituted acyl group" for R¹, R², R³, R^{3'}, R⁴, R⁵, R^{5'} or R⁷, for example, a lower (C₁₋₆) alkanoyl group such as formyl, acetyl, propionyl, butyryl, isobutyryl, valeryl, isovaleryl, pivaloyl and hexanoyl; a lower (C₃₋₇) alkenoyl group such as acryloyl, methacryloyl, crotonoyl and isocrotonoyl; a C₄₋₇ cycloalkanecarbonyl group such as a cyclopropanecarbonyl group, a cyclobutanecarbonyl group, a cyclopentanecarbonyl group and a cyclohexanecarbonyl group; a lower (C₁₋₄) alkanesulfonyl group such as mesyl, ethanesulfonyl and propanesulfonyl; a C₇₋₁₄ aroyl group such as benzoyl, p-toluoyl, 1-naphthoyl and 2-naphthoyl; a C₆₋₁₀ aryl lower (C₂₋₄) alkanoyl group such as phenylacetyl, phenylpropionyl, hydroatropoyl and phenylbutyryl; a C₆₋₁₀ aryl lower (C₃₋₅) alkenoyl group such as cinnamoyl and atropoyl; a C₆₋₁₀ arenesulfonyl group such as benzenesulfonyl and p-toluenesulfonyl group, etc. can be used.
As the "C₁₋₆ acyl group" of the "optionally substituted C₁₋₆ acyl group" for the "substituent on Ring A or Ring B except for R^{a}, R², R^{3'}, R⁴ and R^{5'}", a lower (C₁₋₆) alkanoyl group such as formyl, acetyl, propionyl, butyryl, isobutyryl, valeryl, isovaleryl, pivaloyl and hexanoyl; a lower (C₃₋₆) alkenoyl group such as acryloyl, methacryloyl, crotonoyl and isocrotonoyl; a C₄₋₆ cycloalkanecarbonyl group such as a cyclopropanecarbonyl group, a cyclobutanecarbonyl group and a cyclopentanecarbonyl group, etc. can be used.

As the "optionally esterified or amidated carboxyl group" for R¹, R², R³, R^{3'}, R⁴, R⁵, R^{5'}, R⁷, R⁸, R⁹, R¹⁰, R¹¹, R¹² R¹³ and the "substituent on Ring A or Ring B except for R^{a}, R², R^{3'}, R⁴ and R^{5'}", a carboxyl group, alkoxycarbonyl, aryloxycarbonyl, aralkyloxycarbonyl, carbamoyl, N-monosubstituted carbamoyl and N,N-disubstituted carbamoyl, etc. can be used.
As the "alkoxycarbonyl" used herein, for example, lower (C₁₋₆) alkoxycarbonyl such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl and neopentyloxycarbonyl, etc. can be used, and among these, C₁₋₃ alkoxycarbonyl such as methoxycarbonyl, ethoxycarbonyl and propoxycarbonyl, etc. are preferable. The "lower alkoxycarbonyl" may have a substituent, and as the substituent, a hydroxy group, an optionally substituted amino group [the amino group may have, for example, 1 or 2 substituents such as a lower alkyl group (e.g., C₁₋₆ alkyl such as methyl, ethyl, propyl, isopropyl, butyl, isobutyl, tert-butyl, pentyl, hexyl, etc., preferably, methyl, ethyl, etc.) optionally substituted with 1 to 5 halogen atoms (e.g., fluorine, chlorine, bromine, iodine, etc.), an acyl group (e.g., C₁₋₆ alkanoyl such as formyl, acetyl, propionyl and pivaloyl, benzoyl, etc.), a carboxyl group and C₁₋₆ alkoxycarbonyl, etc.], a halogen atom (e.g., fluorine, chlorine, bromine, iodine, etc.), a nitro group, a cyano group, a lower alkoxy group (e.g., C₁₋₆ alkoxy such as methoxy, ethoxy, n-propoxy, isopropoxy, n-butoxy, isobutoxy, sec-butoxy and tert-butoxy, etc., preferably, methoxy, ethoxy, etc.) optionally substituted with 1 to 5 halogen atoms (e.g., fluorine, chlorine, bromine, iodine, etc.), etc. can be used. Furthermore, these substituents may be the same or different and the number of substituents is preferably 1, 2 or 3 (more preferably 1 or 2).
As the "aryloxycarbonyl" used herein, for example, C₆₋₁₄ aryloxycarbonyl such as phenoxycarbonyl, 1-naphthoxycarbonyl, 2-naphthoxycarbonyl, 1-phenanthoxycarbonyl, etc. can be used. The "aryloxycarbonyl" may have a substituent, and the substituent includes the same number of those similar to the above-mentioned substituents for the "alkoxycarbonyl" as the substituent.
As the "aralkyloxycarbonyl" as used herein is preferably, for example, C₇₋₁₄ aralkyloxycarbonyl such as benzyloxycarbonyl, phenethyloxycarbonyl, etc. (preferably, C₆₋₁₀ aryl-C₁₋₄ alkoxycarbonyl, etc.). The "aralkyloxycarbonyl" may have a substituent, and the substituent includes the same number of those similar to the above-mentioned substituents for the "alkoxycarbonyl" as the substituent.
As the "N-monosubstituted carbamoyl" used herein, for example, lower alkyl (e.g., C₁₋₆ alkyl such as methyl, ethyl, propyl, isopropyl, butyl, isobutyl, tert-butyl, pentyl, hexyl, etc.), lower alkenyl (e.g., C₂₋₆ alkenyl such as vinyl, allyl, isopropenyl, propenyl, butenyl, pentenyl, hexenyl, etc.), cycloalkyl (e.g., C₃₋₆ cycloalkyl such as cyclopropyl, cyclobutyl, cyclopentyl and cyclohexyl, etc.), aryl (e.g., C₆₋₁₀ aryl such as phenyl, 1-naphthyl, 2-naphthyl, etc.), aralkyl (e.g., C₇₋₁₀ aralkyl such as benzyl and phenethyl, preferably, phenyl-C₁₋₄ alkyl, etc.), arylalkenyl (e.g., C₈₋₁₀ arylalkenyl such as cinnamyl, preferably, phenyl-C₂₋₄ alkenyl, etc.), heterocyclic group (e.g., those similar to the "heterocyclic group" of the below-mentioned "optionally substituted heterocyclic group" as a substituent, etc.), etc. can be used. The lower alkyl, lower alkenyl, cycloalkyl, aryl, aralkyl, arylalkenyl and the heterocyclic group may have a substituent, and the substituent includes the same number of those similar to the above-mentioned substituents for the "alkoxycarbonyl" as the substituent.
The "N,N-disubstituted carbamoyl" used herein means a carbamoyl group having two substituents on the nitrogen atom.
As the examples of one of the two substituents, those such as the above-mentioned substituents of the "N-monosubstituted carbamoyl" as the substituent can be used, and as the examples of the other substituent, for example, lower alkyl (e.g., C₁₋₆ alkyl such as methyl, ethyl, propyl, isopropyl, butyl, tert-butyl, pentyl, hexyl, etc.), C₃₋₇ cycloalkyl (e.g., cyclopropyl, cyclobutyl, cyclopentyl, cyclohexyl, etc.), C₇₋₁₀ aralkyl (e.g., benzyl and phenethyl, etc., preferably, phenyl-C₁₋₄ alkyl, etc.), etc. can be used. Furthermore, the two substituents may form a cyclic amino together with the nitrogen atom, and in this case, as the cyclic aminocarbamoyl, for example, a 3- to 8-membered (preferably, a 5- or 6-membered) cyclic aminocarbonyl such as 1-azetidinylcarbonyl, 1-pyrrolidinylcarbonyl, piperidinocarbonyl, morpholinocarbonyl, 1-piperazinylcarbonyl, and 1-piperazinylcarbonyl optionally having lower alkyl (e.g., C₁₋₆ alkyl such as methyl, ethyl, propyl, isopropyl, butyl, tert-butyl, pentyl, hexyl, etc.), aralkyl (e.g., C₇₋₁₀ aralkyl such as benzyl, phenethyl, etc.), aryl (e.g., C₆₋₁₀ aryl such as phenyl, 1-naphthyl, 2-naphthyl, etc.), etc. at the 4-position, etc. can be used.

As the "C₁₋₆ alkyl group substituted with 1 to 5 halogen atoms" for R¹ or R⁷, C₁₋₆ alkyl (e.g., methyl, ethyl, propyl, isopropyl, butyl, isobutyl, sec-butyl, tert-butyl, pentyl, hexyl, etc.) optionally having 1 to 5, preferably 1 to 3, halogen atoms (e.g., fluorine, chlorine, bromine, iodine, etc.) etc. can be used. Specifically, for example, fluoromethyl, chloromethyl, difluoromethyl, trichloromethyl, trifluoromethyl, 1-fluoroethyl, 2-bromoethyl, 2,2,2-trifluoroethyl, pentafluoroethyl, 2-fluoropropyl, 1,2-difluoropropyl, 3,3,3-trifluoropropyl, 1-fluorobutyl, 4,4,4-trifluorobutyl, 1-fluoropentyl, 5,5,5-trifluoropentyl, 1-fluorohexyl, 3,3-difluorohexyl, 6,6,6-trifluorohexyl, etc. can be used.

As the "optionally substituted amino group" of the "substituent on Ring A or Ring B except for R^{a}, R², R³', R⁴ and R^{5'}", groups similar to the "optionally substituted amino group" as the below-defined "substituent" can be used.
As the "C₁₋₆ alkoxyimino group" of the "optionally substituted C₁₋₆ alkoxyimino group" of the "carbon atom substituted with optionally substituted C₁₋₆ alkoxyimino group" for Z¹ or Z², and as the "C₁₋₆ alkoxyimino group" of the "optionally substituted C₁₋₆ alkoxyimino group" for the "substituent on Ring A or Ring B except for R^{a}, R², R^{3'}, R⁴ and R^{5'}", for example, methoxyimino, ethoxyimino, n-propoxyimino, isopropyloxyimino, n-butoxyimino, isobutyloxyimino, sec-butyloxyimino, tert-butyloxyimino, n-pentyloxyimino, isopentyloxyimino, neopentyloxyimino, n-hexyloxyimino, isohexyloxyimino, 1,1-dimethylbutyloxyimino, 2,2-dimethylbutyloxyimino, 3,3-dimethylbutyloxyimino, 2-ethylbutyloxyimino, etc. can be used, and methoxyimino, ethoxyimino, n-propoxyimino, isopropyloxyimino, n-butoxyimino, etc. are preferable.

As the "C₁₋₄ alkylenedioxy group" of the "carbon atom substituted with a C₁₋₄ alkylenedioxy group" for Z¹ or Z², and as the "C₁₋₄ alkylenedioxy group" of the "optionally substituted C₁₋₄ alkylenedioxy group" for the "substituent on Ring A or Ring B except for R^{a}, R², R^{3'}, R⁴ and R^{5'}", for example, a methylenedioxy group, an ethylenedioxy group, a propylenedioxy group, a butylenedioxy group, etc. can be used, and a methylenedioxy group and an ethylenedioxy group are preferable.

As the "C₁₋₆ alkyl group optionally substituted with a halogen atom, a hydroxy group or a C₁₋₆ alkoxy group" for R⁸, R⁹, R¹⁰, R¹¹, R¹² or R¹³, those substituted with 0 to 5, preferably 0 to 3, from the above-defined "halogen atom", a hydroxy group and the above-defined "C₁₋₆ alkoxy group" at the substitutable positions of the above-defined "C₁₋₆ alkyl group" can be used. For example, those substituted with 0 to 5, preferably 0 to 3, from a fluorine atom, a chlorine atom, a bromine atom, an iodine atom; a hydroxy group; a C₁₋₆ alkoxy group such as methoxy, ethoxy, n-propoxy, isopropyloxy, n-butoxy, isobutyloxy, sec-butyloxy, tert-butyloxy, n-pentyloxy, isopentyloxy, neopentyloxy, n-hexyloxy, isohexyloxy, 1,1-dimethylbutyloxy, 2,2-dimethylbutyloxy, 3,3-dimethylbutyloxy and 2-ethylbutyloxy at the substitutable positions of a C₁₋₆ alkyl group such as methyl, ethyl, n-propyl, isopropyl, n-butyl, isobutyl, sec-butyl, tert-butyl, n-pentyl, isopentyl, neopentyl, n-hexyl, isohexyl, 1,1-dimethylbutyl, 2,2-dimethylbutyl, 3,3-dimethylbutyl, 3,3-dimethylpropyl, 2-ethylbutyl and n-heptyl. Specifically, methyl, fluoromethyl, chloromethyl, difluoromethyl, trichloromethyl, trifluoromethyl, hydroxymethyl, methoxymethyl, ethoxymethyl, pentyloxymethyl, ethyl, 1-fluoroethyl, 2-bromoethyl, 1,2-dichloroethyl, 1,2-dichloro-1-hydroxyethyl, 2,2,2-trifluoroethyl, pentafluoroethyl, 1-hydroxyethyl, 1,2-dihydroxyethyl, n-propyl, isopropyl, 1-hydroxypropyl, ethoxypropyl, 2-fluoropropyl, 1,2-difluoropropyl, 3,3,3-trifluoropropyl, n-butyl, isobutyl, 1-chlorobutyl, 4,4,4-trifluorobutyl, fluoromethoxybutyl, 1,1-dimethylbutyl, 2,2-dimethylbutyl, 3,3-dimethylbutyl, 1-hydroxy-2-fluoro-propyl, n-butyl, sec-butyl, tert-butyl, n-pentyl, isopentyl, neopentyl, 1-fluoropentyl, 5,5,5-trifluoropentyl, n-hexyl, isohexyl, 1-fluorohexyl, 3,3-difluorohexyl, 6,6,6-trifluorohexyl, etc. can be used.

As the "C₁₋₆ acyl group optionally substituted with a halogen atom, a hydroxy group or a C₁₋₆ alkoxy group" for R⁸, R⁹ R¹⁰, R¹¹, R¹² or R¹³, those substituted with 0 to 5, preferably 0 to 3, from the above-defined "halogen atom", a hydroxy group and the above-defined "C₁₋₆ alkoxy group" at the substitutable positions of the above-defined "C₁₋₆ acyl group" can be used. For example, those substituted with 0 to 5, preferably, 0 to 3 of a fluorine atom, a chlorine atom, a bromine atom, an iodine atom; a hydroxy group; a C₁₋₆ alkoxy group such as methoxy, ethoxy, n-propoxy, isopropyloxy, n-butoxy, isobutyloxy, sec-butyloxy, tert-butyloxy, n-pentyloxy, isopentyloxy, neopentyloxy, n-hexyloxy, isohexyloxy, 1,1-dimethylbutyloxy, 2,2-dimethylbutyloxy, 3,3-dimethylbutyloxy and 2-ethylbutyloxy, at the substitutable positions of a C₁₋₆ acyl group such as formyl, acetyl, propionyl, butyryl, isobutyryl, valeryl, isovaleryl, pivaloyl, hexanoyl, acryloyl, methacryloyl, crotonoyl, isocrotonoyl, cyclopropanecarbonyl, cyclobutanecarbonyl and cyclopentanecarbonyl can be used.

As the "C₁₋₆ alkoxy group optionally substituted with a halogen atom, a hydroxy group or a C₁₋₆ alkoxy group" for R⁸, R⁹, R¹⁰, R¹¹, R¹² or R¹³, those substituted with 0 to 5, preferably 0 to 3, from the above-defined "halogen atom", a hydroxy group and the above-defined "C₁₋₆ alkoxy group" at the substitutable positions of the above-defined "C₁₋₆ alkoxy group" can be used. For example, those substituted with 0 to 5, preferably 0 to 3, from a fluorine atom, a chlorine atom, a bromine atom, an iodine atom; a hydroxy group; a C₁₋₆ alkoxy group such as methoxy, ethoxy, n-propoxy, isopropyloxy, n-butoxy, isobutyloxy, sec-butyloxy, tert-butyloxy, n-pentyloxy, isopentyloxy, neopentyloxy, n-hexyloxy, isohexyloxy, 1,1-dimethylbutyloxy, 2,2-dimethylbutyloxy, 3,3-dimethylbutyloxy and 2-ethylbutyloxy at the substitutable positions of a C₁₋₆ alkoxy group such as methoxy, ethoxy, n-propoxy, isopropyloxy, n-butoxy, isobutyloxy, sec-butyloxy, tert-butyloxy, n-pentyloxy, isopentyloxy, neopentyloxy, n-hexyloxy, isohexyloxy, 1,1-dimethylbutyloxy, 2,2-dimethylbutyloxy, 3,3-dimethylbutyloxy and 2-ethylbutyloxy can be used.

As the "amino group optionally substituted with a C₁₋₆ alkyl group and/or a C₁₋₆ acyl group" for R¹⁰, R¹¹, R¹² or R¹³, those in which the amino group is substituted with 0 to 2 groups selected from a C₁₋₆ alkyl group such as methyl, ethyl, propyl, isopropyl, butyl, isobutyl, tert-butyl, pentyl and hexyl and a C₁₋₆ acyl group such as formyl, acetyl, propionyl, butyryl, isobutyryl, valeryl, isovaleryl, pivaloyl, hexanoyl, acryloyl, methacryloyl, crotonoyl, isocrotonoyl, cyclopropanecarbonyl, cyclobutanecarbonyl and cyclopentanecarbonyl can be used.

As the "amino group optionally substituted with a C₁₋₆ alkyl group or a C₁₋₆ acyl group" for Z², those in which the amino group is substituted with 0 to 2 groups selected from a C₁₋₆ alkyl group such as methyl, ethyl, propyl, isopropyl, butyl, isobutyl, tert-butyl, pentyl and hexyl and a C₁₋₆ acyl group such as formyl, acetyl, propionyl, butyryl, isobutyryl, valeryl, isovaleryl, pivaloyl, hexanoyl, acryloyl, methacryloyl, crotonoyl, isocrotonoyl, cyclopropanecarbonyl, cyclobutanecarbonyl and cyclopentanecarbonyl can be used.

k, m, p and q represent 0, 1 or 2. Therefore, when k, m and p represent 0 in the formulae S(O)k, S(O)ₘ, and S(O)ₚ, the formulae mean S; when k, m and p represent 1 in the formulae S(O)ₖ, S(O)ₘ and S(O)ₚ, the formulae mean S(O); when k, m and p represent 2 in the formulae S(O)ₖ, S(O)ₘ and S(O)ₚ, the formulae mean S(O)₂. Furthermore, when q represents 0, the formulae mean a chemical bond, when q represents 1, the formulae mean a methylene group, and when q represents 2, the formulae mean an ethylene group.

As the "5- to 8-membered ring" of the "optionally substituted 5- to 8-membered ring" for Ring A, for example, "alicyclic hydrocarbon", "aromatic hydrocarbon", a "heterocycle", etc. can be used.
As the "4- to 10-membered ring" of the "optionally further substituted 4- to 10-membered ring" for Ring B, for example, a "non-aromatic heterocycle", "alicyclic hydrocarbon", etc. can be used.

As the "alicyclic hydrocarbon", for example, a saturated or unsaturated monocyclic or fused polycyclic C₅₋₈ or C₄₋₁₀ alicyclic hydrocarbon such as cycloalkane, cycloalkene, cycloalkanediene and a bicyclic fused ring of these groups and benzene can be used.
As the "cycloalkane", for example, C₃₋₁₀ cycloalkane such as cyclopropane, cyclobutane, cyclopentane, cyclohexane, cycloheptane, cyclooctane, cyclononane, etc. can be used.
As the "cycloalkene", for example, C₃₋₁₀ cycloalkene such as cyclopentene, cyclohexene, cyclobutene, etc. can be used.
As the "cycloalkanediene", for example, C₄₋₆ cycloalkanediene such as cyclopentadiene, cyclohexadiene, cyclohexanediene, etc. can be used.
As the "aromatic hydrocarbon", monocyclic or fused polycyclic aromatic hydrocarbon can be used, and is not particularly limited but preferably, C₆₋₈ aromatic hydrocarbon, more preferably, C₆ aromatic hydrocarbon, etc., specifically, for example, benzene, toluene, xylene, mesitylene, cumene, styrene, 1,2,3-trimethylbenzene, pentalene, etc., preferably, benzene, toluene, etc. can be used.

As the "heterocycle", for example, an aromatic heterocycle, a saturated or unsaturated non-aromatic heterocycle (an aliphatic heterocycle), etc., containing at least one (preferably, 1 to 4, further preferably, 1 or 2) hetero atoms of 1 to 3 kinds (preferably, 1 or 2 kinds) selected from an oxygen atom, a sulfur atom and a nitrogen atom, etc. as a ring-constituting atom (a ring atom) can be used, and is not particularly limited but preferably, 4- to 10-membered or 5- to 8-membered heterocycle, etc.
As the specific examples of the "aromatic heterocycle", 5-to 10-membered aromatic heterocycle, for example, a 5- or 6-membered aromatic monocyclic heterocycle (e.g., furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, etc.), and a 8- to 10-membered aromatic fused heterocycle (e.g., 1H-pyrrolo[1,2-c]imidazole, pyrrolo[1,2-a]imidazol-4-ium, pyrrolo[1,2-c]imidazol-4-ium, pyrrolo[2,3-c]pyrazole, pyrrolo[3,2-c]pyrazole, pyrrolo[3,4-c]pyrazole, 1*H*-pyrrolo[3,2-c]pyrazole, pyrrolo[1,2-b]pyrazol-7-ium, 1*H*-furo[2,3-d]imidazole, 1*H*-furo[3,4-d]imidazole, 1*H-*furo[2,3-c]pyrazole, 1*H*-furo[2,3-d]imidazole, 1*H*-furo[3,2-c]pyrazole, 1*H*-furo[3,4-c]pyrazole, 1*H*-thieno[2,3-d]imidazole, thieno[2,3-b]furan, 4*H*-imidazo[4,5-d]thiazole, imidazo[2,1-b]thiazole, 5*H*-pyrrolo[1,2-c]imidazole, benzofuran, isobenzofuran, benzothiophene, indole, isoindole, 1*H*-indazole, benzoxazole, 1,2-benzoisoxazole, benzothiazole, benzopyran, 1,2-benzoisothiazole, 1*H*-benzotriazole, quinoline, isoquinoline, cinnoline, quinazoline, quinoxaline, phthalazine, naphthyridine, purine, pteridine, indolizine, pyrrolo[1,2-b]pyridazine, pyrazolo[1,5-a]pyridine, imidazo[1,2-a]pyridine, imidazo[1,5-a]pyridine, imidazo[1,2-b]pyridazine, imidazo[1,2-a]pyrimidine, 1,2,4-triazolo[4,3-a]pyridine, 1,2,4-triazolo[4,3-b]pyridazine, etc. (preferably, a heterocycle in which the above-mentioned 5-or 6-membered aromatic monocyclic heterocyclic group is fused with a benzene ring, or a heterocycle in which the same or different two heterocycles of the above-mentioned 5- or 6-membered aromatic monocyclic heterocyclic group are fused with each other, etc.)) can be used.
As the specific examples of the "non-aromatic heterocycle", for example, a 4- to 10-membered or 5- to 8-membered saturated or unsaturated (preferably, saturated) non-aromatic heterocycle (aliphatic heterocycle) such as oxetane, pyrroline, imidazoline, imidazolidine, pyrazoline, pyrazolidine, quinuclidine, aziridine, oxirane, azetidine, pyrrolidine, tetrahydrofuran, thiolane, piperidine, tetrahydropyran, dioxolane, thiazane, morpholine, thiomorpholine, piperazine, azepane, perhydroindole, perhydropyrrolo[2,3-d]pyridine, perhydropyrrolo[3,2-d]pyridine, and 7-azabicyclo[2,2,1]heptane, 6-oxabicyclo[3,1,0]hexane, in addition to these, a compound that the above-mentioned aromatic heterocycle is partially or completely saturated, and the like can be used.
Here, when Ring B is a bicyclic ring optionally further substituted, CR² for Y¹ or CR⁴ or the nitrogen atom for Y² may constitute a part of Ring B.
Further, when Ring B is a bicyclic ring optionally further substituted, CR² for Y¹¹ or CR⁴ or the nitrogen atom for Y²¹ may constitute a part of Ring B.

As the substituent in the present invention such as the substituent of the "optionally substituted hydrocarbon group" for R², R³, R^{3'}, R⁴, R⁵ or R^{5'}; the substituent of the "optionally substituted 5- to 8-membered ring" for Ring A; the substituent of the "optionally further substituted 4- to 10-membered ring" for Ring B; the substituent of the "optionally further substituted benzene ring" for Ring C; the substituent of the "optionally substituted benzene ring", the "optionally substituted thiophene ring", the "optionally substituted furan ring" for Ring A; the substituent of the "optionally substituted pyrrolidine ring", the "optionally substituted piperidine ring", the "optionally substituted piperazine ring", the "optionally substituted morpholine ring", the "optionally substituted thiomorpholine ring", the "optionally substituted pyrazoline ring", the "optionally substituted pyrazolidine ring", the "optionally substituted isoxazoline ring", the "optionally substituted cyclopentane ring", the "optionally substituted cyclopentene ring" or the "optionally substituted perhydroazepine ring" for Ring B is not particularly limited, but for example, (i) an optionally substituted alkyl group, (ii) an optionally substituted alkenyl group, (iii) an optionally substituted alkynyl group, (iv) an optionally substituted aryl group, (v) an optionally substituted aralkyl group, (vi) an optionally substituted cycloalkyl group, (vii) an optionally substituted cycloalkenyl group, (viii) an optionally substituted heterocyclic group, (ix) an optionally substituted amino group, (x) an optionally substituted imidoyl group (e.g., a group represented by the formula -C(U')=N-U [wherein U and U' represent a hydrogen atom or a substituent, respectively (U represents preferably a hydrogen atom), etc.], (xi) an optionally substituted amidino group (e.g., a group represented by the formula -C(NE^{'}E^{''})=N-E [wherein E, E^{'} and E^{"} represent a hydrogen atom or a substituent, respectively (E represents preferably a hydrogen atom)], etc.), (xii) an optionally substituted hydroxy group, (xiii) an optionally substituted thiol group, (xiv) an optionally substituted alkylsulfinyl group, (xv) an optionally esterified or amidated carboxyl group, (xvi) an optionally substituted thiocarbamoyl group, (xvii) an optionally substituted sulfamoyl group, (xviii) a halogen atom (e.g., fluorine, chlorine, bromine, iodine, etc., preferably, chlorine, bromine, etc.), (xix) a cyano group, (xx) an isocyano group, (xxi) a cyanate group, (xxii) an isocyanate group, (xxiii) a thiocyanate group, (xxiv) an isothiocyanate group, (xxv) a nitro group, (xxvi) a nitroso group, (xxvii) a sulfonic acid-derived acyl group, (xxviii) a carboxylic acid-derived acyl group, (xxix) an oxo group, (xxx) a thioxo group, (xxxi) a C₁₋₄ alkylenedioxy group, etc. are used. These optional substituents may exist in the number of 1 to 5 (preferably, 1 to 3) at substitutable positions.

As the alkyl group of the "optionally substituted alkyl group" as the above-mentioned substituent, for example, C₁₋₆ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, etc. can be used. Here, as the substituent of the alkyl group, a lower alkoxy group (e.g., C₁₋₆ alkoxy such as methoxy, ethoxy, propoxy, etc.), a halogen atom (e.g., fluorine, chlorine, bromine, iodine, etc.), a lower alkyl group (e.g., C₁₋₆ alkyl such as methyl, ethyl, propyl, etc.), a lower alkenyl group (e.g., C₂₋₆ alkenyl such as vinyl, allyl, etc.), a lower alkynyl group (e.g., C₂₋₆ alkynyl such as ethynyl, propargyl, etc.), an optionally substituted amino group, an optionally substituted hydroxy group, a cyano group, an optionally substituted amidino group, a carboxy group, a lower alkoxycarbonyl group (e.g., C₁₋₆ alkoxycarbonyl such as methoxycarbonyl, ethoxycarbonyl, etc.), an optionally substituted carbamoyl group (e.g., a carbamoyl group optionally substituted with a C₁₋₆ alkyl group optionally substituted with a 5- or 6-membered monocyclic aromatic heterocyclic group (e.g., pyridyl, etc.) or an acyl group (e.g., formyl, C₂₋₆ alkanoyl, benzoyl, optionally halogenated C₁₋₆ alkoxycarbonyl, optionally halogenated C₁₋₆ alkylsulfonyl, benzenesulfonyl, etc.), 1-azetidinylcarbonyl, 1-pyrrolidinylcarbonyl, piperidinocarbonyl, morpholinocarbonyl, 1-piperazinylcarbonyl, etc.), etc. can be used. These optional substituents may exist at substitutable positions in the number of 1 to 3.

As the "optionally substituted amino group", the "optionally substituted hydroxy group" and the "optionally substituted amidino group" as the substituent of the above-mentioned "optionally substituted alkyl group", those such as the "optionally substituted amino group", the "optionally substituted hydroxy group" and the "optionally substituted amidino group" as the below-described substituent can be used.

As the alkenyl group in the "optionally substituted alkenyl group" as the above-mentioned substituent, for example, C₂₋₆ alkenyl such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc. can be used. Here, as the substituent of the alkenyl, those such as the above-mentioned substituent in the "optionally substituted alkyl group" as the substituent in the same number can be used.

As the alkynyl group in the "optionally substituted alkynyl group" as the above-mentioned substituent, for example, C₂₋₆ alkynyl such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl can be used. Here, as the substituent of the alkynyl group, those such as the above-mentioned substituent in the "optionally substituted alkyl group" as the substituent in the same number can be used.

As the aryl group in the "optionally substituted aryl group" as the above-mentioned substituent, for example, C₆₋₁₄ aryl such as phenyl, naphthyl, anthryl, phenantholyl, acenaphthylenyl, etc. can be used. Here, as the substituent of the aryl group, those such as the above-mentioned substituent in the "optionally substituted alkyl group" as the substituent in the same number can be used.

As the aralkyl group in the "optionally substituted aralkyl group" as the above-mentioned substituent, for example, C₇₋₁₁ aralkyl such as benzyl, phenethyl, naphthylmethyl, etc. can be used. Here, as the substituent of the aralkyl group, those such as the above-mentioned substituent in the "optionally substituted alkyl group" as the substituent in the same number can be used.

As the cycloalkyl group in the "optionally substituted cycloalkyl group" as the above-mentioned substituent, for example, C₃₋₇ cycloalkyl such as cyclopropyl, cyclobutyl, cyclopentyl, cyclohexyl, cycloheptyl, etc. can be used. Here, as the substituent of the cycloalkyl group, those such as the above-mentioned substituent in the "optionally substituted alkyl group" as the substituent in the same number can be used.

As the cycloalkenyl group in the "optionally substituted cycloalkenyl group" as the above-mentioned substituent, for example, C₃₋₇ cycloalkenyl such as cyclopropenyl, cyclobutenyl, cyclopentenyl, cyclohexenyl, etc. can be used. Here, as the substituent of the optionally substituted cycloalkenyl group, those such as the above-mentioned substituent in the "optionally substituted alkyl group" as the substituent in the same number can be used.

The heterocyclic group of the "optionally substituted heterocyclic group" as the above-mentioned substituent includes, for example, an aromatic heterocyclic group, a saturated or unsaturated non-aromatic heterocyclic group (an aliphatic heterocyclic group), etc., containing at least one (preferably, 1 to 4, further preferably, 1 or 2) hetero atoms of 1 to 3 kinds (preferably, 1 or 2 kinds) selected from an oxygen atom, a sulfur atom and a nitrogen atom, etc. as a ring-constituting atom (a ring atom).
Here, the "aromatic heterocyclic group" includes, for example, a 5- or 6-membered monocyclic aromatic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl, and, for example, a 8 to 12-membered fused polycyclic aromatic heterocyclic group such as benzofuranyl, isobenzofuranyl, benzo[b]thienyl, indolyl, isoindolyl, 1H-indazolyl, benzindazolyl, benzoxazolyl, 1,2-benzoisoxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.

Here, the "non-aromatic heterocyclic group" includes, for example, a 3- to 8-membered (preferably, 5- or 6-membered) saturated or unsaturated (preferably, saturated) non-aromatic heterocyclic group (aliphatic heterocyclic group) such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, etc., or non-aromatic heterocyclic group in which the double bonds of the above-mentioned monocyclic aromatic heterocyclic group or the fused polycyclic aromatic heterocyclic group are saturated partially or completely such as 1,2,3,4-tetrahydroquinolyl and 1,2,3,4-tetrahydroisoquinolyl, etc.
The substituent which the "optionally substituted heterocyclic group" as the substituent may have, includes a lower alkyl group (e.g., C₁₋₆ alkyl such as methyl, ethyl, propyl, etc.), a lower alkenyl group (e.g., C₂₋₆ alkenyl such as vinyl, allyl, etc.), a lower alkynyl group (e.g., C₂₋₆ alkynyl such as ethynyl, propargyl, etc.), an acyl group (e.g., C₁₋₆ alkanoyl such as formyl, acetyl, propionyl, pivaloyl, benzoyl, etc.), an optionally substituted amino group, an optionally substituted hydroxy group, a halogen atom (e.g., fluorine, chlorine, bromine, iodine, etc., preferably, chlorine, bromine, etc.), an optionally substituted imidoyl group, an optionally substituted amidino group, etc. These optional substituents may exist in the number of 1 to 5 (preferably, 1 to 3) at the substitutable positions.
The "optionally substituted amino group", the "optionally substituted hydroxy group", the "optionally substituted imidoyl group" and the "optionally substituted amidino group", which the "optionally substituted heterocyclic group" as the substituent may have, include those such as the "optionally substituted amino group", the "optionally substituted hydroxy group", the "optionally substituted imidoyl group" and the "optionally substituted amidino group" as the below-described substituent.

The substituent of the "optionally substituted amino group", the "optionally substituted imidoyl group", the "optionally substituted amidino group", the "optionally substituted hydroxy group" or the "optionally substituted thiol group" as the above-mentioned substituent, includes, for example, a lower alkyl group (e.g., C₁₋₆ alkyl such as methyl, ethyl, propyl, isopropyl, butyl, isobutyl, tert-butyl, pentyl, hexyl, etc.) optionally substituted with a substituent selected from optionally halogenated C₁₋₆ alkoxy (e.g., methoxy, ethoxy, trifluoromethoxy, 2,2,2-trifluoroethoxy, trichloromethoxy, 2,2,2-trichloroethoxy, etc.) and a C₇₋₁₁ alkylaryl group (e.g., o-tolyl, m-tolyl, p-tolyl, xylyl, mesityl, etc., preferably, C₁₋₅ alkyl-phenyl, etc.), an acyl group (C₁₋₆ alkanoyl (e.g., formyl, acetyl, propionyl and pivaloyl, etc.), benzoyl, a C₁₋₆ alkylsulfonyl (e.g., methanesulfonyl, etc.), benzenesulfonyl, etc.), an optionally halogenated C₁₋₆ alkoxycarbonyl group (e.g., methoxycarbonyl, ethoxycarbonyl, trifluoromethoxycarbonyl, 2,2,2-trifluoroethoxycarbonyl, trichloromethoxycarbonyl, 2,2,2-trichloroethoxycarbonyl, etc.), a C₁₋₆ alkoxycarbonyl group optionally substituted with a phenyl group (e.g., benzyloxycarbonyl, etc.), aryl (e.g., C₆₋₁₀ aryl such as phenyl, 1-naphthyl, 2-naphthyl, etc.), aralkyl (e.g., C₇₋₁₀ aralkyl such as benzyl and phenethyl, preferably, phenyl-C₁₋₄ alkyl, etc.), arylalkenyl (e.g., C₈₋₁₀ arylalkenyl such as cinnamyl, preferably, phenyl-C₂₋₄ alkenyl, etc.), a heterocyclic group (those such as the "heterocyclic group" in the "optionally substituted heterocyclic group" as the above-mentioned substituent, preferably, pyridyl, further preferably, 4-pyridyl, etc.), etc. These optional substituents may exist at the substitutable positions in the number of 1 to 3.

Furthermore, the "amino group" of the "optionally substituted amino group" as the above-mentioned substituent may be substituted with an optionally substituted imidoyl group (e.g., a C₁₋₆ alkylimidoyl (e.g., formylimidoyl, acetylimidoyl, etc.), a C₁₋₆ alkoxyimidoyl, a C₁₋₆ alkylthioimidoyl, amidino, etc.), an amino group optionally substituted with 1 or 2 C₁₋₆ alkyl groups, etc. These optional substituents may exist at the substitutable positions in the number of 1 or 2. Furthermore, the two substituents may form a cyclic amino group together with the nitrogen atom, and in such case, the cyclic amino group includes, for example, 3- to 8-membered (preferably, 5- or 6-membered) cyclic amino such as 1-azetidinyl, 1-pyrrolidinyl, piperidino, thiomorpholino, morpholino, 1-piperazinyl and 1-piperazinyl optionally having lower alkyl (e.g., C₁₋₆ alkyl such as methyl, ethyl, propyl, isopropyl, butyl, t-butyl, pentyl and hexyl, etc.), aralkyl (e.g., C₇₋₁₀ aralkyl such as benzyl, phenethyl, etc.), aryl (e.g., C₆₋₁₀ aryl such as phenyl, 1-naphthyl, 2-naphthyl, etc.), etc. at the position 4, 1-pyrrolyl, 1-imidazolyl, etc.

The alkylsulfinyl group of the "optionally substituted alkylsulfinyl group" as the above-mentioned substituent includes C₁₋₆ alkylsulfinyl such as methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl and hexylsulfinyl. Here, the substituent of the alkylsulfinyl includes those such as the above-mentioned substituent in the "optionally substituted alkyl" as the substituent in the same number. The "optionally esterified or amidated carboxyl group" as the above-mentioned substituent includes a carboxyl group, alkoxycarbonyl, aryloxycarbonyl, aralkyloxycarbonyl, carbamoyl, N-monosubstituted carbamoyl and N,N-disubstituted carbamoyl.
Here, the "alkoxycarbonyl" includes, for example, C₁₋₆ alkoxycarbonyl (lower alkoxycarbonyl) such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl, etc., among these preferably, C₁₋₃ alkoxycarbonyl such as methoxycarbonyl, ethoxycarbonyl and propoxycarbonyl, etc. The "lower alkoxycarbonyl" may have a substituent, and the substituent includes a hydroxy group, an optionally substituted amino group [for example, the amino group may have 1 or 2 substituents, such as a lower alkyl group (e.g., C₁₋₆ alkyl such as methyl, ethyl, propyl, isopropyl, butyl, isobutyl, tert-butyl, pentyl, hexyl, etc., preferably, methyl, ethyl, etc.) optionally substituted with 1 to 5 halogen atoms (e.g., fluorine, chlorine, bromine, iodine, etc.), an acyl group (e.g., C₁₋₆ alkanoyl such as formyl, acetyl, propionyl and pivaloyl, benzoyl, etc.), a carboxyl group and a C₁₋₆ alkoxycarbonyl.], a halogen atom (e.g., fluorine, chlorine, bromine, iodine, etc.), a nitro group, a cyano group, a lower alkoxy group (e.g., C₁₋₆ alkoxy such as methoxy, ethoxy, n-propoxy, isopropoxy, n-butoxy, isobutoxy, sec-butoxy and tert-butoxy, etc., preferably, methoxy, ethoxy, etc.) optionally substituted with 1 to 5 halogen atoms (e.g., fluorine, chlorine, bromine, iodine, etc.), etc. Furthermore, these substituents may be the same or different and the number of the substituent is preferably 1, 2 or 3 (more preferably 1 or 2).

Here, the "aryloxycarbonyl" is preferably, for example, C₆₋₁₄ aryloxycarbonyl such as phenoxycarbonyl, 1-naphthoxycarbonyl, 2-naphthoxycarbonyl, 1-phenanthoxycarbonyl, etc. The "aryloxycarbonyl" may have a substituent, and the substituent includes those such as the above-mentioned substituents in the "alkoxycarbonyl" as the substituent in the same number.
Here, the "aralkyloxycarbonyl" is preferably, for example, C₇₋₁₄ aralkyloxycarbonyl such as benzyloxycarbonyl, phenethyloxycarbonyl, etc. (preferably, C₆₋₁₀ aryl-C₁₋₄ alkoxycarbonyl, etc.). The "aralkyloxycarbonyl" may have a substituent, and the substituent includes those such as the above-mentioned substituents in the "alkoxycarbonyl" as the substituent in the same number.
Here, the "N-monosubstituted carbamoyl" includes, for example, lower alkyl (e.g., C₁₋₆ alkyl such as methyl, ethyl, propyl, isopropyl, butyl, isobutyl, tert-butyl, pentyl, hexyl, etc.), lower alkenyl (e.g., C₂₋₆ alkenyl such as vinyl, allyl, isopropenyl, propenyl, butenyl, pentenyl, hexenyl, etc.), cycloalkyl (e.g., C₃₋₆ cycloalkyl such as cyclopropyl, cyclobutyl, cyclopentyl and cyclohexyl, etc.), aryl (e.g., C₆₋₁₀ aryl such as phenyl, 1-naphthyl, 2-naphthyl, etc.), aralkyl (e.g., C₇₋₁₀ aralkyl such as benzyl and phenethyl, preferably, phenyl-C₁₋₄ alkyl, etc.), arylalkenyl (e.g., C₈₋₁₀ arylalkenyl such as cinnamyl, preferably, phenyl-C₂₋₄ alkenyl, etc.), a heterocyclic group (e.g., those such as the "heterocyclic group" in the "optionally substituted heterocyclic group" as the above-mentioned substituent, etc.), etc. The lower alkyl, the lower alkenyl, the cycloalkyl, the aryl, the aralkyl, the arylalkenyl and the heterocyclic group may have a substituent, and the substituent includes those such as the above-mentioned substituents in the "alkoxycarbonyl" as the substituent in the same number.

Here, the "N,N-disubstituted carbamoyl" means a carbamoyl group having two substituents on the nitrogen atom. Examples of one of the two substituents include those such as the above-mentioned substituent in the "N-monosubstituted carbamoyl" as the substituent, and examples of the other substituent include, for example, lower alkyl (e.g., C₁₋₆ alkyl such as methyl, ethyl, propyl, isopropyl, butyl, tert-butyl, pentyl, hexyl, etc.), C₃₋₇ cycloalkyl (e.g., cyclopropyl, cyclobutyl, cyclopentyl, cyclohexyl, etc.), C₇₋₁₀ aralkyl (e.g., benzyl and phenethyl, etc., preferably, phenyl-C₁₋₄ alkyl, etc.), etc. Furthermore, the two substituents may form a cyclic amino together with the nitrogen atom, and in such case, the cyclic aminocarbamoyl includes, for example, a 3- to 8-membered (preferably, a 5- or 6-membered) cyclic aminocarbonyl such as 1-azetidinylcarbonyl, 1-pyrrolidinylcarbonyl, piperidinocarbonyl, morpholinocarbonyl, 1-piperazinylcarbonyl, and 1-piperazinylcarbonyl optionally having lower alkyl (e.g., C₁₋₆ alkyl such as methyl, ethyl, propyl, isopropyl, butyl, tert-butyl, pentyl, hexyl, etc.), aralkyl (e.g., C₇₋₁₀ aralkyl such as benzyl, phenethyl, etc.), aryl (e.g., C₆₋₁₀ aryl such as phenyl, 1-naphthyl, 2-naphthyl, etc.), etc. at the position 4, etc.

The substituent of the "optionally substituted thiocarbamoyl group" and the "optionally substituted sulfamoyl group" as the above-mentioned substituent includes those such as the substituent of the "N-monosubstituted carbamoyl" or the "N,N-disubstituted carbamoyl" in the "optionally esterified or amidated carboxyl group" as the above-mentioned substituent.
The "sulfonic acid-derived acyl" as the above-mentioned substituent includes, for example, those in which the one substituent on the nitrogen atom of the above-mentioned "N-monosubstituted carbamoyl" is bonded to sulfonyl, etc., preferably, acyl such as C₁₋₆ alkylsulfonyl such as methanesulfonyl and ethanesulfonyl.
The "carboxylic acid-derived acyl" as the substituent includes a hydrogen atom or those in which the one substituent on the nitrogen atom of the above-mentioned "N-monosubstituted carbamoyl" is bonded to carbonyl, preferably, acyl such as C₁₋₆ alkanoyl such as formyl, acetyl, propionyl and pivaloyl, and benzoyl.
The "C₁₋₄ alkylenedioxy group" as the substituent includes a methylenedioxy group, an ethylenedioxy group, a propylenedioxy group, a butylenedioxy group, etc., which may be substituted on the same carbon or different carbons.

The substituent in the "optionally substituted C₁₋₆ alkyl group" for R⁶ and the "substituent on Ring A or Ring B except for R^{a}, R², R^{3'}, R⁴ and R^{5'}" includes those such as the above-mentioned substituent used in the "optionally substituted alkyl group" as the substituent in the same number.
The substituent in the "optionally substituted C₁₋₆ alkoxy group" for the "substituent on Ring A or Ring B except for R^{a}, R², R^{3'} R⁴ and R^{5'}" includes those such as the above-mentioned substituent used in the "optionally substituted alkyl group" as the substituent in the same number.
The substituent in the "optionally substituted alkoxy group" for R^{a} includes those such as the above-mentioned substituent used in the "optionally substituted alkyl group" as the substituent in the same number.
The substituent in the "optionally substituted acyl group" for R¹, R², R³, R^{3'}, R⁴, R⁵, R^{5'} and R⁷ includes those such as the above-mentioned substituent used in the "optionally substituted alkyl group" as the substituent in the same number.
The substituent in the "optionally substituted C₁₋₆ acyl group" for the "substituent on Ring A or Ring B except for R^{a}, R², R^{3'}, R⁴ and R^{5'}" includes those such as the above-mentioned substituent used in the "optionally substituted alkyl group" as the substituent in the same number.
The substituent in the "optionally substituted C₁₋₆ alkoxyimino group" in the "carbon atom substituted with an optionally substituted C₁₋₆ alkoxyimino group" for Z¹ and Z² and the "optionally substituted C₁₋₆ alkoxyimino group" for the "substituent on Ring A or Ring B except for R^{a}, R², R^{3'}, R⁴ and R^{5'}" includes those such as the above-mentioned substituent used in the "optionally substituted alkyl group" as the substituent in the same number.
The substituent in the "optionally substituted C₁₋₄ alkylenedioxy group" for the "substituent on Ring A or Ring B except for R^{a}, R², R^{3'}, R⁴ and R^{5'}" includes those such as the above-mentioned substituent used in the "optionally substituted alkyl group" as the substituent in the same number.

Ring A and Ring B may be substituted.
The substituent in the "optionally substituted carbon atom" represented by X¹ and X² includes 1 or 2 of those such as the above-mentioned substituent in the "optionally substituted hydrocarbon group" for R², R³, R^{3'}, R⁴, R⁵ or R^{5'}. Here, when the "optionally substituted carbon atom" has no substituent, the carbon atom has 1 or 2 of hydrogen atom, and when the "optionally substituted carbon atom" has one substituent, the carbon atom has 0 or 1 of a hydrogen atom in addition to the substituent.
The substituent in the "optionally substituted nitrogen atom" for Y² or Y²¹ includes those such as the substituent in the "optionally substituted amino group" as the substituent in the definition of the above-mentioned "substituent on Ring A or Ring B except for R^{a}, R², R^{3'}, R⁴ and R^{5'}". Here, when the "optionally substituted nitrogen atom" has no substituent, the nitrogen atom has 0 or 1 of a hydrogen atom.

W and W¹ represent a nitrogen atom or a group represented by the formula CR^{a} (wherein the symbol is as defined above), provided that when Ring A represents an optionally substituted benzene ring, W represents a group represented by the formula CR^{a} (wherein the symbol is as defined above).
Y¹ represents a group represented by the formula CR²R³ (wherein each symbol is as defined above), and Y² represents a group represented by a group represented by the formula CR⁴R⁵ (wherein each symbol is as defined above), a nitrogen atom, an oxygen atom or a group represented by the formula S(O)ₘ (wherein each symbol is as defined above).
Y¹¹ represents a group represented by the formula CR²R^{3'} (wherein each symbol is as defined above), and Y²¹ represents 1) when W is a nitrogen atom, a group represented by the formula CR⁴R^{5'} (wherein each symbol is as defined above), a nitrogen atom, an oxygen atom or a group represented by the formula S(O)ₘ (wherein each symbol is as defined above), or 2) when W is a group represented by the formula CR^{a} (wherein the symbol is as defined above), a group represented by the formula CR⁴R^{5'} (wherein each symbol is as defined above) or a nitrogen atom (provided that when Y²¹ is a nitrogen atom and W is a group represented by the formula CR^{a} (wherein the symbol is as defined above), the bond between CR^{a} and Y²¹ is a double bond).

The compound (I') to be used in the present invention is preferably a compound represented by the formula

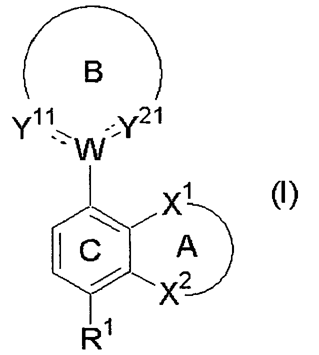

wherein each symbol is as defined above, or a salt thereof, more preferably, a compound represented by the formula

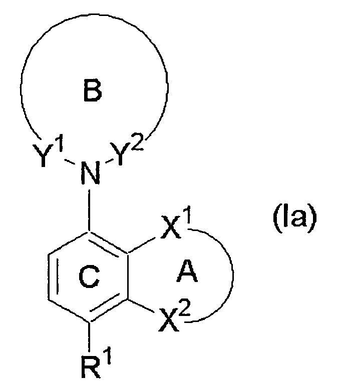

wherein each symbol is as defined above, or a salt thereof, further more preferably a compound represented by the formula

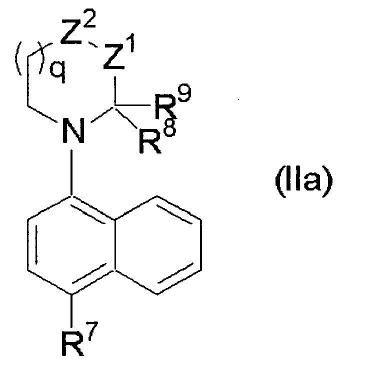

wherein each symbol is as defined above, or a salt thereof, or a compound represented by the formula

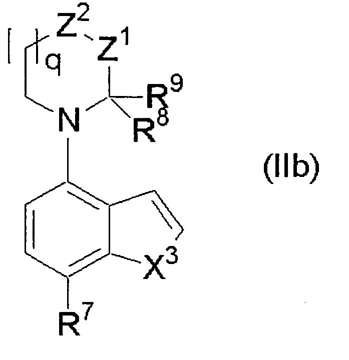

wherein each symbol is as defined above, or a salt thereof.
In compound (IIa), R⁷ is preferably a cyano group, a nitro group, a halogen atom, a C₁₋₆ alkanoyl group optionally substituted by a halogen atom, a carboxyl group or a C₁₋₆ alkyl group substituted by 1 to 5 halogen atoms and the like.
In compound (IIb), R⁷ is preferably a cyano group and the like.

In compound (I'), preferable embodiments are as follows. Ring A is
(i) a C₅₋₈ alicyclic hydrocarbon (e.g., cycloheptane, cyclohexane, cyclopentene etc.),
(ii) a C₆₋₈ aromatic hydrocarbon (e.g., benzene etc.),
(iii) a 5- or 6-membered aromatic monocyclic heterocycle containing, as ring-constituting atom, 1 to 4 of 1 to 3 kinds of hetero atoms selected from an oxygen atom, a sulfur atom and a nitrogen atom (e.g., furan, thiophene etc.), or
(iv) a 5- to 8-membered saturated non-aromatic heterocycle containing, as ring-constituting atom, 1 to 4 of 1 to 3 kinds of hetero atoms selected from an oxygen atom, a sulfur atom and a nitrogen atom (e.g., tetrahydrofuran etc.),
   each of which is optionally substituted by substituent(s) selected from the group consisting of (1) a hydrogen atom, (2) a C₁₋₆ alkyl group (e.g., methyl, ethyl etc.), (3) a hydroxyl group and (4) an oxo group,
   Ring B is

(i) a 5- to 8-membered saturated or unsaturated non-aromatic heterocycle containing, as ring-constituting atom, 1 to 4 of 1 to 3 kinds of hetero atoms selected from an oxygen atom, a sulfur atom and a nitrogen atom (e.g., pyrrolidine, piperidine, morpholine, thiomorpholine, perhydroazepine, piperazine, pyrazoline, isoxazoline, pyrazolidine, 1,2-oxazinan, 1,4-oxazepane etc.),
(ii) a non-aromatic heterocycle formed by the fusion of a 5- or 6-membered non-aromatic monocyclic heterocyclic group containing, as ring-constituting atom, 1 to 4 of 1 to 3 kinds of hetero atoms selected from an oxygen atom, a sulfur atom and a nitrogen atom, and a benzene ring (e.g., 1,2,3,4-tetrahydroisoquinoline, isoindoline etc.), or
(iii) a C₅₋₈ alicyclic hydrocarbon (e.g., cyclopentane, cyclopentene etc.), each of which is, except for R^{a}, R², R^{3'}, R⁴ and R^{5'}, further optionally substituted by 1 to 6 groups selected from the group consisting of (1) a hydrogen atom, (2) a C₁₋₆ alkyl group (e.g., methyl, ethyl, propyl, isopropyl, etc.) optionally substituted by substituent(s) selected from (i) a hydroxyl group optionally substituted by substituent(s) selected from a C₆₋₁₀ aryl optionally substituted by a cyano (e.g., phenyl, naphthyl, 4-cyano-1-naphthyl etc.) and a carbamoyl, (ii) a C₁₋₆ alkoxy group (e.g., methoxy etc.), (iii) a C₁₋₆ alkoxycarbonyl group (e.g., methoxycarbonyl, ethoxycarbonyl etc.), (iv) a carboxyl group and
(v) an amino group optionally substituted by substituent(s) selected from a C₁₋₆ alkylsulfonyl (e.g., methanesulfonyl etc.) and a C₁₋₆ alkoxycarbonyl group (e.g., tert-butoxycarbonyl etc.), (3) a C₂₋₆ alkenyl group (e.g., vinyl etc.), (4) a C₆₋₁₄ aryl group (e.g., phenyl etc.), (5) an amino group optionally substituted by substituent(s) selected from (i) a C₁₋₆ alkyl (e.g., methyl etc.), (ii) a C₁₋₆ alkanoyl (e.g., acetyl etc.), (iii) a C₁₋₆ alkoxycarbonyl (e.g., tert-butoxycarbonyl etc.) and (iv) a C₁₋₆ alkylsulfonyl (e.g., methanesulfonyl etc.), (6) a hydroxyl group optionally substituted by a substituent selected from (i) a C₁₋₆ alkyl (e.g., methyl etc.), (ii) a C₆₋₁₀ aryl (e.g., phenyl etc.) and (iii) a C₇₋₁₀ aralkyl (e.g., benzyl etc.), (7) a carboxyl, (8) a C₁₋₆ alkoxycarbonyl (e.g., methoxycarbonyl, ethoxycarbonyl etc.), (9) a carbamoyl, (10) a halogen atom (e.g., fluorine atom etc.), (11) a cyano group, (12) a hydroxyimino group, (13) an oxo group and (14) a C₁₋₄ alkylenedioxy group (e.g., ethylenedioxy etc.),
   Ring C is a benzene ring further optionally substituted by substituent(s) selected from a hydrogen atom and a halogen atom (e.g., bromine atom etc.),
   X¹ represents a carbon atom optionally substituted by substituent(s) selected from the group consisting of (1) a hydrogen atom, (2) a hydroxyl group and (3) an oxo groups group, X² is a carbon atom, an oxygen atom or a group represented by the formula S(O)ₖ (wherein k is 0, 1 or 2),
   W¹ is a nitrogen atom or
   a group represented by the formula CR^{a} (wherein R^{a} is a bond, a hydrogen atom or a hydroxyl group),
   Y¹¹ is CH₂,
   Y²¹ is

1) when W¹ is a nitrogen atom,
   (i) a group represented by the formula CR⁴R^{5'} (wherein R⁴ is (1) a hydrogen atom, (2) a cyano group, (3) a carbamoyl group, (4) a C₁₋₆ alkyl (e.g., methyl, ethyl, propyl, isopropyl etc.) group optionally substituted by substituent(s) selected from (i) a hydroxy, (ii) a C₁₋₆ alkoxy (e.g., methoxy etc.) and (iii) a C₆₋₁₀ aryloxy optionally substituted by a cyano group (e.g., 4-cyano-1-naphthyloxy etc.), or (5) a C₂₋₆ alkenyl group (e.g., vinyl etc.), and R^{5'} is a hydrogen atom or a C₁₋₆ alkyl group),
   (ii) a nitrogen atom optionally substituted by a hydrogen atom or a C₁₋₆ alkyl group, or
   (iii) an oxygen atom, and
2) when W¹ is a group represented by the formula CR^{a} (wherein the symbol is as defined above),
   a group represented by the formula CR⁴R^{5'} (wherein R⁴ is a C₁₋₆ alkyl group, and R^{5'} is a bond or a hydrogen atom) or a nitrogen atom (provided that when Y²¹ is a nitrogen atom and W¹ is a group represented by the formula CR^{a} (wherein the symbol is as defined above), the bond between CR^{a} and Y²¹ is a double bond), and when Ring B is a further optionally substituted non-aromatic heterocycle formed by the fusion of a 5- or 6-membered non-aromatic monocyclic heterocyclic group and a benzene ring, CR² for Y¹¹ or CR⁴ or the nitrogen atom for Y²¹ may constitute a part of Ring B,
   R¹ is a cyano group, a nitro group, a halogen atom (e.g., bromine atom etc.), a C₁₋₆ alkanoyl group optionally substituted by a halogen atom (e.g., acetyl, trifluoroacetyl etc.), a carboxyl group or a C₁₋₆ alkyl group substituted by 1 to 5 halogen atoms (e.g., trifluoromethyl etc.), and
   the formula

-̅ -̅ -̅

is a single bond or a double bond.

In compound (I), preferable embodiments are as follows. Ring A is
(i) a C₅₋₈ alicyclic hydrocarbon (e.g., cycloheptane, cyclohexane, cyclopentene etc.),
(ii) a C₆₋₈ aromatic hydrocarbon (e.g., benzene etc.),
(iii) a 5- or 6-membered aromatic monocyclic heterocycle containing, as ring-constituting atom, 1 to 4 of 1 to 3 kinds of hetero atoms selected from an oxygen atom, a sulfur atom and a nitrogen atom (e.g., furan, thiophene etc.), or
(iv) a 5- to 8-membered saturated non-aromatic heterocycle containing, as ring-constituting atom, 1 to 4 of 1 to 3 kinds of hetero atoms selected from an oxygen atom, a sulfur atom and a nitrogen atom (e.g., tetrahydrofuran etc.),
   each of which is optionally substituted by substituent(s) selected from the group consisting of (1) a hydrogen atom, (2) a C₁₋₆ alkyl group (e.g., methyl, ethyl etc.), (3) a hydroxyl group and (4) an oxo group,
   Ring B is

(i) a 5- to 8-membered saturated or unsaturated non-aromatic heterocycle containing, as ring-constituting atom, 1 to 4 of 1 to 3 kinds of hetero atoms selected from an oxygen atom, a sulfur atom and a nitrogen atom (e.g., pyrrolidine, piperidine, morpholine, thiomorpholine, perhydroazepine, piperazine, pyrazoline, isoxazoline, pyrazolidine, 1,2-oxazinan, 1,4-oxazepane etc.),
(ii) a non-aromatic heterocycle formed by the fusion of a 5- or 6-membered non-aromatic monocyclic heterocyclic group containing, as ring-constituting atom, 1 to 4 of 1 to 3 kinds of hetero atoms selected from an oxygen atom, a sulfur atom and a nitrogen atom, and a benzene ring (e.g., 1,2,3,4-tetrahydroisoquinoline, isoindoline etc.), or
(iii) a C₅₋₈ alicyclic hydrocarbon (e.g., cyclopentane, cyclopentene etc.),
   each of which is, except for R^{a}, R², R^{3'}, R⁴ and R^{5'}, further optionally substituted by 1 to 6 groups selected from the group consisting of (1) a hydrogen atom, (2) a C₁₋₆ alkyl group (e.g., methyl, ethyl, propyl, isopropyl, etc.) optionally substituted by substituent(s) selected from (i) a hydroxyl group optionally substituted by substituent(s) selected from a C₆₋₁₀ aryl optionally substituted by a cyano (e.g., phenyl, naphthyl, 4-cyano-1-naphthyl etc.) and a carbamoyl, (ii) a C₁₋₆ alkoxy group (e.g., methoxy etc.), (iii) a C₁₋₆ alkoxycarbonyl group (e.g., methoxycarbonyl, ethoxycarbonyl etc.), (iv) a carboxyl group and
(v) an amino group optionally substituted by substituent(s) selected from a C₁₋₆ alkylsulfonyl (e.g., methanesulfonyl etc.) and a C₁₋₆ alkoxycarbonyl group (e.g., tert-butoxycarbonyl etc.), (3) a C₂₋₆ alkenyl group (e.g., vinyl etc.), (4) a C₆₋₁₄ aryl group (e.g., phenyl etc.), (5) an amino group optionally substituted by substituent(s) selected from (i) a C₁₋₆ alkyl (e.g., methyl etc.), (ii) a C₁₋₆ alkanoyl (e.g., acetyl etc.), (iii) a C₁₋₆ alkoxycarbonyl (e.g., tert-butoxycarbonyl etc.) and (iv) a C₁₋₆ alkylsulfonyl (e.g., methanesulfonyl etc.), (6) a hydroxyl group optionally substituted by a substituent selected from (i) a C₁₋₆ alkyl (e.g., methyl etc.), (ii) a C₆₋₁₀ aryl (e.g., phenyl etc.) and (iii) a C₇₋₁₀ aralkyl (e.g., benzyl etc.), (7) a carboxyl, (8) a C₁₋₆ alkoxycarbonyl(e.g., methoxycarbonyl, ethoxycarbonyl etc.), (9) a carbamoyl, (10) a halogen atom (e.g., fluorine atom etc.), (11) a cyano group, (12) a hydroxyimino group, (13) an oxo group and (14) a C₁₋₄ alkylenedioxy group (e.g., ethylenedioxy etc.), Ring C is a benzene ring further optionally substituted by substituent(s) selected from a hydrogen atom and a halogen atom (e.g., bromine atom etc.),
   X¹ is a carbon atom optionally substituted by substituent(s) selected from the group consisting of (1) a hydrogen atom, (2) a hydroxyl group and (3) an oxo group,
   X² is a carbon atom, an oxygen atom or a group represented by the formula S(O)ₖ (wherein k is 0, 1 or 2).
   W is a nitrogen atom, or
   when Ring A is an optionally substituted benzene ring, a group represented by the formula CR^{a} (wherein R^{a} is a bond, a hydrogen atom or a hydroxyl group),
   Y¹¹ is CH₂,
   Y²¹ is

1) when W is a nitrogen atom,
   (i) a group represented by the formula CR⁴R^{5'} (wherein R⁴ is (1) a hydrogen atom, (2) a cyano group, (3) a carbamoyl group, (4) a C₁₋₆ alkyl group (e.g., methyl, ethyl, propyl, isopropyl etc.) optionally substituted by substituent(s) selected from (i) a hydroxy, (ii) a C₁₋₆ alkoxy (e.g., methoxy etc.) and (iii) a C₆₋₁₀ aryloxy optionally substituted by a cyano group (e.g., 4-cyano-1-naphthyloxy etc.), or (5) a C₂₋₆ alkenyl group (e.g., vinyl etc.), and R^{5'} is a hydrogen atom or a C₁₋₆ alkyl group),
   (ii) a nitrogen atom optionally substituted by a hydrogen atom or a C₁₋₆ alkyl group, or
   (iii) an oxygen atom, and
2) when W is a group represented by the formula CR^{a} (wherein the symbol is as defined above),
   a group represented by the formula CR⁴R^{5'} (wherein R⁴ is a C₁₋₆ alkyl group, and R^{5'} is a bond or a hydrogen atom) or a nitrogen atom (provided that when Y²¹ is a nitrogen atom and W is a group represented by the formula CR^{a} (wherein the symbol is as defined above), the bond between CR^{a} and Y²¹ is a double bond), and when Ring B is a further optionally substituted non-aromatic heterocycle formed by the fusion of a 5- or 6-membered non-aromatic monocyclic heterocyclic group and a benzene ring, CR² for Y¹¹ or CR⁴ or the nitrogen atom for Y²¹ may constitute a part of Ring B,
   R¹ is a cyano group, a nitro group, a halogen atom (e.g., bromine atom etc.), a C₁₋₆ alkanoyl group optionally substituted by a halogen atom (e.g., acetyl, trifluoroacetyl etc.), a carboxyl group or a C₁₋₆ alkyl group substituted by 1 to 5 halogen atoms (e.g., trifluoromethyl etc.), and
   the formula

-̅ -̅ -̅

is a single bond or a double bond.

In compound (IIa), preferable embodiments are as follows. R⁷ is a cyano group, a nitro group, a halogen atom (e.g., bromine atom etc.), a C₁₋₆ alkanoyl group optionally substituted by a halogen atom (e.g., acetyl, trifluoroacetyl etc.), a carboxyl group or a C₁₋₆ alkyl group substituted by 1 to 5 halogen atoms (e.g., trifluoromethyl etc.),
R⁸ and R⁹ are the same or different and each is
(1) a hydrogen atom,
(2) a cyano group,
(3) a C₁₋₆ alkyl group (e.g., methyl, ethyl, propyl, isopropyl etc.) optionally substituted by a hydroxyl group or a C₁₋₆ alkoxy group (e.g., methoxy etc.), or
(4) a carbamoyl group,
   q is 0, 1 or 2,
   Z¹ is (1) a carbonyl group, (2) a carbon atom substituted by a hydroxyimino group, or
(3) a group represented by the formula

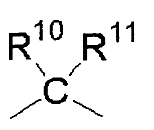

(wherein R¹⁰ and R¹¹ are the same or different and each is
(1) a hydrogen atom,
(2) a halogen atom (e.g., fluorine atom etc.),
(3) a cyano group,
(4) a hydroxyl group,
(5) a C₁₋₆ alkyl group (e.g., methyl, ethyl, propyl etc.) optionally substituted by substituent(s) selected from (i) a hydroxyl group and (ii) a C₁₋₆ alkoxy group (e.g., methoxy),
(6) a C₁₋₆ alkoxy group (e.g., methoxy etc.),
(7) an amino group optionally substituted by substituent(s) selected from a C₁₋₆ alkyl group and a C₁₋₆ acyl group,
(8) a carboxyl group, or
(9) a C₁₋₆ alkoxycarbonyl group (e.g., methoxycarbonyl, ethoxycarbonyl etc.),
Z² is
(1) an oxygen atom,
(2) a sulfur atom,
(3) SO,
(4) SO₂,
(5) a carbonyl group,
(6) a carbon atom substituted by a hydroxyimino group,
(7) a nitrogen atom substituted by a hydrogen atom or a C₁₋₆ alkyl group (e.g., methyl etc.),
(8) a carbon atom substituted by a C₁₋₄ alkylenedioxy group, or
(9) a group represented by the formula

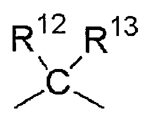

(wherein R¹² and R¹³ are the same or different and each is
(1) a hydrogen atom,
(2) a hydroxyl group,
(3) a C₁₋₆ alkyl group (e.g., methyl, ethyl, propyl, isopropyl etc.) optionally substituted by substituent(s) selected from a hydroxyl group and a C₁₋₆ alkoxy group (e.g., methoxy etc.),
(4) a C₁₋₆ alkoxy group (e.g., methoxy etc.), or
(5) a carbamoyl group).

In compound (IIb), preferable embodiments are as follows.
X³ is a sulfur atom or an oxygen atom,
R⁷ is a cyano group,
R⁸ and R⁹ are the same or different and each is (1) a hydrogen atom or (2) a C₁₋₆ alkyl group (e.g., methyl etc.),
q is 0 or 1,
Z¹ is a group represented by the formula

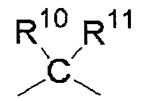

(wherein R¹⁰ and R¹¹ are the same or different and each is
(1) a hydrogen atom, (2) a halogen atom (e.g., fluorine atom etc.), (3) a hydroxyl group or (4) a C₁₋₆ alkyl group (e.g., methyl, ethyl etc.) optionally substituted by substituent(s) selected from a hydroxyl group and a C₁₋₆ alkoxy group (e.g., methoxy etc.)),
   Z² is a group represented by the formula

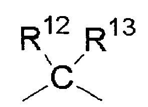

(wherein R¹² and R¹³ are the same or different and each is
(1) a hydrogen atom, (2) a halogen atom (e.g., fluorine atom etc.), (3) a hydroxyl group or (4) a C₁₋₆ alkyl group (e.g., ethyl etc.) optionally substituted by substituent(s) selected from a hydroxyl group and a C₁₋₆ alkoxy group (e.g., methoxy etc.)).

The compound to be used in the present invention is preferably a compound represented by the aforementioned formula (I) and the like and, specifically, the following compounds 1 - 161 are preferably used.
Compound 1: 4-(1-pyrrolidinyl)-1-naphtonitrile
Compound 2: 4-(1-pyrrolidinyl)-1-naphthoic acid
Compound 3: 4-(1-piperidinyl)-1-naphtonitrile
Compound 4: 4-(1-piperidinyl)-1-naphtonitrile hydrochloride
Compound 5: 1-(4-bromo-1-naphthyl)piperidine
Compound 6: 1-[4-(trifluoromethyl)-1-naphthyl]piperidine
Compound 7: 4-(4-morpholinyl)-1-naphtonitrile
Compound 8: 4-(4-thiomorpholinyl)-1-naphtonitrile
Compound 9: 4-(1-oxide-4-thiomorpholinyl)-1-naphtonitrile
Compound 10: 4-(1-azepanyl)-1-naphtonitrile
Compound 11: 4-(4-hydroxy-1-piperidinyl)-1-naphtonitrile
Compound 12: 4-(1,1-dioxide-4-thiomorpholinyl)-1-naphtonitrile
Compound 13: 4-(1,4-dioxa-8-azaspiro[4,5]deca-8-yl)-1-naphtonitrile
Compound 14: 4-(4-oxo-1-piperidinyl)-1-naphtonitrile
Compound 15: 1-(4-cyano-1-naphthyl)-4-piperidinecarboxamide
Compound 16: 3-bromo-4-(1-piperidinyl)-1-naphtonitrile
Compound 17: 4-(4-methyl-1-piperazinyl)-1-naphtonitrile
Compound 18: 4-(3-hydroxy-1-pyrrolidinyl)-1-naphtonitrile
compound19: 4-[3-(hydroxymethyl)-1-piperidinyl]-1-naphtonitrile
compound 20: 4-[3-(hydroxymethyl)-1-piperidinyl]-1-naphtonitrile hydrochloride
compound 21: 4-[(3S)-3-(hydroxymethyl)-1-piperidinyl]-1-naphtonitrile
compound 22: 4-[(3R)-3-(hydroxymethyl)-1-piperidinyl]-1-naphtonitrile
compound 23: 9-[(3S)-3-(hydroxymethyl)-1-piperidinyl]-1-naphtonitrile hydrochloride
compound 24: 4-[(3R)-3-(hydroxymethyl)-1-piperidinyl]-1-naphtonitrile hydrochloride
compound 25: tert-butyl 1-(4-cyano-1-naphthyl)-3-pyrrolidinylcarbamate
compound 26: 2-bromo-4-(1-piperidinyl)-1-naphtonitrile
compound 27: 4-(3-oxo-1-pyrrolidinyl)-1-naphtonitrile
compound 28: 4-(3-amino-1-pyrrolidinyl)-1-naphtonitrile dihydrochloride
compound 29: 4-(3-methoxy-1-pyrrolidinyl)-1-naphtonitrile
compound 30: 4-[4-(hydroxymethyl)-1-piperidinyl]-1-naphtonitrile
compound 31: ethyl (3S)-1-(4-cyano-1-naphthyl)-3-piperidinecarboxylate
compound 32: ethyl (3R)-1-(4-cyano-1-naphthyl)-3-piperidinecarboxylate
compound 33: (3S)-1-(4-cyano-1-naphthyl)-3-piperidinecarboxylic acid
compound 34: (3R)-1-(4-cyano-1-naphthyl)-3-piperidinecarboxylic acid
compound 35: 4-[2-(hydroxymethyl)-1-piperidinyl]-1-naphtonitrile
compound 36: 7-[3-(hydroxymethyl)-1-piperidinyl]-4-nitro-1-indanone
compound 37: 7-[3-(hydroxymethyl)-1-piperidinyl]-4-nitro-1-indanol
compound 38: [1-(7-nitro-1H-inden-4-yl)-3-piperidinyl]methanol
compound 39: [1-(4-nitro-1H-inden-7-yl)-3-piperidinyl]methanol
compound 40: 4-(3-hydroxy-1-piperidinyl)-1-naphtonitrile
compound 41: 4-(3-methoxy-1-piperidinyl)-1-naphtonitrile
compound 42: N-[1-(4-cyano-1-naphthyl)pyrrolidin-3-yl]acetamide
compound 43: 4-[(2S)-2-(hydroxymethyl)pyrrolidin-1-yl]-1-naphtonitrile hydrochloride
compound 44: 4-[(2R)-2-(hydroxymethyl)pyrrolidin-1-yl]-1-naphtonitrile hydrochloride
compound 45: 4-[(2R)-2-(methoxymethyl)pyrrolidin-1-yl]-1-naphtonitrile hydrochloride
compound 46: 1-(4-cyano-1-naphthyl)-L-prolinamide
compound 47: 4-(2-methylpyrrolidin-1-yl)-1-naphtonitrile hydrochloride
compound 48: 4-(2-methylpyrrolidin-1-yl)-1-naphtonitrile
compound 49: N-[1-(4-cyano-1-naphthyl)pyrrolidin-3-yl]-N-methylacetamide
compound 50: 4-[3-(hydroxymethyl)pyrrolidin-1-yl]-1-naphtonitrile hydrochloride
compound 51: 4-[3-(methoxymethyl)pyrrolidin-1-yl]-1-naphtonitrile
compound 52: cis-4-[4-(hydroxymethyl)-2-methylpyrrolidin-1-yl]-1-naphtonitrile
compound 53: trans-4-[4-(hydroxymethyl)-2-methylpyrrolidin-1-yl]-1-naphtonitrile
compound 54: cis-4-[4-(methoxymethyl)-2-methylpyrrolidin-1-yl]-1-naphtonitrile
compound 55: trans-4-[4-(methoxymethyl)-2-methylpyrrolidin-1-yl]-1-naphtonitrile
compound 56: 4-[(3-hydroxymethyl)-3-methyl-1-piperidinyl]-1-naphtonitrile
compound 57: 4-[(3-methoxymethyl)-3-methyl-1-piperidinyl]-1-naphtonitrile
compound 58: 4-(3,5-dimethyl-1-piperidinyl)-1-naphtonitrile
compound 59: 4-(1-pyrrolidinyl)-5,6,7,8-tetrahydro-1-naphthalenecarbonitrile
compound 60: 4-(1-piperidinyl)-5,6,7,8-tetrahydro-1-naphthalenecarbonitrile
compound 61: 4-[3-(hydroxyimino)-1-pyrrolidinyl]-1-naphtonitrile
compound 62: 4-[3-(hydroxyimino)-1-pyrrolidinyl]-1-naphtonitrile
compound 63: 2,2-dimethyl-4-(1-pyrrolidinyl)-2,3-dihydro-1-benzofuran-7-carbonitrile
compound 64: 4-(2-methyl-3-oxocyclopenta-1-en-1-yl)-1-naphtonitrile
compound 65: 4-(3-hydroxy-2-methylcyclopenta-1-en-1-yl)-1-naphtonitrile
compound 66: 4-(3-methoxy-2-methylcyclopenta-1-en-1-yl)-1-naphtonitrile
compound 67: 4-[4-(methoxymethyl)-1-piperidinyl]-1-naphtonitrile
compound 68: 4-(3-hydroxy-3-methyl-1-piperidinyl)-1-naphtonitrile
compound 69: N-[1-(4-cyano-1-naphthyl)-3-pyrrolidinyl]methanesulfonamide
compound 70: 4-(3,4-dihydro-2(1H)-isoquinolinyl)-1-naphtonitrile
compound 71: tert-butyl [[1-(4-cyano-1-naphthyl)-3-piperidinyl]methyl](methylsulfonyl)carbamate
compound 72: N-[[1-(4-cyano-1-naphthyl)-3-piperidinyl]methyl]methanesulfonamide
compound 73: 4-(1-piperidinyl)-1-benzothiophene-7-carbonitrile
compound 74: 1-(4-cyano-1-naphthyl)-3-piperidinecarboxamide
compound 75: 4-(1,2-oxazinan-2-yl)-1-naphtonitrile
compound 76: 4-(2-methyl-1-pyrrolidinyl)-1-benzothiophene-7-carbonitrile
compound 77: 4-[4-(hydroxymethyl)-4-methyl-1-piperidinyl]-1-naphtonitrile
compound 78: 4-[4-(2-hydroxyethyl)-1-piperidinyl]-1-naphtonitrile
compound 79: 4-[4-(2-methoxyethyl)-1-piperidinyl]-1-naphtonitrile
compound 80: 4-[4-(1-hydroxy-1-methylethyl)-1-piperidinyl]-1-naphtonitrile
compound 81: 4-[(2R)-2-vinyl-1-pyrrolidinyl]-1-naphtonitrile
compound 82: 4-[(2S)-2-vinyl-1-pyrrolidinyl]-1-naphtonitrile
compound 83: 4-[(2S)-2-ethyl-1-pyrrolidinyl]-1-naphtonitrile
compound 84: 4-[(2R)-2-ethyl-1-pyrrolidinyl]-1-naphtonitrile
compound 85: 4-[(2S)-2-methyl-1-pyrrolidinyl]-1-naphtonitrile
compound 86: 4-[(2R)-2-methyl-1-pyrrolidinyl]-1-naphtonitrile
compound 87: 3-[1-(4-cyano-1-naphthyl)-4-piperidinyl]ethyl butyrate
compound 88: 4-[4-(3-hydroxypropyl)-1-piperidinyl]-1-naphtonitrile
compound 89: 2,2,2-trifluoro-1-[4-(2-methyl-1-pyrrolidinyl)-1-naphthyl]ethanone
compound 90: 1-[4-(2-methyl-1-pyrrolidinyl)-1-naphthyl]ethanone
compound 91: 4-(3-oxo-1-pyrazolidinyl)-1-naphtonitrile
compound 92: 4-(3-methoxy-4,5-dihydro-1H-pyrazol-1-yl)-1-naphtonitrile
compound 93: 4-(2-methyl-3-oxo-1-pyrazolidinyl)-1-naphtonitrile
compound 94: 4-[4-(2-hydroxyethyl)-1-piperidinyl]-1-benzothiophene-7-carbonitrile
compound 95: 4-(2-methyl-1-pyrrolidinyl)-1-benzofuran-7-carbonitrile
compound 96: 4-[4-(2-hydroxyethyl)-1-piperidinyl]-1-benzofuran-7-carbonitrile
compound 97: 2-methyl-4-(2-methyl-1-pyrrolidinyl)-1-benzofuran-7-carbonitrile
compound 98: 4-[4-(2-hydroxyethyl)-1-piperidinyl]-2-methyl-1-benzofuran-7-carbonitrile
compound 99: 4-(3-fluoro-1-pyrrolidinyl)-1-naphtonitrile
compound 100: 4-(3-fluoro-1-pyrrolidinyl)-1-benzothiophene-7-carbonitrile
compound 101: 4-[3-(2-hydroxyethyl)-1-pyrrolidinyl]-1-benzothiophene-7-carbonitrile
compound 102: 4-[3-(2-methoxyethyl)-1-pyrrolidinyl]-1-benzothiophene-7-carbonitrile
compound 103: 4-(3,3-difluoro-1-pyrrolidinyl)-1-naphtonitrile
compound 104: 4-(3,3-difluoro-1-pyrrolidinyl)-1-benzothiophene-7-carbonitrile
compound 105: [1-(4-cyano-1-naphthyl)-4-piperidinyl]ethyl acetate
compound 106: [1-(4-cyano-1-naphthyl)-4-piperidinyl]acetic acid
compound 107: 4-[(2S,3S)-3-(hydroxymethyl)-2-methyl-1-pyrrolidinyl]-1-naphtonitrile
compound 108: 4-[(2S,3S)-3-(hydroxymethyl)-2-methyl-1-pyrrolidinyl]-1-benzothiophene-7-carbonitrile
compound 109: 4-[(2S,4R)-4-hydroxy-2-methyl-1-pyrrolidinyl]-1-benzothiophene-7-carbonitrile
compound 110: 4-[(2S,3S)-3-(methoxymethyl)-2-methyl-1-pyrrolidinyl]-1-naphtonitrile
compound 111: 4-[(2S,4S)-4-hydroxy-2-methyl-1-pyrrolidinyl]-1-benzothiophene-7-carbonitrile
compound 112: 4-[(2S)-2-methyl-3-oxo-1-pyrrolidinyl]-1-naphtonitrile
compound 113: 4-[(2S,3R)-3-hydroxy-2-methyl-1-pyrrolidinyl]-1-naphtonitrile
compound 114: 4-[(2S,3R)-3-hydroxy-2-methyl-1-pyrrolidinyl]-1-naphtonitrile methanesulfonate
compound 115: 4-[(2S,3R)-3-hydroxy-2-methyl-1-pyrrolidinyl]-1-benzothiophene-7-carbonitrile
compound 116: 4-[(2S,3S)-3-hydroxy-2-methyl-1-pyrrolidinyl]-1-naphtonitrile
compound 117: 4-[(2S,3S)-3-hydroxy-2-methyl-1-pyrrolidinyl]-1-naphtonitrile sulfate
compound 118: 4-[(2S,3S)-3-hydroxy-2-methyl-1-pyrrolidinyl]-1-benzothiophene-7-carbonitrile
compound 119: 4-[(2S,3S)-3-hydroxy-2-methyl-1-pyrrolidinyl]-1-benzothiophene-7-carbonitrile sulfate
compound 120: 4-[(2R,3R)-3-hydroxy-2-methyl-1-pyrrolidinyl]-1-benzothiophene-7-carbonitrile
compound 121: 4-[(2R,3S)-3-hydroxy-2-methyl-1-pyrrolidinyl]-1-benzothiophene-7-carbonitrile
compound 122: 4-[(2R,3R)-3-hydroxy-2-methyl-1-pyrrolidinyl]-1-naphtonitrile
compound 123: 4-[(2R,3S)-3-hydroxy-2-methyl-1-pyrrolidinyl]-1-naphtonitrile
compound 124: [3-(4-bromo-1-naphthyl)-4,5-dihydroisoxazol-5-yl]methanol
compound 125: 4-[5-(hydroxymethyl)-4,5-dihydroisoxazol-3-yl]-1-naphtonitrile
compound 126: 4-[5-(methoxymethyl)-4,5-dihydroisoxazol-3-yl]-1-naphtonitrile
compound 127: 4-[4-(hydroxymethyl)-2-methyl-1-pyrrolidinyl]-1-naphtonitrile hydrochloride
compound 128: 4-[(2R,4R)-4-hydroxy-2-(hydroxymethyl)-1-pyrrolidinyl]-1-naphtonitrile
compound 129: 4-{[(2R,4R)-1-(4-cyano-1-naphthyl)-4-hydroxypyrrolidin-2-yl]methoxy}-1-naphtonitrile
compound 130: 4-[4-(hydroxymethyl)-2,4-dimethyl-1-pyrrolidinyl]-1-naphtonitrile
compound 131: 4-(1,3-dihydro-2H-isoindol-2-yl)-1-naphtonitrile
compound 132: 4-[(2R,4R)-2-(hydroxymethyl)-4-methoxy-1-pyrrolidinyl]-1-naphtonitrile
compound 133: 4-[(2R,4R)-4-methoxy-2-(methoxymethyl)-1-pyrrolidinyl]-1-naphtonitrile
compound 134: 4-(2,2-dimethyl-1-pyrrolidinyl)-1-naphtonitrile
compound 135: 1-(4-cyano-1-naphthyl)-D-prolineamide
compound 136: (2R)-1-(4-cyano-1-naphthyl)pyrrolidine-2-carbonitrile
compound 137: 4-(3-phenyl-1-pyrrolidinyl)-1-naphtonitrile
compound 138: 4-(3-phenoxy-1-pyrrolidinyl)-1-naphtonitrile
compound 139: 4-[(2S,3R)-3-methoxy-2-methyl-1-pyrrolidinyl]-1-naphtonitrile
compound 140: [1-(4-cyano-1-naphthyl)pyrrolidin-3-yl]ethyl acetate
compound 141: 4-[3-(2-hydroxyethyl)-1-pyrrolidinyl]-1-naphtonitrile
compound 142: 4-(2-propyl-1-pyrrolidinyl)-1-naphtonitrile
compound 143: 4-[(2R)-2-(1-hydroxy-1-methylethyl)-1-pyrrolidinyl]-1-naphtonitrile
compound 144: 4-[(2R)-2-isopropyl-1-pyrrolidinyl]-1-naphtonitrile
compound 145: 4-[3-(benzyloxy)-1-pyrrolidinyl]-1-naphtonitrile
compound 146: 4-[3-(2-methoxyethyl)-1-pyrrolidinyl]-1-naphtonitrile
compound 147: 1-(4-bromo-1-naphthyl)-2-methylcyclopentanol
compound 148: 4-(1-hydroxy-2-methylcyclopentyl)-1-naphtonitrile
compound 149: 4-(5-methylcyclopenta-1-en-1-yl)-1-naphtonitrile and 4-(2-methylcyclopenta-1-en-1-yl)-1-naphtonitrile( about 6:4 mixture)
compound 150: 4-(1,4-oxazepan-4-yl)-1-naphtonitrile
compound 151: 4-(2-methylcyclopentyl)-1-naphtonitrile
compound 152:4-(5-methyl-6-oxabicyclo[3.1.0]hexa-1-yl)-1-naphtonitrile
compound 153:4-[(2S,4R)-4-hydroxy-2-methyl-1-pyrrolidinyl]-1-naphtonitrile
compound 154: 4-[(2S,4S)-4-hydroxy-2-methyl-1-pyrrolidinyl]-1-naphtonitrile
compound 155: 4-[(2S,4R)-4-methoxy-2-methyl-1-pyrrolidinyl]-1-naphtonitrile
compound 156: methyl (2S,3S)-1-(4-cyano-1-naphthyl)-2-methylpyrrolidine-3-carboxylate
compound 157: (2S,3S)-1-(4-cyano-1-naphthyl)-2-methylpyrrolidine-3-carboxylic acid
compound 158: 4-[(2S,3S)-3-(1-hydroxy-1-methylethyl)-2-methyl-1-pyrrolidinyl]-1-naphtonitrile
compound 159: carbamate 1-[(2S,3S)-1-(4-cyano-1-naphthyl)-2-methylpyrrolidin-3-yl]-1-methylethyl
compound 160: (2S,3S)-1-(4-cyano-1-naphthyl)-2-methylpyrrolidine-3-carboxamide
compound 161:( 2S,3S)-1-(4-cyano-1-naphthyl)-2-methylpyrrolidine-3-carbonitrile

The structural formula of the aforementioned compounds 1 - 161 are shown in Table 1 to Table 12. In the tables, "Ex." indicates the Example No. in W02004/16576. Furthermore, "HCl", "H₂SO₄", "MsOH" and the like of the B ring section in the tables indicate that the compound is "hydrochloride", "sulfate", "methanesulfonate", respectively.

**[Table 1]**

| | | | | | | |
|---|---|---|---|---|---|---|
| 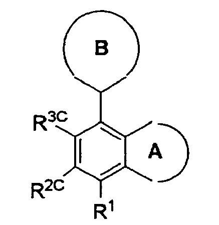 | | | | | | |

| Ex. | Comp. No. | A ring | B ring | R¹ | R^{2C} | R^{3C} |
|---|---|---|---|---|---|---|
| 1 | 1 | 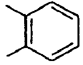 | 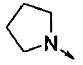 | CN | H | H |
| 2 | 2 | 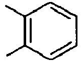 | 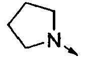 | CO₂H | H | H |
| 3 | 3 | 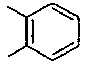 | 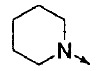 | CN | H | H |
| 4 | 4 | 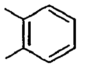 | 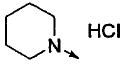 | CN | H | H |
| 5 | 5 | 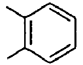 | 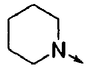 | Br | H | H |
| 6 | 6 | 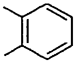 | 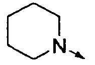 | CF₃ | H | H |
| 7 | 7 | 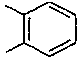 | 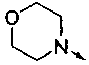 | CN | H | H |
| 8 | 8 | 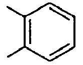 | 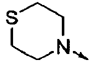 | CN | H | H |
| 9 | 9 | 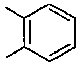 | 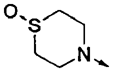 | CN | H | H |
| 10 | 10 | 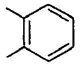 | 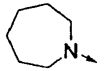 | CN | H | H |
| 11 | 11 | 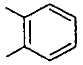 | 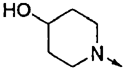 | CN | H | H |
| 12 | 12 | 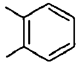 | 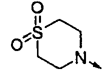 | CN | H | H |
| 13 | 13 | 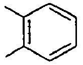 | 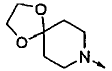 | CN | H | H |
| 14 | 14 | 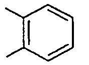 | 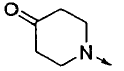 | CN | H | H |

**[Table 2]**

| | | | | | | |
|---|---|---|---|---|---|---|
| 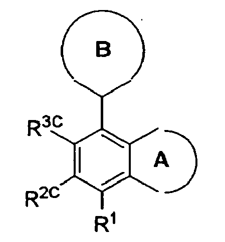 | | | | | | |

| Ex. | Comp. No. | A ring | B ring | R¹ | R^{2C} | R^{3C} |
|---|---|---|---|---|---|---|
| 15 | 15 | 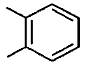 | 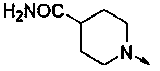 | CN | H | H |
| 16 | 16 | 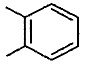 | 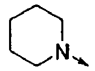 | CN | H | Br |
| 17 | 17 | 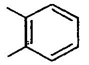 | 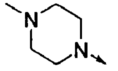 | CN | H | H |
| 18 | 18 | 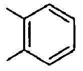 | 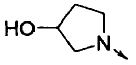 | CN | H | H |
| 19 | 19 | 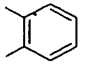 | 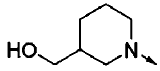 | CN | H | H |
| 20 | 20 | 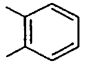 | 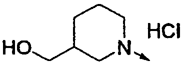 | CN | H | H |
| 21 23 | 21 | 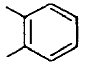 | 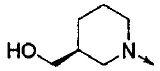 | CN | H | H |
| 22 23 | 22 | 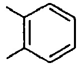 | 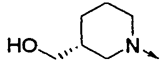 | CN | H | H |
| 24 | 23 | 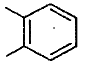 | 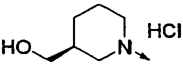 | CN | H | H |
| 25 | 24 | 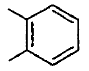 | 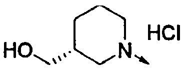 | CN | H | H |
| 26 | 25 | 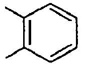 | 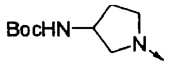 | CN | H | H |
| 27 | 26 | 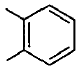 | 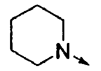 | CN | Br | H |
| 28 | 27 | 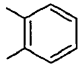 | 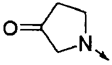 | CN | H | H |
| 29 | 28 | 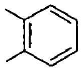 | 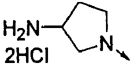 | CN | H | H |

**[Table 3]**

| | | | | |
|---|---|---|---|---|
| 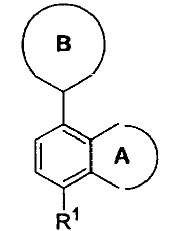 | | | | |

| Ex. | Comp. No. | A ring | B ring | R¹ |
|---|---|---|---|---|
| 30 | 29 | 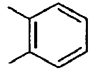 | 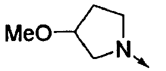 | CN |
| 31 | 30 | 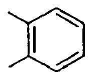 | 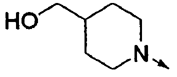 | CN |
| 32 | 31 | 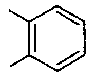 | 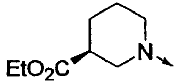 | CN |
| 33 | 32 | 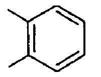 | 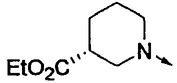 | CN |
| 34 | 33 | 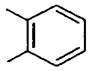 | 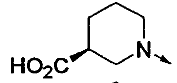 | CN |
| 35 | 34 | 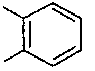 | 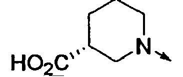 | CN |
| 36 | 35 | 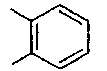 | 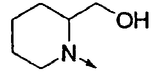 | CN |
| 37 | 36 | 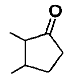 | 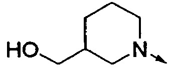 | NO₂ |
| 38 | 37 | 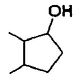 | 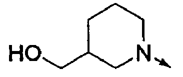 | NO₂ |
| 39 | 38 | 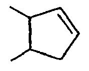 | 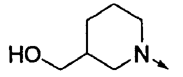 | NO₂ |
| 39 | 39 | 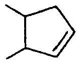 | 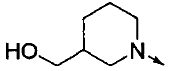 | NO₂ |
| 40 | 40 | 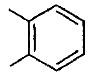 | 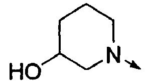 | CN |
| 41 | 41 | 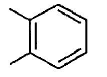 | 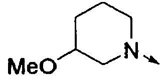 | CN |
| 42 | 42 | 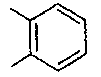 | 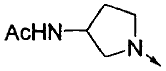 | CN |

**[Table 4]**

| | | | | |
|---|---|---|---|---|
| 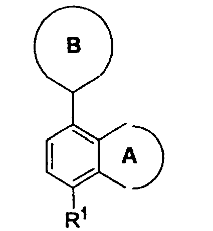 | | | | |

| Ex. | Comp. No. | A ring | B ring | R¹ |
|---|---|---|---|---|
| 43 | 43 | 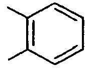 | 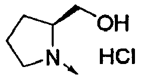 | CN |
| 44 | 44 | 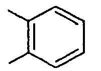 | 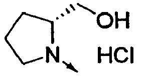 | CN |
| 45 | 45 | 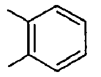 | 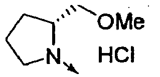 | CN |
| 46 | 46 | 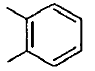 | 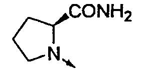 | CN |
| 47 | 47 | 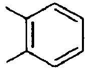 | 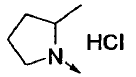 | CN |
| 48 | 48 | 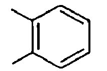 | 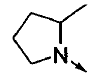 | CN |
| 49 | 49 | 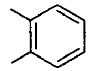 | 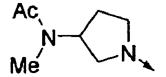 | CN |
| 50 | 50 | 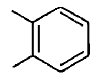 | 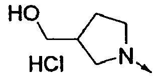 | CN |
| 51 | 51 | 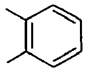 | 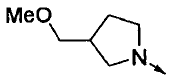 | CN |
| 52 | 52 | 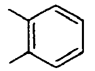 | 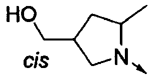 | CN |
| 53 | 53 | 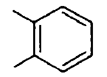 | 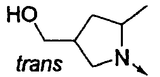 | CN |
| 54 | 54 | 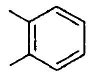 | 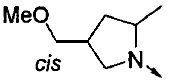 | CN |
| 55 | 55 | 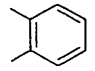 | 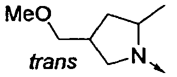 | CN |
| 56 | 56 | 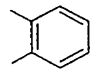 | 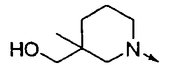 | CN |

**[Table 5]**

| | | | | |
|---|---|---|---|---|
| 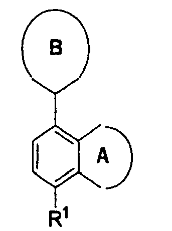 | | | | |

| Ex. | Comp. No. | A ring | B ring | R¹ |
|---|---|---|---|---|
| 57 | 57 | 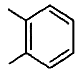 | 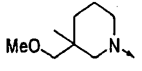 | CN |
| 58 | 58 | 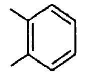 | 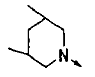 | CN |
| 59 | 59 | 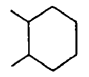 | 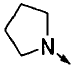 | CN |
| 60 | 60 | 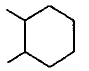 | 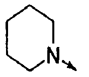 | CN |
| 61 | 61 | 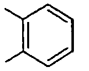 | 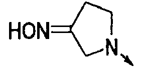 | CN |
| | 62 | 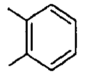 | 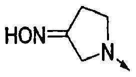 | CN |
| 62 | 63 | 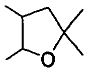 | 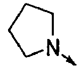 | CN |
| 63 | 64 | 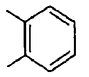 | 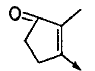 | CN |
| 64 | 65 | 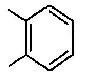 | 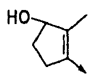 | CN |
| 65 | 66 | 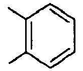 | 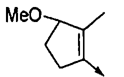 | CN |
| 66 | 67 | 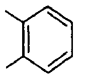 | 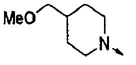 | CN |
| 67 | 68 | 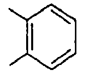 | 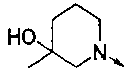 | CN |
| 68 | 69 | 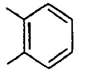 | 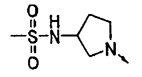 | CN |
| 69 | 70 | 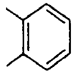 | 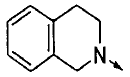 | CN |

**[Table 6]**

| | | | | |
|---|---|---|---|---|
| 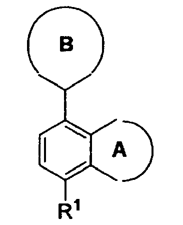 | | | | |

| Ex. | Comp. No. | A ring | B ring | R¹ |
|---|---|---|---|---|
| 70 | 71 | 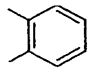 | 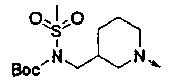 | CN |
| 71 | 72 | 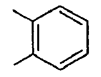 | 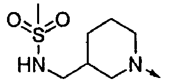 | CN |
| 72 | 73 | 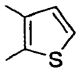 | 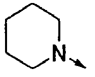 | CN |
| 73 | 74 | 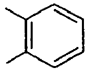 | 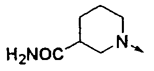 | CN |
| 74 | 75 | 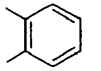 | 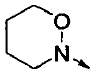 | CN |
| 75 | 76 | 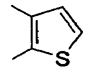 | 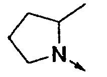 | CN |
| 76 | 77 | 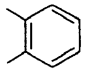 | 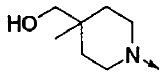 | CN |
| 77 | 78 | 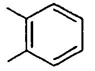 | 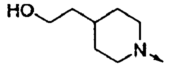 | CN |
| 78 | 79 | 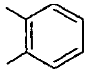 | 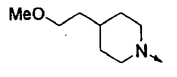 | CN |
| 79 | 80 | 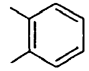 | 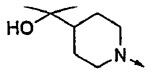 | CN |
| 80 | 81 | 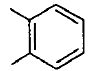 | 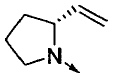 | CN |
| | 82 | 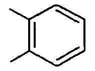 | 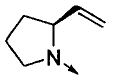 | CN |
| 81 | 83 | 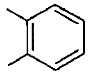 | 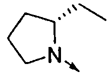 | CN |
| | 84 | 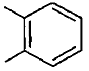 | 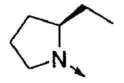 | CN |

**[Table 7]**

| | | | | |
|---|---|---|---|---|
| 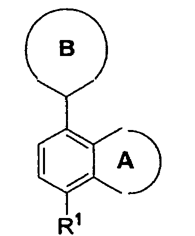 | | | | |

| Ex. | Comp. No. | A ring | B ring | R¹ |
|---|---|---|---|---|
| 82 83 | 85 | 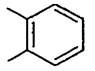 | 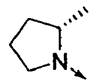 | CN |
| 83 | 86 | 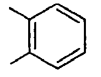 | 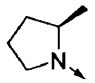 | CN |
| 84 | 87 | 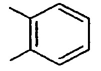 | 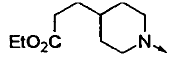 | CN |
| 85 | 88 | 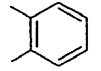 | 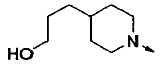 | CN |
| 86 | 89 | 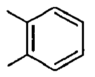 | 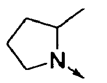 | COCF₃ |
| 87 | 90 | 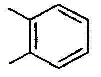 | 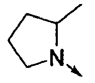 | COCH₃ |
| 88 | 91 | 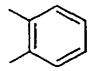 | 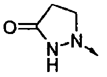 | CN |
| 89 | 92 | 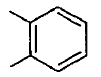 | 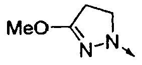 | CN |
| | 93 | 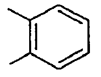 | 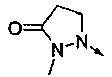 | CN |
| 90 | 94 | 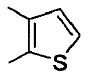 | 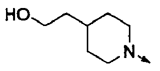 | CN |
| 91 | 95 | 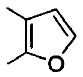 | 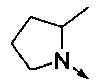 | CN |
| 92 | 96 | 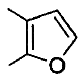 | 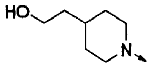 | CN |
| 93 | 97 | 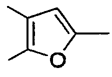 | 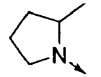 | CN |
| 94 | 98 | 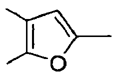 | 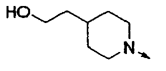 | CN |

**[Table 8]**

| | | | | |
|---|---|---|---|---|
| 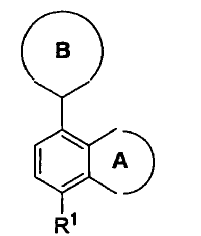 | | | | |

| Ex. | Comp. No. | A ring | B ring | R¹ |
|---|---|---|---|---|
| 95 | 99 | 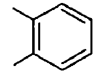 | 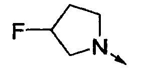 | CN |
| 96 | 100 | 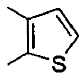 | 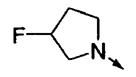 | CN |
| 97 | 101 | 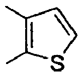 | 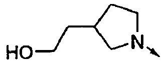 | CN |
| 98 | 102 | 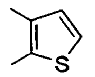 | 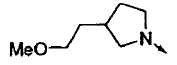 | CN |
| 99 | 103 | 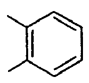 | 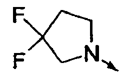 | CN |
| 100 | 104 | 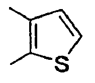 | 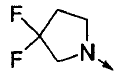 | CN |
| 101 | 105 | 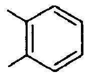 | 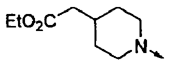 | CN |
| 102 | 106 | 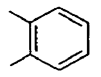 | 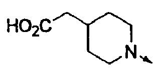 | CN |
| 103 | 107 | 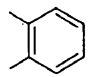 | 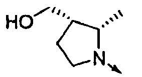 | CN |
| 104 | 108 | 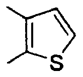 | 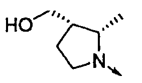 | CN |
| 105 | 109 | 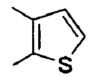 | 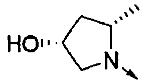 | CN |
| 106 | 110 | 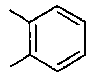 | 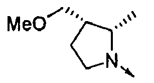 | CN |
| 107 | 111 | 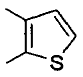 | 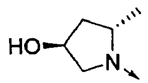 | CN |
| 108 | 112 | 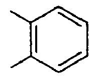 | 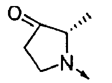 | CN |

**[Table 9]**

| | | | | |
|---|---|---|---|---|
| 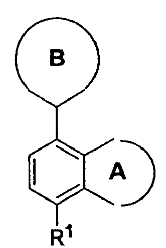 | | | | |

| Ex. | Comp. No. | A ring | B ring | R¹ |
|---|---|---|---|---|
| 109 | 113 | 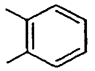 | 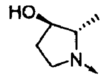 | CN |
| 110 | 114 | 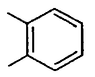 | 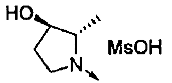 | CN |
| 111 | 115 | 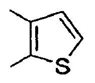 | 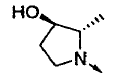 | CN |
| 112 113 | 116 | 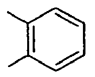 | 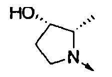 | CN |
| 114 | 117 | 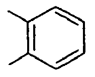 | 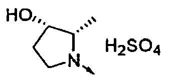 | CN |
| 115 116 | 118 | 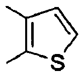 | 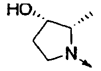 | CN |
| 117 | 119 | 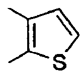 | 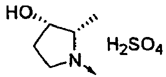 | CN |
| 118 | 120 | 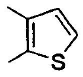 | 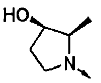 | CN |
| 119 | 121 | 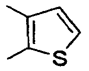 | 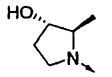 | CN |
| 120 | 122 | 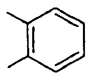 | 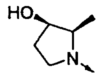 | CN |
| 121 | 123 | 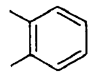 | 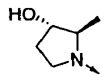 | CN |
| 122 | 124 | 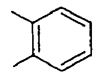 | 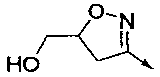 | Br |
| 123 | 125 | 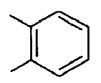 | 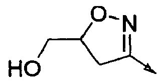 | CN |
| 124 | 126 | 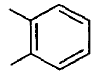 | 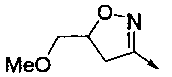 | CN |

**[Table 10]**

| | | | | |
|---|---|---|---|---|
| 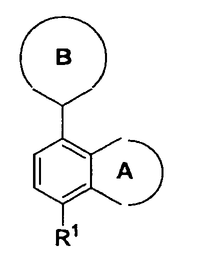 | | | | |

| Ex. | Comp. No. | A ring | B ring | R¹ |
|---|---|---|---|---|
| 125 | 127 | 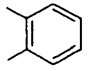 | 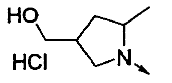 | CN |
| 126 | 128 | 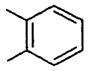 | 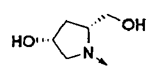 | CN |
| 127 | 129 | 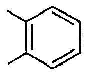 | 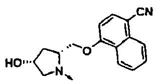 | CN |
| 128 | 130 | 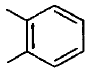 | 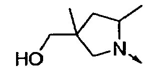 | CN |
| 129 | 131 | 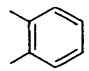 | 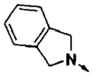 | CN |
| 130 | 132 | 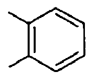 | 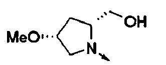 | CN |
| 131 | 133 | 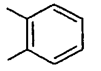 | 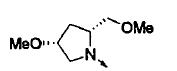 | CN |
| 132 | 134 | 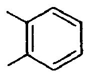 | 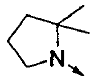 | CN |
| 133 | 135 | 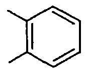 | 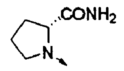 | CN |
| 134 | 136 | 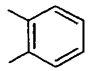 | 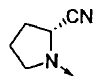 | CN |
| 135 | 137 | 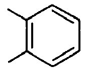 | 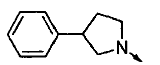 | CN |
| 136 | 138 | 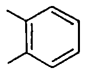 | 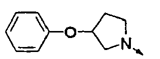 | CN |
| 137 | 139 | 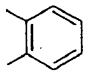 | 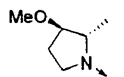 | CN |
| 138 | 140 | 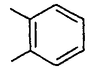 | 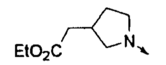 | CN |

**[Table 11]**

| | | | | |
|---|---|---|---|---|
| 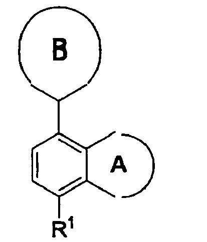 | | | | |

| Ex. | Comp. No. | A ring | B ring | R¹ |
|---|---|---|---|---|
| 139 | 141 | 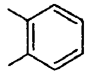 | 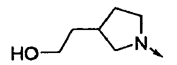 | CN |
| 140 | 142 | 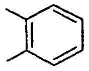 | 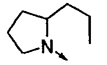 | CN |
| 141 | 143 | 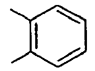 | 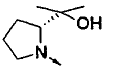 | CN |
| 142 | 144 | 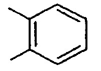 | 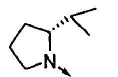 | CN |
| 143 | 145 | 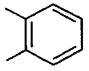 | 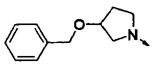 | CN |
| 144 | 146 | 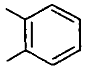 | 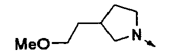 | CN |
| 145 | 147 | 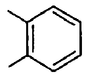 | 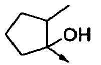 | Br |
| 146 | 148 | 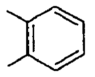 | 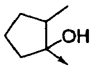 | CN |
| 147 | 149 | 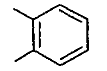 | 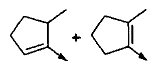 | CN |
| 148 | 150 | 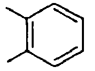 | 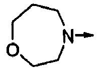 | CN |
| 149 | 151 | 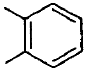 | 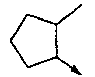 | CN |
| | 152 | 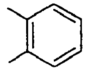 | 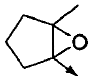 | CN |
| 150 | 153 | 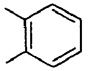 | 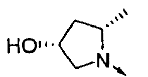 | CN |
| 151 | 154 | 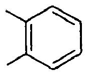 | 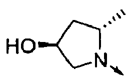 | CN |

**[Table 12]**

| | | | | |
|---|---|---|---|---|
| 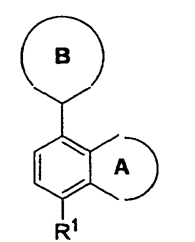 | | | | |

| Ex. | Comp. No. | A ring | B ring | R¹ |
|---|---|---|---|---|
| 152 | 155 | 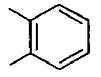 | 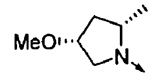 | CN |
| 153 | 156 | 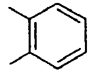 | 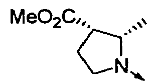 | CN |
| 154 | 157 | 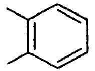 | 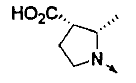 | CN |
| 155 | 158 | 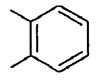 | 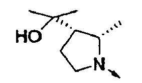 | CN |
| 156 | 159 | 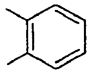 | 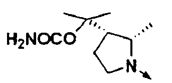 | CN |
| 157 | 160 | 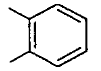 | 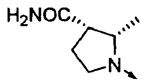 | CN |
| 158 | 161 | 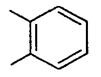 | 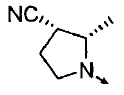 | CN |

Among those, as the compound to be used in the present invention, for example, 4-[4-(hydroxymethyl)-1-piperidinyl]-1-naphthonitrile, 4-[3-(hydroxymethyl)-1-piperidinyl]-1-naphthonitrile, 4-[3-(hydroxymethyl)-3-methyl-1-piperidinyl]-1-naphthonitrile, 4-(2-methyl-1-pyrrolidinyl)-1-naphthonitrile, 4-(2-ethyl-1-pyrrolidinyl)-1-naphthonitrile, 4-(2-vinyl-1-pyrrolidinyl)-1-naphthonitrile, 4-(2-isopropyl-1-pyrrolidinyl)-1-naphthonitrile, 4-(3-hydroxy-2-methyl-1-pyrrolidinyl)-1-naphthonitrile, 4-(3-methoxy-2-methyl-1-pyrrolidinyl)-1-naphthonitrile, 4-(4-methoxy-2-methyl-1-pyrrolidinyl)-1-naphthonitrile 4-[3-(hydroxymethyl)-2-methyl-1-pyrrolidinyl]-1-naphthonitrile, 4-[3-(1-hydroxy-1-methylethyl)-2-methyl-1-pyrrolidinyl]-1-naphthonitrile, 1-(4-cyano-1-naphthyl)-2-methylpyrrolidine-3-carboxamide, 1-(4-cyano-1-naphthyl)-2-methylpyrrolidine-3-carbonitrile, 4-(2-methyl-1-pyrrolidinyl)-1-benzothiophene-7-carbonitrile, 4-(3-hydroxy-2-methyl-1-pyrrolidinyl)-1-benzothiophene-7-carbonitrile, 4-(4-hydroxy-2-methyl-1-pyrrolidinyl)-1-benzothiophene-7-carbonitrile or an optically active substance or a salt thereof can be preferably used.

In the compound of the present invention represented by the formula:

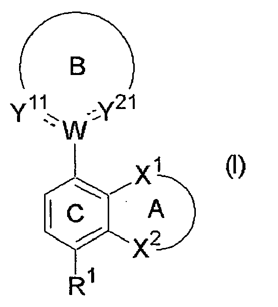

wherein each symbol is as defined above, the compound in the case that "when Ring B is a further optionally substituted bicyclic ring, CR² for Y¹¹ or CR⁴ or the nitrogen atom for Y²¹ may constitute a part of Ring B", represents for example, a compound represented by the formula:

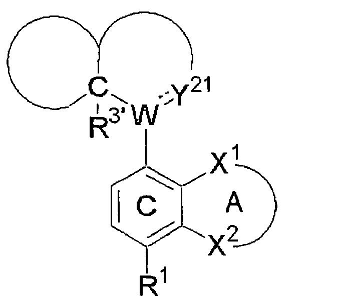

wherein each symbol is as defined above, or a compound represented by the formula:

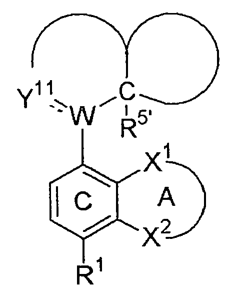

wherein each symbol is as defined above, or a compound represented by the formula:

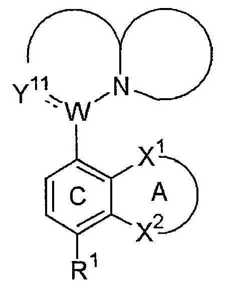

wherein each symbol is as defined above, etc.

Compounds represented by the formula (I'), (I), (Ia), (IIa) or (IIb) etc. (hereinafter to be sometimes referred to as compound (I) etc.) may be hydrated or non-hydrated.
When compound (I), etc. are obtained as a mixture of optically active substances, it can be separated into (S)-isomer or (R)-isomer with a known optical resolution per se.
compound (I), etc. may be labeled with an isotope (e.g., ³H, ¹⁴C, etc.), etc.

The compounds to be used in the present invention may form salts. Salts of the compounds are not particularly limited as long as they do not interfere with the reaction, and include, for example, a salt with an inorganic base, an ammonium salt, a salt with an organic base a salt with an inorganic acid, a salt with an organic acid, a salt with an amino acid, etc.
Preferable examples of the salt with an inorganic base include an alkali metal salt such as sodium salt, potassium salt, etc.; an alkaline earth metal salt such as calcium salt, magnesium salt, etc.; aluminum salt; ammonium salt; etc. Preferable examples of the salt with an organic base include a salt with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.
Preferable examples of the salt with an inorganic acid include a salt with hydrochloric acid, hydrobromic acid, nitric acid, sulfuric acid, phosphoric acid, etc. Preferable examples of the salt with an organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferable examples of the salt with a basic amino acid include a salt with arginine, lysine, ornithine, etc. Preferable examples of the salt with an acidic amino acid include a salt with aspartic acid, glutamic acid, etc.

The prodrug of compound (I), etc. or a salt thereof (hereinafter, abbreviated to compound (I)) means a compound which is converted to compound (I) with a reaction using an enzyme, a gastric acid, etc. under the physiological condition in the living body, that is, a compound which is converted to compound (I) with oxidation, reduction, hydrolysis, etc. according to an enzyme and a compound which is converted to compound (I) with hydrolysis by gastric acid, etc. Examples of the prodrug of compound (I) include a compound wherein an amino group of compound (I) is substituted with acyl, alkyl, phosphoric acid, etc. (e.g., a compound wherein an amino group of compound (I) is substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc,); a compound wherein a hydroxy group of compound (I) is substituted with acyl, alkyl, phosphoric acid, boric acid, etc. (e.g., a compound wherein a hydroxy group of compound (I) is substituted with acetyl, palmitoyl, propanoyl, pivaloyl, succinyl, fumaryl, alanyl, dimethylaminomethylcarbonyl, etc.); a compound wherein a carboxyl group of compound (I) is substituted with ester, amide, etc. (e.g., a compound wherein a carboxyl group of compound (I) is substituted with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); etc. These prodrugs can be manufactured by the known method per se from compound (I).
In addition, the prodrug of compound (I) may be a compound which is converted into compound (I) under the physiological conditions as described in "Pharmaceutical Research and Development", Vol.7 (Molecular Design), pages 163-198 published in 1990 by Hirokawa Publishing Co.

Compound (I') or a salt thereof or a prodrug thereof to be used in the present invention (hereinafter sometimes to be abbreviated as the compound of the present invention) has a tissue-selective androgen receptor regulating action, and can be used as tissue-selective androgen receptor modulator for a mammal.
The "tissue-selective" means that a compound shows an agonistic action on an organ, and an antagonistic action on another organ. For example, the compound of the present invention shows an antagonistic action on the prostate, and an agonistic action on the muscle. Specifically, the compound of the present invention shows an action to inhibit an increase in the prostate weight with a dose that increases the muscle weight (e.g., levator ani muscle and the like). More specifically, it increases the prostate weight by not more than about 10% (preferably not more than 0%) with a dose that increases the levator ani muscle weight by not less than about 20% (preferably about 20% to about 50%). Here, the "increase in the prostate weight by not more than 0%" means that when the prostate weight increases by 0%, the prostate weight does not increase or decrease, and when the prostate weight increases by less than 0%, the prostate weight decreases by the absolute value thereof.
As in the below-mentioned Experimental Example, changes in the organ weight is desirably measured and evaluated in a test system supplemented with testosterone such that the blood testosterone concentration will be that of patients with hypogonadism and male climacterium.
Furthermore, the compound of the present invention can be used as the following pharmaceutical agents.
(1) Frailty suppressant.
(2) Muscular strength enhancer or muscle increasing agent (which provides an effect of preventing aged inpatients from being bedridden, shortening the rehabilitation period and the like).
(3) Cachexia suppressant (e.g., suppressant of cachexia caused by AIDS, cancer and the like).
(4) Body weight decrease suppressant.
(5) Agent for the prophylaxis or treatment of prostate hypertrophy (reduces prostate weight).
(6) Agent for the prophylaxis or treatment of amyotrophy.
(7) Agent for reducing the prostate weight.
(8) Agent for the prophylaxis or treatment of muscle loss caused by diseases (e.g., muscular dystrophy, muscular atrophy, X-linkage spinal cord medulla oblongata muscular atrophy (SBMA), cachexia, malnutrition, Hansen's disease, diabetes, renal diseases, COPD (chronic obstructive pulmonary diseases), cancer, end stage renal failure, sarcopenia (muscle loss in aged men), emphysema, osteomalacia, HIV infection, AIDS, cardiomyopathy and the like).
(9) Agent for the prophylaxis or treatment of hypertriglyceridemia (hyperlipidemia).
(10) Cholesterol-lowering agent.
(11) Agent for the prophylaxis or treatment of metabolic syndrome.
(12) Suppressant of loss of muscle strength in postmenopausal female.
(13) Suppressant of loss of bone mineral density in postmenopausal female.
(14) Suppressant of hot flash (e.g., hot flash, sweating and the like) in postmenopausal female.
(15) Agent for reducing the side effects of LHRH modulator such as LHRH agonist (leuprorelin, goserelin, buserelin, nafarelin, triptorelin, gonadorelin and the like), LHRH antagonist (ganirelix, cetrorelix, antarelix, abarelix, sufugolix and the like) and the like.
(16) Suppressant of loss of muscle strength after drug administration of LHRH modulator and the like.
(17) Suppressant of loss of bone mineral density after drug administration of LHRH modulator and the like.
(18) Suppressant of hot flash (e.g., hot flash, sweating and the like) after drug administration of LHRH modulator and the like.
   Particularly, the compound is preferably used as the following pharmaceutical agents.

(1) frailty suppressant.
(2) Muscular strength enhancer or muscle increasing agent (which has an effect of preventing aged inpatients from being bedridden, shortening the rehabilitation period and the like).
(3) Cachexia suppressant (e.g., suppressant of cachexia caused by AIDS, cancer and the like).
(4) Body weight decrease suppressant.
(5) Agent for the prophylaxis or treatment of prostate hypertrophy (reduces prostate weight).
(6) Agent for the prophylaxis or treatment of amyotrophy.
(7) Agent for reducing the prostate weight.
(8) Agent for the prophylaxis or treatment of muscle loss caused by diseases (e.g., muscular dystrophy, muscular atrophy, X-linkage spinal cord medulla oblongata muscular atrophy (SBMA), cachexia, malnutrition, Hansen's disease, diabetes, renal diseases, COPD (chronic obstructive pulmonary diseases), cancer, end stagerenal failure, sarcopenia (aged muscle loss), emphysema, osteomalacia, HIV infection, AIDS, cardiomyopathy and the like).
(9) Agent for the prophylaxis or treatment of hypertriglyceridemia (hyperlipidemia).
(10) Cholesterol-lowering agent.
(11) Agent for the prophylaxis or treatment of metabolic syndrome.
   Furthermore, since the compound of the present invention reduces the prostate weight, or prevents increase in the
   prostate weight, it can be administered to patients having a possibility of developing prostate cancer. Therefore, the compound of the present invention can also be used as an agent for the prophylaxis or treatment of hypogonadism, osteoporosis, hormone refractory cancer, climacteric disorder (particularly male climacteric disorder), anemia, arteriosclerosis, Alzheimer's disease, erectile dysfunction, depression, wasting disease and the like in patients having a possibility of developing prostate cancer. As the hormone refractory cancer, for example, LHRH derivative resistant cancer, particularly LHRH agonist resistant cancer, can be mentioned.
   In addition, the compound of the present invention is useful as an agent for the prophylaxis or treatment of prostate cancer, urinary bladder cancer, thyroid cancer, osteosarcoma and penile cancer each of which has acquired hormone resistance, in patients having a possibility of developing breast cancer, endometrial cancer, cervical cancer or ovarian cancer as well as prostate cancer.
   The compound of the present invention has low toxicity, and can be used as a medicine as itself, or as a pharmaceutical composition for a mammal (e.g., human, horse, bovine, dog, cat, rat, mouse, rabbit, pig, monkey, etc.) by mixing with pharmaceutically acceptable carriers according to a known method per se.
   The pharmaceutical composition may contain other active ingredients, for example, following drugs for hormone therapy, anticancer agents (e.g., chemotherapeutic agents, immunotherapeutic agents, or cell growth factor and inhibitors for the receptor actions, etc.), in combination with the compound of the present invention etc.

As a medicine for mammals such as humans, the compound of the present invention can be administered orally in the form of, for example, tablets, capsules (including soft capsules and microcapsules), powders, and granules, or parenterally in the form of injections, suppositories, and pellets. Examples of the "parenteral administration route" include intravenous, intramuscular, subcutaneous, intra-tissue, intranasal, intradermal, instillation, intracerebral, intrarectal, intravaginal, intraperitoneal, intratumoral, juxtaposition of tumor and administration directly to the lesion.
The dose of the compound of the present invention varies depending on route for administration, symptoms, etc. For example, in case of oral administration for patient (40 to 80 kg body weight) with breast cancer or prostate cancer as an anticancer agent, the daily dose is 0.1 mg to 200 mg/kg body weight, preferably 1 to 100 mg/kg body weight, more preferably 1 to 50 mg/kg body weight, and it can be administered once or twice or three times per day.

The compound of the present invention may be administrated orally or parenterally as solid formulation such as tablet, capsule, granule, powder, etc.; or liquid formulation such as syrup, injection, etc. as admixture with a pharmaceutically acceptable carrier.
Examples of the pharmaceutically acceptable carrier include various organic or inorganic carriers which are generally used in this field. For example, an excipient, a lubricant, a binder, a disintegrating agent, etc. are used in solid formulations, and a solvent, a solubilizer, a suspending agent, an isotonizing agent, a buffer, a soothing agent, etc. are used in liquid formulations. In addition, if desired, an appropriate additive such as an antiseptic, antioxidant, a colorant, a sweetener, etc. may be used.
Suitable examples of the excipient include lactose, sucrose, D-mannitol, starch, crystalline cellulose, light silicic acid anhydride, etc.
Suitable examples of the lubricant include magnesium stearate, calcium stearate, talc, colloidal silica, etc.
Suitable examples of the binder include crystalline cellulose, sucrose, D-mannitol, dextrin, hydroxypropyl cellulose, hydroxypropylmethyl cellulose, polyvinylpyrrolidone, etc.
Suitable examples of the disintegrating agent include starch, carboxymethyl cellulose, carboxymethyl cellulose calcium, croscarmellose sodium, sodium carboxymethyl starch, etc.
Suitable examples of the solvent include water for injection, alcohol, propylene glycol, macrogol, sesame oil, corn oil, etc.
Suitable examples of the solubilizer include polyethylene glycol, propylene glycol, D-mannitol, benzyl benzoate, ethanol, trisaminomethane, cholesterol, triethanolamine, sodium carbonate, sodium citrate, etc.
Suitable examples of the suspending agent include surfactants such as stearyl triethanolamine, sodium laurylsulfate, laurylaminopropionic acid, lecithin, benzalkonium chloride, benzethonium chloride, glycerin monostearate, etc.; hydrophilic polymers such as polyvinyl alcohol, polyvinylpyrrolidone, sodium carboxymethyl cellulose, methyl cellulose, hydroxymethyl cellulose hydroxyethyl cellulose, hydroxypropyl cellulose, etc.
Suitable examples of the isotonizing agent include sodium chloride, glycerin, D-mannitol, etc.
Suitable examples of the buffer include a buffer solution of phosphate, acetate, carbonate, citrate, etc.
Suitable examples of the soothing agent include benzyl alcohol, etc.
Suitable examples of the antiseptic include paraoxybenzoates, chlorobutanol, benzyl alcohol phenethyl alcohol, dehydroacetic acid, sorbic acid, etc.
Suitable examples of the antioxidant include sulfites, ascorbic acid, etc.

A pharmaceutical composition can be manufactured by a conventional method by containing the compound of the present invention in a ratio of normally 0.1 to 95% (w/w) to the total amount of the preparation, although the ratio varies depending on dosage form, method of administration, carrier, etc.
A combination of (1) administering an effective amount of a compound of the present invention and (2) 1 to 3 selected from the group consisting of (i) administering an effective amount of other anti-cancer agents, (ii) administering an effective amount of hormonal therapeutic agents and (iii) non-drug therapy can prevent and/or treat cancer more effectively. The non-drug therapy includes, for example, surgery, radiotherapy, gene therapy, thermotherapy, cryotherapy, laser cauterization, etc., and two or more of these may be combined.
For example, the compound of the present invention can be administered to the same subject simultaneously with hormonal therapeutic agents, anticancer agents (e.g., chemotherapeutic agents, immunotherapeutic agents, or drugs that inhibit the activity of growth factors or growth factor receptors), antiemetic agents (hereinafter, these are abbreviated to as a combination drug).
Although the compound of the present invention exhibits excellent anticancer action even when used as a simple agent, its effect or QOL of patients can be enhanced by using it in combination with one or more of the combination drug(s) mentioned above (multi-agent co-administration).

The "hormonal therapeutic agents" include fosfestrol, diethylstylbestrol, chlorotrianiserin, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogens (e.g., tamoxifen citrate, toremifene citrate, etc.), pill preparations, mepitiostane, testrolactone, aminoglutethimide, LH-RH agonists (e.g., goserelin acetate, buserelin, leuprorelin, etc.), droloxifene, epitiostanol, ethinylestradiol sulfonate, aromatase inhibitors (e.g., fadrozole hydrochloride, anastrozole, letrozole, exemestane, vorozole, formestane, etc.), antiandrogens (e.g., flutamide, bicalutamide, nilutamide), 5α-reductase inhibitors (e.g., finasteride, epristeride, etc.), adrenocorticohormone drugs (e.g., dexamethasone, prednisolone, betamethasone, triamcinolone, etc.), androgen synthesis inhibitors (e.g., abiraterone etc.), retinoid and drugs that retard retinoid metabolism (e.g., liarozole, etc.), etc. and LH-RH derivatives are preferable.
The "chemotherapeutic agents" include alkylating agents, antimetabolites, anticancer antibiotics, plant-derived anticancer agents, etc.
The "alkylating agents" include nitrogen mustard, nitrogen mustard N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, ranimustine, sodium estramustine phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, carboquone, adozelesin, cystemustine, bizelesin etc.
The "antimetabolites" include mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emitefur, etc.), aminopterine, leucovorin calcium, tabloid, butocin, folinate calcium, levofolinate calcium, cladribine, emitefur, fludarabine, gemcitabine, hydroxycarbamide, pentostatin, piritrexim, idoxuridine, mitoguazone, thiazophrine, ambamustine, etc.
The "anticancer antibiotics" include actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, etc.
The "plant-derived anticancer agents" include etoposide, etoposide phosphate, vinblastine sulfate, vincristine sulfate, vindesine sulfate, teniposide, paclitaxel, docetaxel, DJ-927, vinorelbine, etc.
The "immunotherapeutic agents (BRM)" include picibanil, krestin, sizofiran, lentinan, ubenimex, interferons, interleukins, macrophage colony-stimulating factor, granulocyte colony-stimulating factor, erythropoietin, lymphotoxin, BCG vaccine, Corynebacterium parvum, levamisole, polysaccharide K, procodazole, etc.
The "growth factor" in the "drugs that inhibit the activity of growth factors or growth factor receptors" includes any substances that promote cell proliferation, which are normally peptides having a molecular weight of not more than 20,000 that are capable of exhibiting their activity at low concentrations by binding to a receptor, including (1) EGF (epidermal growth factor) or substances possessing substantially the same activity as it [e.g., EGF, heregulin (HER2 ligand), etc.], (2) insulin or substances possessing substantially the same activity as it [e.g., insulin, IGF (insulin-like growth factor)-1, IGF-2, etc.], (3) FGF (fibroblast growth factor) or substances possessing substantially the same activity as it [e.g., acidic FGF, basic FGF, KGF (keratinocyte growth factor), FGF-10, etc.], (4) other cell growth factors [e.g., CSF (colony stimulating factor), EPO (erythropoietin), IL-2 (interleukin-2), NGF (nerve growth factor), PDGF (platelet-derived growth factor), TGF β (transforming growth factor β), HGF (hepatocyte growth factor), VEGF (vascular endothelial growth factor), etc.], etc.
The "growth factor receptors" include any receptors capable of binding to the aforementioned growth factors, including EGF receptor, heregulin receptor (HER2), insulin receptor, IGF receptor, FGF receptor-1 or FGF receptor-2, etc.
The "drugs that inhibit the activity of cell growth factor" include various kinase inhibitors, trastuzumab (Herceptin (trademark): (HER2 antibody)), imatinib mesylate, ZD1839, cetuximab, etc.
In addition to the aforementioned drugs, L-asparaginase, aceglatone, procarbazine hydrochloride, protoporphyrin-cobalt complex salt, mercuric hematoporphyrin-sodium, topoisomerase I inhibitors (e.g., irinotecan, nogitecan, exatecan (DX-8951f, DE-310, rubitecan, T-0128, etc.), topoisomerase II inhibitors (e.g., sobuzoxane, etc.), differentiation inducers (e.g., retinoid, vitamin D, etc.), angiogenesis inhibitors, α-blockers (e.g., tamsulosin hydrochloride), TZT-1027, etc., may be used.
The "antiemetic agents" includes 5-HT₃ antagonist such as ondansetron, tropisetron hydrochloride, azasetron, ramosetron, granisetron, dolasetron mesylate and palonosetron, a gastrointestinal tract motility promoter such as 5-HT₄ antagonist such as domperidone, mosapride and metoclopramide; a gastrointestinal tract motility regulator such as trimebutine; phenothiazine drugs such as prochlorperazine maleate, promethazine and thiethylperazine; anxiolytics such as haloperidole, phenol phthalate chlorpromazine, diazepam and droperidol; steroids such as dexamethasone, prednisolone, betamethasone and triamcinolone; other drugs such as dimethylhydric acid, diphenhydramine, hyoscine, hyoscine bromide and tetrabenazine, etc.

The LH-RH derivative includes an LH-RH derivative or salt thereof which is effective against hormone-dependent diseases, especially sex hormone-dependent diseases such as sex hormone-dependent cancers (e.g., prostate cancer, uterine cancer, breast cancer, hypophyseal tumor, liver cancer, etc.), prostatic hypertrophy, endometriosis, uterine myoma, precocious puberty, dysmenorrhea, amenorrhea, premenstrual syndrome, polycystic ovary disease, etc., and contraception (or infertility when rebound effect after drug withdrawal is applied). Further it includes an LH-RH derivative or salt thereof which is effective against benign tumor or malignant tumor which is sex hormone-independent and LH-RH sensitive.
Specific examples of the LH-RH derivatives or salt thereof include peptides described in "Treatment with GnRH analogs: Controversies and perspectives" issued in 1996 by The Parthenon Publishing Group Ltd., PCT Japanese Translation Patent Publication No. 91-503165, JP-A 91-101695, JP-A 95-97334 and JP-A 96-259460, etc.

The LH-RH derivative includes LH-RH agonists and LH-RH antagonists. The LH-RH antagonist includes, for example, a physiologically active peptide represented by the formula:

X-D2Nal-D4ClPhe-D3Pal-Ser-A-B-Leu-C-Pro-DAlaNH₂

[wherein X is N(4H₂-furoyl)Gly or NAc, A is a residue selected from NMeTyr, Tyr, Aph(Atz) and NMeAph(Atz), B is a residue selected from DLys(Nic), DCit, DLys(AzaglyNic), DLys(AzaglyFur), DhArg(Et₂), DAph(Atz) and DhCi, and C is Lys(Nisp), Arg or hArg(Et₂)] or a salt thereof, etc., especially preferably, abarelix, ganirelix, cetrorelix, 5-(N-benzyl-N-methylaminomethyl)-1-(2,6-difluorobenzyl)-6-[4-(3-methoxyureido)phenyl]-3-phenylthieno[2,3-d]pyrimidin-2,4(1H,3H)-dione, 5-(N-benzyl-N-methylaminomethyl)-1-(2,6-difluorobenzyl)-6-[4-(3-ethylureido)phenyl]-3-phenylthieno[2,3-d]pyrimidin-2,4(1H,3H)-dione, 5-(N-benzyl-N-methylaminomethyl)-1-(2,6-difluorobenzyl)-6-[4-(3-ethylureido)phenyl]-3-phenylthieno[2,3-d]pyrimidin-2,4(1H,3H)-dione hydrochloride, etc.
The LH-RH agonist includes, for example, a physiologically active peptide represented by the formula:

5-oxo-Pro-His-Trp-Ser-Tyr-Y-Leu-Arg-Pro-Z

[wherein Y is a residue selected from DLeu, DAla, DTrp, DSer(tBu), D2Nal and DHis(ImBzl) and Z is NH-C₂H₅ or Gly-NH₂] or a salt thereof, etc, especially, for example, goserelin acetate, buserelin, etc., suitably, a peptide wherein Y is DLeu, and Z is NH-C₂H₅ (that is, Peptide A represented by 5-oxo-Pro-His-Trp-Ser-Tyr-DLeu-Leu-Arg-Pro-NH-C₂H₅; leuprorelin) or a salt thereof (e.g., acetate).

The abbreviations for an amino acid, a peptide, a protecting group etc. in polypeptides described herein are based on abbreviations according to IUPAC-IUB Commission on Biochemical Nomenclature or conventional abbreviations in the art. In addition, when the amino acids have optical isomers, they represent L-form unless otherwise indicated.
Examples of abbreviations are shown below:
- Abu:: Aminobutyric acid
- Aibu:: 2-Aminobutyric acid
- Ala:: Alanine
- Arg:: Arginine
- Gly:: Glycine
- His:: Histidine
- Ile:: Isoleucine
- Leu:: Leucine
- Met:: Methionine
- Nle:: Norleucine
- Nval:: Norvaline
- Phe:: Phenylalanine
- Phg:: Phenylglycine
- Pro:: Proline
- (Pyr)Glu:: Pyroglutamic acid
- Ser:: Serine
- Thr:: Threonine
- Trp:: Tryptophan
- Tyr:: Tyrosine
- Val:: Valine
- D2Nal:: D-3-(2-naphthyl)alanine residue
- DSer(tBu):: O-tert-butyl-D-serine
- DHis (ImBzl) :: N^{im}-benzyl-D-histidine
- PAM:: Phenylacetamidomethyl
- Boc:: t-Butyloxycarbonyl
- Fmoc:: 9-fluorenylmethyloxycarbonyl
- Cl-Z:: 2-Chloro-benzyloxycarbonyl
- Br-Z:: 2-Bromo-benzyloxycarbonyl
- Bzl:: Benzyl
- Cl₂-Bzl:: 2,6-Dichlorobenzyl
- Tos:: p-Toluenesulfonyl
- HONb:: N-hydroxy-5-norbornene-2,3-dicarboxyimide
- HOBt:: 1-Hydroxybenzotriazole
- HOOBt:: 3-Hydroxy-3,4-dihydro-4-oxo-1,2,3-benzotriazine
- MeBzl:: 4-Methylbenzyl
- Bom:: Benzyloxymethyl
- Bum:: t-Butoxymethyl
- Trt:: Trityl
- DNP:: Dinitrophenyl
- DCC:: N,N'-dicyclohexylcarbodiimide

Among the above-mentioned these especially, the combination drug is preferably a LH-RH agonist (e.g., goserelin acetate, buserelin, leuprorelin, etc.), etc.

In combinations of the compound of the present invention and the combination drug, the administration time of the compound of the present invention and the combination drug is not restricted, and the compound of the present invention or the combination drug can be administered to the administration subject simultaneously, or may be administered at different times. The dosage of the combination drug may be determined according to the dose clinically used, and can be appropriately selected depending on the administration subject, administration route, disease, combination etc.
The administration mode of the compound of the present invention and the combination drug is not particularly limited, and it is sufficient that the compound of the present invention and the combination drug are combined in administration. Examples of such administration mode include the following methods: (1) The compound of the present invention and the combination drug are simultaneously produced to give a single preparation which is administered. (2) The compound of the present invention and the combination drug are separately produced to give two kinds of preparations which are administered simultaneously by the same administration route. (3) The compound of the present invention and the combination drug are separately produced to give two kinds of preparations which are administered by the same administration route only at the different times. (4) The compound of the present invention and the combination drug are separately produced to give two kinds of preparations which are administered simultaneously by different administration routes. (5) The compound of the present invention and the combination drug are separately produced to give two kinds of preparations which are administered by different administration routes at different times (e.g., the compound of the present invention and the combination drug are administered in this order, or in the reverse order). Hereafter, these administration modes are referred to as the combination preparation of the present invention.

The combination preparation of the present invention has low toxicity, and for example, the compound of the present invention or (and) the above-mentioned combination drug can be mixed, according to a per se known method, with a pharmaceutically acceptable carrier to give pharmaceutical compositions, for example, tablets (including a sugar-coated tablet, film-coated tablet), powders, granules, capsules (including a soft capsule), solutions, injections, suppositories, sustained release agents etc. which can be safely administered orally or parenterally (e.g., local, rectum, vein, etc.). The injection can be administered by intravenous, intramuscular, subcutaneous, intra-tissue, intranasal, intradermal, instillation, intracerebral, intrarectal, intravaginal, intraperitoneal, intratumoral, juxtaposition of tumor and administration directly to the lesion.
The pharmaceutically acceptable carrier which may be used in production of the combination preparation includes those used for the above mentioned pharmaceutical composition of the present invention.

The compounding ratio of the compound of the present invention to the combination drug in the combination preparation of the present invention can be appropriately selected depending on the administration subject, administration route, diseases etc.
For example, the content of the compound of the present invention in the combination preparation differs depending on the form of preparation, and is usually from about 0.01% by weight to 100% by weight, preferably from about 0.1% by weight to 50% by weight, more preferably from about 0.5% by weight to 20% by weight, to the total of the preparation.
The content of the combination drug in the combination preparation of the present invention differs depending on the form of preparation, and is usually from about 0.01% by weight to 100% by weight, preferably from about 0.1% by weight to 50% by weight, more preferably from about 0.5% by weight to 20% by weight, to the total of the preparation.
The content of additives such as a carrier etc. in the combination preparation of the present invention differs depending on the form of preparation, and is usually from about 1% by weight to 99.99% by weight, preferably from about 10% by weight to 90% by weight, to the total of the preparation.
When the compound of the present invention and the combination drug are formulated separately, the same contents may be adopted.

These preparations can be manufactured by a per se known method commonly used in the pharmaceutical manufacturing process.
For example, the compound of the present invention and the combination drug can be made as an injection such as an aqueous injection together with a dispersing agent (e.g., Tween 80 (manufactured by Atlas Powder, US), HCO 60 (manufactured by Nikko Chemicals Co., Ltd.), polyethylene glycol, carboxymethyl cellulose, sodium alginate, hydroxypropylmethyl cellulose, dextrin etc.), a stabilizer (e.g., ascorbic acid, sodium pyrosulfite, etc.), a surfactant (e.g., Polysorbate 80, macrogol etc.), a solubilizer (e.g., glycerin, ethanol etc.), a buffer (e.g., phosphoric acid and alkali metal salt thereof, citric acid and alkali metal salt thereof, etc.), an isotonizing agent (e.g., sodium chloride, potassium chloride, mannitol, sorbitol, glucose etc.), a pH regulator (e.g., hydrochloric acid, sodium hydroxide etc.), an antiseptic (e.g., ethyl p-oxybenzoate, benzoic acid, methylparaben, propylparaben, benzyl alcohol etc.), a dissolving agent (e.g., cone. glycerin, meglumine etc.), a solubilizing agent (e.g., propylene glycol, sucrose etc.), a soothing agent (e.g., glucose, benzyl alcohol etc.), etc., or an oily injection by dissolving, suspending or emulsifying them in a vegetable oil such as olive oil, sesame oil, cotton seed oil, corn oil etc. or a solubilizing agent such as propylene glycol, and molding them.

In the case of a preparation for oral administration, the compound of the present invention and the combination drug can be made as a preparation for oral administration by adding an excipient (e.g., lactose, sucrose, starch etc.), a disintegrating agent (e.g., starch, calcium carbonate etc.), a binder (e.g., starch, arabic gum, carboxymethyl cellulose, polyvinylpyrrolidone, hydroxypropyl cellulose etc.), a lubricant (e.g., talc, magnesium stearate, polyethylene glycol 6000 etc.) etc., to the compound of the present invention or the combination drug, according to a per se known method, and compressing and molding the mixture, then if desired, coating the molded product by a per se known method for the purpose of masking of taste, enteric property or sustained release. The film forming agent includes, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (methacrylic acid/acrylic acid copolymer, manufactured by Rohm, DE), pigment (e.g., iron oxide red, titanium dioxide, etc.) etc. The preparation for oral administration may be either a rapid release preparation or a sustained release preparation.

For example, in the case of a suppository, the compound of the present invention and the combination drug can be made into an oily or aqueous solid, semisolid or liquid suppository according to a per se known method. The oily substrate used in the above-mentioned composition includes, for example, glycerides of higher fatty acids [e.g., cacao butter, Witepsols (manufactured by Dynamite Nobel, DE), etc.], intermediate grade fatty acids [e.g., Miglyols (manufactured by Dynamite Nobel, DE), etc.], or vegetable oils (e.g., sesame oil, soy bean oil, cotton seed oil etc.), etc. Further, the aqueous base includes, for example, polyethylene glycols and propylene glycol, and the aqueous gel base includes, for example, natural gums, cellulose derivatives, vinyl polymers, acrylic acid polymers, etc.
The above-mentioned sustained release agent includes sustained release microcapsules, etc.
For obtaining a sustained release microcapsule, a per se known method can be adopted. For example, it is preferable to mold into a sustained release preparation shown in [2] below.
A compound of the present invention is preferably molded into an oral administration preparation such as a solid preparation (e.g., powder, granule, tablet, capsule, etc.) etc., or molded into a rectal administration preparation such as a suppository. Particularly, an oral administration preparation is preferable.
The combination drug can be made into the above-mentioned drug form depending on the kind of the drug.

In the following, there will be shown specifically [1] an injection of the compound of the present invention or the combination drug and preparation thereof, [2] a rapid release preparation or sustained release preparation of the compound of the present invention or the combination drug and preparation thereof and [3] a sublingual tablet, a buccal or an intraoral quick integrating agent of the compound of the present invention or the combination drug or preparation thereof.

### [1] Injection and preparation thereof

It is preferred that an injection is prepared by dissolving the compound of the present invention or the combination drug in water. This injection may be allowed to contain a benzoate and/or a salicylate.
The injection is obtained by dissolving the compound of the present invention or the combination drug, and if desired, a benzoate and/or a salicylate, into water.
The above-mentioned salts of benzoic acid and salicylic acid include, for example, salts of alkali metals such as sodium, potassium etc., salts of alkaline earth metals such as calcium, magnesium etc., ammonium salts, meglumine salts, organic acid salts such as tromethamol, etc.
The concentration of the compound of the present invention or the combination drug in the injection is from 0.5 w/v% to 50 w/v%, preferably from about 3 w/v% to about 20 w/v%. The concentration of a salt of benzoic acid or/and a salt of salicylic acid is from 0.5 w/v% to 50 w/v%, preferably from 3 w/v% to 20 w/v%.

Conventional additives to be used in an injection may be appropriately added in a preparation of the present invention. Examples of the additives include a stabilizer (e.g., ascorbic acid, sodium pyrosulfite, etc.), a surfactant (e.g., Polysorbate 80, macrogol etc.), a solubilizer (e.g., glycerin, ethanol etc.), a buffer (e.g., phosphoric acid and alkali metal salt thereof, citric acid and alkali metal salt thereof, etc.), an isotonizing agent (e.g., sodium chloride, potassium chloride, etc.), a dispersing agent (e.g., hydroxypropylmethyl cellulose, dextrin), a pH regulator (e.g., hydrochloric acid, sodium hydroxide etc.), an antiseptic (e.g., ethyl p-oxybenzoate, benzoic acid etc.), a dissolving agent (e.g., conc. glycerin, meglumine etc.), a solubilizing agent (e.g., propylene glycol, sucrose etc.), a soothing agent (e.g., glucose, benzyl alcohol etc.), etc. These additives are blended in a usual proportion generally employed in an injection.
It is advantageous that the pH of the injection is controlled from 2 to 12, preferably from 2.5 to 8.0 by addition of a pH regulator.
An injection is obtained by dissolving the compound of the present invention or the combination drug and if desired, a salt of benzoic acid and/or a salt of salicylic acid, and if necessary, the above-mentioned additives into water. These may be dissolved in any order, and can be appropriately dissolved in the same manner as in a conventional method of producing an injection.
An aqueous solution for injection may be advantageously heated, alternatively, for example, filter sterilization, high pressure heat sterilization, etc. can be conducted in the same manner as those for a usual injection, to provide an injection.
It may be advantageous that an aqueous solution for injection is subjected to high pressure heat sterilization at 100°C to 121°C for 5 minutes to 30 minutes. Further, a preparation endowed with the antibacterial property of a solution may also be produced so that it can be used as a preparation which is divided and administered multiple-times.

### [2] Sustained release preparation or rapid release preparation, and preparation thereof

Preferred is a sustained release preparation which is obtained, by coating a core containing the compound of the present invention or the combination drug with a film forming agent such as a water-insoluble substance, swellable polymer, etc., if desired. For example, a sustained release preparation for oral once-a-day administration is preferable.
The water insoluble substance used in a film forming agent includes, for example, a cellulose ether such as ethyl cellulose, butyl cellulose, etc.; a cellulose ester such as cellulose acetate, cellulose propionate, etc.; a polyvinyl ester such as polyvinyl acetate, polyvinyl butyrate, etc.; an acrylic acid polymer such as acrylic acid/methacrylic acid copolymer, methylmethacrylate copolymer, ethoxyethyl methacrylate/cinnamoethylmethacrylate/aminoalkyl methacrylate copolymer, polyacrylic acid, polymethacrylic acid, methacrylic acid alkyl amide copolymer, poly(methyl methacrylate), polymethacrylate, polymethacryl amide, amino alkyl methacrylate copolymer, poly(methacrylic acid anhydride), glycidyl methacrylate copolymer, specially an Eudragit (manufactured by Rohm Pharma) such as Eudragit RS-100, RL-100, RS-30D, RL-30D, RL-PO, RS-PO (copolymer of ethyl acylate/methyl methacrylate/trimethyl chloride methacrylate/ammonium ethyl), Eudragit NE-30D (copolymer of methyl methacrylate/ethyl acrylate), etc., a hydrogenated oil such as hardened caster oil (e.g., Lovely wax (Freund Corporation), etc.), etc.; a wax such as carnauba wax, fatty acid glycerin ester, paraffin, etc.; polyglycerin fatty acid ester, etc.

The swellable polymer is preferably a polymer having acidic dissociating group and pH-dependent swelling property, and a polymer having acidic dissociating group which swells little in an area such as stomach and swells in a neutral area such as the small intestine or the large intestine.
The polymer having acidic dissociating group and pH-dependent swelling property includes, for example, crosslinkable polyacrylic polymer such as Carbomer 934P, 940, 941, 974P, 980, 1342 etc., polycarbophil, calcium polycarbophil (all are manufactured by BF Goodrich.), Hibiswako 103, 104, 105, 304 (all are manufactured by Wako Pure Chemical Industries, Ltd.), etc.

The film forming agent used in a sustained release preparation may further contain a hydrophilic substance.
The hydrophilic substance includes, for example, a polysaccharide optionally having sulfuric acid group such as pullulans, dextrin, arginic acid alkali metal salt, etc.; a polysaccharide having a hydroxyalkyl group or a carboxyalkyl group such as hydroxypropyl cellulose, hydroxypropyl methyl cellulose, sodium carboxymethyl cellulose, etc.; methyl cellulose; polyvinyl pyrrolidone; polyvinyl alcohol; polyethylene glycol; etc.
The content of water-insoluble substance in the film forming agent of sustained release preparation is about 30% (w/w) to about 90% (w/w), preferably about 35% (w/w) to about 80% (w/w), and more preferably about 40% (w/w) to about 75% (w/w). The content of swellable polymer is about 3% (w/w) to about 30% (w/w), preferably about 3% (w/w) to about 15% (w/w). The film forming agent may further contain a hydrophilic substance, in this case, the content of the hydrophilic substance in the film forming agent is about 50% (w/w) or less, preferably about 5% (w/w) to about 40% (w/w), and more preferably about 5% (w/w) to about 35% (w/w). This % (w/w) indicates % by weight based on a film forming agent composition which is obtained by removing a solvent (e.g., water, lower alcohols such as methanol, ethanol etc.) from a film forming agent liquid.

The sustained release preparation is manufactured by preparing a core containing drug as exemplified below, then, coating the resultant core with a film forming agent liquid prepared by heating and dissolving a water-insoluble substance, swellable polymer, etc. or by dissolving or dispersing it in a solvent.

### I. Preparation of core containing a drug

The form of a core containing a drug to be coated with a film forming agent (hereinafter, sometimes simply referred to as the core) is not particularly limited, and preferably, the core is formed into particles such as granules or fine particles.
When the core is composed of granules or fine particles, the average particle size thereof is preferably from about 150 to about 2,000 µm, further preferably, from about 500 µm to about 1,400 µm.
Preparation of the core can be conducted by a usual preparation. For example, it can be prepared by mixing a suitable excipient, binding agent, disintegrating agent, lubricant, stabilizer, etc. with a drug, and subjecting the mixture to wet-extrusion granulating method or fluidized bed granulating method, etc.
The content of drugs in a core is from about 0.5% (w/w) to about 95% (w/w), preferably from about 5.0% (w/w) to about 80% (w/w), further preferably from about 30% (w/w) to about 70% (w/w).

The excipient contained in the core includes, for example, saccharides such as sucrose, lactose, mannitol, glucose etc., starch, crystalline cellulose, calcium phosphate, corn starch etc. Among them, crystalline cellulose, corn starch are preferable.
The binders include, for example, polyvinyl alcohol, hydroxypropyl cellulose, polyethylene glycol, polyvinyl pyrrolidone, Pluronic F68, arabic gum, gelatin, starch, etc.
The disintegrators include, for example, carboxymethyl cellulose calcium (ECG505), croscarmellose sodium (Ac-Di-Sol), crosslinkable polyvinyl pyrrolidone (crospovidone), low-substituted hydroxypropyl cellulose (L-HPC), etc. Among these, hydroxypropyl cellulose, polyvinyl pyrrolidone and low-substituted hydroxypropyl cellulose are preferable. The lubricants or the aggregation inhibitor includes, for example, talc, magnesium stearate and an inorganic salt thereof. The lubricant includes a polyethylene glycol, etc. The stabilizing agent includes an acid such as tartaric acid, citric acid, succinic acid, fumaric acid, maleic acid, etc.

In addition to the above-mentioned, the core can also be prepared by, for example, a rolling granulation method in which a drug or a mixture of the drug with an excipient, lubricant, etc. is added portionwise onto an inert carrier particle which is the core of the core while spraying a binder dissolved in a suitable solvent such as water, lower alcohol (e.g., methanol, ethanol, etc.) etc., a pan coating method, a fluidized bed coating method or a melt granulating method. The inert carrier particle includes, for example, those made of sucrose, lactose, starch, crystalline cellulose or waxes, and the average particle size thereof is preferably from about 100 µm to about 1,500 µm.
For the purpose of separating the drug contained in the core from the film forming agent, the surface of the core may be coated with a protective agent. The protective agent includes, for example, the above-mentioned hydrophilic substances, water-insoluble substances etc. The protective agent includes, preferably polyethylene glycol, and polysaccharides having a hydroxyalkyl group or carboxyalkyl group, more preferably, hydroxypropylmethyl cellulose and hydroxypropyl cellulose. The protective agent may contain a stabilizer such as acids such as tartaric acid, citric acid, succinic acid, fumaric acid, maleic acid etc., and a lubricant such as talc etc. When the protective agent is used, the coating amount is from about 1% (w/w) to about 15% (w/w), preferably from about 1% (w/w) to about 10% (w/w), further preferably from about 2% (w/w) to about 8% (w/w), based on the core.
The coating of the protective agent can be carried out by a usual coating method, and specifically, the coating can be carried out by spraying the protective agent by a fluidized bed coating method, pan coating method etc.

### II. Coating of core with a film forming agent

A core obtained in the above-mentioned step I is coated with a film forming agent liquid obtained by heating and dissolving the above-mentioned water-insoluble substance and pH-dependent swellable polymer, and a hydrophilic substance, or by dissolving or dispersing them in a solvent, to give a sustained release preparation.
The method for coating a core with a film forming agent liquid includes, for example, a spray coating method etc.
The composition ratio of a water-insoluble substance, swellable polymer and hydrophilic substance in a film forming agent liquid is appropriately selected so that the contents of these components in a coated film are the above-mentioned contents, respectively.
The coating amount of a film forming agent is from about 1% (w/w) to about 90% (w/w), preferably from about 5% (w/w) to about 50% (w/w), further preferably from about 5% (w/w) to 35% (w/w), based on a core (exclusive of the coating amount of the protective agent).

The solvent in the film forming agent liquid includes water or an organic solvent, alone or in admixture thereof. In the case of use in admixture, the mixing ratio of water to an organic solvent (water/organic solvent: weight ratio) can be varied in the range from 1 to 100%, and preferably from % to about 30%. The organic solvent is not particularly limited as long as it dissolves a water-insoluble substance, and for example, it includes lower alcohols such as methyl alcohol, ethyl alcohol, isopropyl alcohol, n-butyl alcohol, etc., lower alkanones such as acetone, etc., acetonitrile, chloroform, methylene chloride, etc. Among them, lower alcohols are preferable, and ethyl alcohol and isopropyl alcohol are particularly preferable. Water, and a mixture of water with an organic solvent are preferably used as a solvent for a film forming agent. In this case, if necessary, an acid such as tartaric acid, citric acid, succinic acid, fumaric acid, maleic acid, etc. may also be added into a film forming agent liquid for stabilizing the film forming agent liquid.
An operation of coating by spray coating can be conducted by a usual coating method, and specifically, it can be conducted by spray-coating a film forming agent liquid onto a core, for example, by a fluidized bed coating method, pan coating method etc. In this case, if necessary, talc, titanium oxide, magnesium stearate, calcium stearate, light anhydrous silicic acid etc. may also be added as a lubricant, and glycerin fatty ester, hydrogenated castor oil, triethyl citrate, cetyl alcohol, stearyl alcohol etc. may also be added as a plasticizer.
After coating with a film forming agent, if necessary, an antistatic agent such as talc etc. may be mixed.

The rapid release preparation may be liquid (solution, suspension, emulsion etc.) or solid (particle, pill, tablet etc.). It may be oral agents or parenteral agents such as an injection, etc., and preferably, oral agents.
The rapid release preparation, usually, may contain, in addition to an active component drug, also carriers, additives and excipients conventionally used in the field of formulation (hereinafter, sometimes abbreviated as the excipient). The preparation excipient used is not particularly limited as long as it is an excipient ordinarily used as a preparation excipient. For example, the excipient for an oral solid preparation includes lactose, starch, corn starch, crystalline cellulose (Avicel PH101, manufactured by Asahi Kasei Corporation, etc.), powder sugar, granulated sugar, mannitol, light anhydrous silicic acid, magnesium carbonate, calcium carbonate, L-cysteine, etc., and preferably, corn starch and mannitol, etc. These excipients can be used alone or in combination of two or more. The content of the excipient is, for example, from about 4.5 w/w% to about 99.4 w/w%, preferably from about 20 w/w% to about 98.5 w/w%, further preferably from about 30 w/w% to about 97 w/w%, based on the total amount of the rapid release preparation.
The content of a drug in the rapid release preparation can be appropriately selected in the range from about 0.5% to about 95%, preferably from about 1% to about 60% based on the total amount of the rapid release preparation.

When the rapid release preparation is an oral solid preparation, it usually contains a disintegrating agent in addition to the above-mentioned components. The disintegrating agent includes, for example, carboxymethyl cellulose calcium (ECG-505, manufactured by GOTOKU CHEMICAL COMPANY LTD.), croscarmellose sodium (e.g., acjizol, manufactured by Asahi Kasei Corporation), crospovidone (e.g., colidone CL, manufactured by BASF), low-substituted hydroxypropyl cellulose (manufactured by Shin-Etsu Chemical Co., Ltd.), carboxymethylstarch (manufactured by Matsutani Chemical Industry Co., Ltd.), carboxymethylstarch sodium (Exprotab, manufactured by Kimura Sangyo), partially α-starch (PCS, manufactured by Asahi Kasei Corporation), etc., and for example, includes those which disintegrate a granule by absorbing water in contact with water, causing swelling, or making a channel between an effective ingredient constituting the core and an excipient. These disintegrating agents can be used alone or in combinations of two or more. The amount of the disintegrating agent used is appropriately selected depending on the kind and blending amount of a drug used, formulation design for release property, etc., and for example, from about 0.05 w/w% to about 30 w/w%, preferably from about 0.5 w/w% to about 15 w/w%, based on the total amount of the rapid release preparation.

When the rapid release preparation is an oral solid preparation, it may further contain if desired, additives conventional in solid preparations in addition to the above-mentioned composition. Such an additive includes, for example, a binder (e.g., sucrose, gelatin, arabic gum powder, methyl cellulose, hydroxypropyl cellulose, hydroxypropylmethyl cellulose, carboxylmethyl cellulose, polyvinylpyrrolidone, pullulans, dextrin, etc.), a lubricant (e.g., polyethylene glycol, magnesium stearate, talc, light anhydrous silicic acid (e.g., aerosil (Nippon Aerosil)), a surfactant (e.g., anionic surfactants such as sodium alkylsulfate, etc., nonionic surfactants such as polyoxyethylene fatty acid ester and polyoxyethylene sorbitan fatty acid ester, polyoxyethylene castor oil derivatives, etc.), a colorant (e.g., tar coloring matter, caramel, iron oxide red, titanium oxide, riboflavins), if necessary, a corrigent (e.g., sweetening agent, flavor, etc.), an adsorbent, an antiseptic, a wetting agent, an antistatic agent, etc. Further, a stabilizer such as an organic acid such as tartaric acid, citric acid, succinic acid, fumaric acid, etc. may also be added.
The above-mentioned binder includes preferably hydroxypropyl cellulose, polyethylene glycol and polyvinylpyrrolidone, etc.
The rapid release preparation can be prepared by mixing the above-mentioned components, and if necessary, further kneading the mixture, and molding it based on a usual technology of producing preparations. The above-mentioned mixing is conducted by generally used methods, for example, mixing, kneading, etc. Specifically, when a rapid release preparation is formed, for example, into a particle, it can be prepared, according to the same means as in the above-mentioned method for preparing a core of a sustained release preparation, by mixing the components using a vertical granulator, universal kneader (manufactured by Hata Iron Works Co., Ltd.), fluid bed granulator FD-5S (manufactured by Powrex Corporation), etc., then, subjecting the mixture to a wet extrusion granulation method, fluidized bed granulation method, etc.
Thus obtained quick releasing preparation and sustained releasing preparation may be themselves made into products or made into products appropriately together with preparation excipients etc., separately, by an ordinary method, then, may be administered simultaneously or may be administered in combination at any administration interval, or they may be themselves made into one oral preparation (e.g., granule, fine particle, tablet, capsule etc.) or made into one oral preparation together with preparation excipients etc. It may also be permissible that they are made into granules or fine particles, and filled in the same capsule to be used as a preparation for oral administration.

### [3] Sublingual, buccal or intraoral quick disintegrating agent and preparation thereof

Sublingual, buccal or intraoral quick disintegrating agents may be a solid preparation such as tablet etc., or may be an oral mucosa membrane patch (film).
The sublingual, buccal or intraoral quick disintegrating agent is preferably a preparation containing the compound of the present invention or the combination drug and an excipient. It may contain also auxiliary agents such as a lubricant, isotonizing agent, hydrophilic carrier, water-dispersible polymer, stabilizer etc. Further, for easy absorption and increased bioavailability, β-cyclodextrin or β-cyclodextrin derivatives (e.g., hydroxypropyl-β-cyclodextrin etc.), etc. may also be contained.
The above-mentioned excipient includes lactose, sucrose, D-mannitol, starch, crystalline cellulose, light anhydrous silicic acid, etc. The lubricant includes magnesium stearate, calcium stearate, talc, colloidal silica, etc., and particularly preferably, magnesium stearate and colloidal silica. The isotonizing agent includes sodium chloride, glucose, fructose, mannitol, sorbitol, lactose, saccharose, glycerin, urea, etc., and particularly preferably, mannitol. The hydrophilic carrier includes swellable hydrophilic carriers such as crystalline cellulose, ethyl cellulose, crosslinkable polyvinylpyrrolidone, light anhydrous silicic acid, silicic acid, dicalcium phosphate, calcium carbonate etc., and particularly preferably, crystalline cellulose (e.g., fine crystalline cellulose, etc.). The water-dispersible polymer includes gums (e.g., gum tragacanth, acacia gum, cyamoposis gum), alginates (e.g., sodium alginate), cellulose derivatives (e.g., methyl cellulose, carboxymethyl cellulose, hydroxymethyl cellulose, hydroxypropyl cellulose, hydroxypropylmethyl cellulose), gelatin, aqueous starch, polyacrylic acids (e.g., Carbomer), polymethacrylic acid, polyvinyl alcohol, polyethylene glycol, polyvinylpyrrolidone, polycarbophil, ascorbate, palmitates, etc., and preferably, hydroxypropylmethyl cellulose, polyacrylic acid, alginate, gelatin, carboxymethyl cellulose, polyvinylpyrrolidone, polyethylene glycol, etc., particularly preferably, hydroxypropylmethyl cellulose. The stabilizer includes cysteine, thiosorbitol, tartaric acid, citric acid, sodium carbonate, ascorbic acid, glycine, sodium sulfite, etc., and particularly preferably, citric acid and ascorbic acid.

The sublingual, buccal or intraoral quick disintegrating agent can be manufactured by mixing the compound of the present invention or the combination drug and an excipient by a per se known method. Further, if desired, auxiliary agents such as a lubricant, isotonizing agent, hydrophilic carrier, water-dispersible polymer, stabilizer, colorant, sweetening agent, antiseptic etc. may be mixed. The sublingual, buccal or intraoral quick disintegrating agent is obtained by mixing the above-mentioned components simultaneously or at a time interval, then subjecting the mixture to tablet-making molding under pressure. For obtaining suitable hardness, it may also be permissible that the materials are moistened by using a solvent such as water, alcohol etc. if desired before and after the tablet making process, and after the molding, the materials are dried, to obtain a product.

In the case of molding into a mucosa membrane patch (film), the compound of the present invention or the combination drug and the above-mentioned water-dispersible polymer (preferably, hydroxypropyl cellulose, hydroxypropylmethyl cellulose), excipient etc. are dissolved in a solvent such as water etc., and the resulted solution is cast to give a film. Further, additives such as a plasticizer, a stabilizer, an antioxidant, an antiseptic, a colorant, a buffer, a sweetening agent etc. may also be added. For imparting suitable elasticity to the film, glycols such as polyethylene glycol, propylene glycol, etc. may be contained, or for enhancing adhesion of the film to an intraoral mucosa membrane lining, a bio-adhesive polymer (e.g., polycarbophil, carbopol) may also be contained. In the casting, a solution is poured on the non-adhesive surface, spread to uniform thickness (preferably, about 10 micron to about 1,000 micron) by an application tool such as a doctor blade etc., then, the solution is dried to form a film. It may be advantageous that thus formed film is dried at room temperature or under heat, and cut into given area.

The intraoral quick disintegrating preparation is preferably solid quick diffuse preparation composed of a network body comprising the compound of the present invention or the combination drug, and a aqueous or water-diffusible carrier which is inert to the compound of the present invention or the combination drug. This network body is obtained by sublimating a solvent from the solid composition constituted of a solution prepared by dissolving the compound of the present invention or the combination drug in a suitable solvent.
The composition of an intraoral quick disintegrating agent preferably contains a matrix forming agent and a secondary component in addition to the compound of the present invention or the combination drug.
The matrix forming agent includes animal proteins or vegetable proteins such as gelatins, dextrins, soybean, wheat and psyllium seed protein etc.; rubber substances such as arabic gum, guar gum, agar, xanthane gum, etc.; polysaccharides; alginic acids; carboxymethyl celluloses; carrageenans; dextrans; pectins; synthetic polymers such as polyvinylpyrrolidone, etc.; substances derived from a gelatin-arabic gum complex, etc. Further, it includes saccharides such as mannitol, dextrose, lactose, galactose, trehalose, etc.; cyclic saccharides such as cyclodextrin etc.; inorganic salts such as sodium phosphate, sodium chloride and aluminum silicate, etc.; amino acids having 2 to 12 carbon atoms such as glycine, L-alanine, L-aspartic acid, L-glutamic acid, L-hydroxyproline, L-isoleucine, L-leucine, L-phenylalanine, etc.
One or more of the matrix forming agent(s) can be introduced in a solution or suspension before solidification. Such matrix forming agent may be present in addition to a surfactant, or may be present with the surfactant excluded. The matrix forming agents may help to keep the compound of the present invention or the combination drug diffused in the solution or suspension, in addition to formation of the matrix.

The composition may contain secondary components such as a preservative, an antioxidant, a surfactant, a thickening agent, a colorant, a pH controlling agent, a flavoring agent, a sweetening agent, a food taste masking agent, etc. The colorant includes red, black and yellow iron oxides, and FD & C dyes such as FD & C Blue 2, FD & C Red 40, etc. manufactured by Elis and Eberald. Examples of the suitable flavoring agent include mint, raspberry, licorice, orange, lemon, grape fruit, caramel, vanilla, cherry, grape flavor and combinations thereof. Examples of the suitable pH controlling agent include citric acid, tartaric acid, phosphoric acid, hydrochloric acid and maleic acid. Examples of the suitable sweetening agent include aspartame, acesulfame K and thaumatine, etc. Examples of the suitable food taste masking agent include sodium bicarbonate, ion exchange resin, cyclodextrin-inclusion compounds, adsorbent substances and microcapsulated apomorphine.
The preparation contains the compound of the present invention or the combination drug in an amount usually from about 0.1% by weight to about 50% by weight, preferably from about 0.1% by weight to about 30% by weight, and is preferably a preparation (such as the above-mentioned sublingual agent, buccal etc.) which can dissolve 90% or more the compound of the present invention or the combination drug (into water) within the time range of about 1 minute to about 60 minutes, preferably of about 1 minute to 15 minutes, more preferably of about 2 minutes to about 5 minutes, and intraoral quick disintegrating preparations which are disintegrated within the range of 1 second to 60 seconds, preferably of 1 to 30 seconds, further preferably of 1 to 10 seconds after being placed in the oral cavity.

The content of the above-mentioned excipient in the whole preparation is from about 10% by weight to about 99% by weight, preferably from about 30% by weight to about 90% by weight. The content of β-cyclodextrin or β-cyclodextrin derivative in the whole preparation is from 0 to about 30% by weight. The content of the lubricant in the whole preparation is from about 0.01% by weight to about 10% by weight, preferably from about 1% by weight to about 5% by weight. The content of the isotonizing agent in the whole preparation is from about 0.1% by weight to about 90% by weight, preferably, from about 10% by weight to about 70% by weight. The content of the hydrophilic carrier agent in the whole preparation is from about 0.1% by weight to about 50% by weight, preferably, from about 10% by weight to about 30% by weight. The content of the water-dispersible polymer in the whole preparation is from about 0.1 to about 30% by weight, preferably, from about 10% by weight to about 25% by weight. The content of the stabilizer in the whole preparation is from about 0.1% by weight to about 10% by weight, preferably, from about 1% by weight to about 5% by weight. The above-mentioned preparation may further contain additives such as a colorant, a sweetening agent, an antiseptic, etc., if necessary.

The dose of a combination preparation of the present invention differs depending on the kind of the compound (I) of the present invention, age, body weight, condition, drug form, administration method, administration period etc., and for example, for a prostate cancer patient (adult, body weight: about 60 kg), the combination preparation is administered intravenously, at a dose of about 0.01 to about 1,000 mg/kg/day, preferably about 0.01 to about 100 mg/kg/day, more preferably about 0.1 to about 100 mg/kg/day, particularly about 0.1 to about 50 mg/kg/day, especially about 1.5 to about 30 mg/kg/day, in terms of the compound of the present invention or the combination drug, respectively, once or several times a day in divided portions. Of course, since the dose as described above varies depending on various conditions, it may be sometimes sufficient to administer smaller amounts than the above-mentioned dosage, and further, it may be sometimes necessary to administer greater amounts than that.
The amount of the combination drug can be set at any value unless side effects are problematical. The daily dosage in terms of the combination drug differs depending on the severity of symptoms, age, sex, body weight, sensitivity difference of the subject, administration time and interval, property, prescription, and kind of the pharmaceutical preparation, kind of effective ingredient, etc., and not particularly limited; for example, in the case of oral administration, the dose of the drug is usually from about 0.001 mg to 2,000 mg, preferably from about 0.01 mg to 500 mg, further preferably from about 0.1 mg to 100 mg, per 1 kg body weight of a mammal, which is usually administered once to four times a day in divided portions.

In administration of the combination preparation, the compound of the present invention may be administered after administration of the combination drug or the combination drug may be administered after administration of the compound of the present invention, though they may be administered simultaneously. When administered at a time interval, the interval differs depending on the effective ingredient, drug form and administration method. For example, when the combination drug is administered first, the compound of the present invention is administered within time range of from 1 minute to 3 days, preferably from 10 minutes to 1 day, more preferably from 15 minutes to 1 hour after administration of the combined drug. When the compound of the present invention is administered first, the combined drug is administered within time range of from 1 minute to 1 day, preferably from 10 minutes to 6 hours, more preferably from 15 minutes to 1 hour after administration of the compound of the present invention.
In a preferable administration method, for example, the combination drug formulated into an oral administration preparation is administered orally at a daily dose of about 0.001 mg/kg to 200 mg/kg, and 15 minutes later, the compound of the present invention formulated into an oral administration preparation is administered orally at a daily dose of about 0.005 mg/kg to 100 mg/kg.

In addition, the pharmaceutical composition of the present invention or the combination preparation of the present invention can be combined with a non-drug therapy such as (1) surgery, (2) hypertensive chemotherapy using angiotensin II etc., (3) gene therapy, (4) thermotherapy, (5) cryotherapy, (6) laser cauterization, (7) radiotherapy, etc.
For example, the pharmaceutical composition of the present invention or the combination preparation of the present invention exhibits effects of inhibiting an expression of resistance, extending disease-free survival, suppressing cancer metastasis or recurrence, prolonging survival, etc. when used before or after surgery, etc., or a combination treatment comprising 2 or 3 of these therapies.
Also, treatment with the pharmaceutical composition of the present invention or the combination preparation of the present invention can be combined with supportive therapies [e.g., (i) administration of antibiotics (e.g., β-lactams such as pansporin, etc., macrolides such as clarithromycin, etc.) to a combined expression of various infectious diseases, (ii) administration of intravenous hyperalimentations, amino acid preparations and general vitamin preparations for improvement of malnutrition, (iii) morphine administration for pain mitigation, (iv) administration of drugs which mitigate adverse reactions such as nausea, vomiting, anorexia, diarrhea, leukopenia, thrombocytopenia, hemoglobin concentration reduction, hair loss, hepatopathy, renopathy, DIC, fever, etc., (v) administration of drugs for inhibition of multiple drug resistance in cancer, etc.].
As a drug for such purpose, for example, the "antiemetic agents" includes specifically 5-HT3 antagonist such as ondansetron, tropisetron hydrochloride, azasetron, ramosetron, granisetron, dorasetronmesilate and palonosetron; NK1 receptor antagonist such as sendide, CP-99994, CP-100263, CP-122721-1, CP-96345, FK224, RPR100893, NKP608 and aprepitant (EMEND (trademark)); a gastrointestinal tract motility promoter such as 5-HT₄ antagonist such as domperidone, mosapride and metoclopramide; a gastrointestinal tract motility regulator such as trimebutine; phenothiazine drugs such as prochlorperazine maleate, promethazine and thiethylperazine; anxiolytics such as haloperidole, phenol phthalate chlorpromazine, diazepam and droperidole; steroids such as dexamethasone, prednisolone, betamethasone, triamcinolone, etc.; other drugs such as dimethylhydric acid, diphenhydramine, hyoscin, hyoscin bromide, tetrabenazine, etc.
Preferably, the pharmaceutical composition of the present invention or the combination preparation of the present invention is administered orally (including sustained-release preparations), intravenously (including boluses, infusions and clathrates), subcutaneously and intramuscularly (including boluses, infusions and sustained-release preparations), transdermally, intratumorally or proximally before or after conducting the above-described treatment.
As a period for administering the pharmaceutical composition of the present invention or the combination preparation of the present invention before surgery, etc., for example, it can be administrated once about 30 minutes to 24 hours before surgery, etc., or in 1 to 3 cycles about 3 months to 6 months before surgery, etc. In this way, surgery, etc. can be conducted easily because, for example, cancer tissue would be reduced by administering the pharmaceutical composition of the present invention or the combination preparation of the present invention before surgery, etc.
For administering time of the pharmaceutical composition of the present invention or the combination preparation of the present invention after surgery, etc., for example, it can be administrated repeatedly in a unit of a few weeks to 3 months, about 30 minutes to 24 hours after surgery, etc. In this way, it increases the effect of the surgery, etc. by administering the pharmaceutical composition of the present invention or the combination preparation of the present invention after the surgery, etc.

### Examples

The present invention is described in detail in the following by means of the following Reference Examples, Experimental Examples and Formulation Examples, which are not to be construed as limitative.

### Reference Example 1

### (2S,3S)-3-(1-hydroxy-1-methylethyl)-2-methylpyrrolidine 1/2 oxalic acid salt

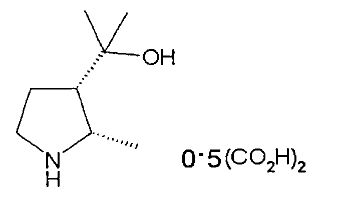

A mixture of 2-[(2S,3S)-2-methyl-1-[(1S)-1-phenylethyl]pyrrolidin-3-yl]propan-2-ol (2.00 g) synthesized by a known method, 10% palladium-carbon (50% water-containing product, 172 mg) and methanol (30 ml) was stirred at room temperature for 60 hr under hydrogen atmosphere. The catalyst was removed by filtration through celite, and the mother liquor was concentrated. The obtained oily substance was dissolved in methanol, oxalic acid dehydrate (510 mg) was added, and the mixture was concentrated. The residue was crystallized from diethyl ether to give the object product (1.47 g, 1/2 hydrate). mp 120-122°C
¹H-NMR (DMSO-d₆) δ : 1.128 (3H, s), 1.129 (3H, d), 1.17 (3H, s), 1.74-2.08 (3H, m), 3.01-3.19 (2H, m), 3.56-3.65 (1H, m).

### Reference Example 2

### 4-[(2S,3S)-3-(1-hydroxy-1-methylethyl)-2-methylpyrrolidin-1-yl]-1-benzothiophene-7-carbonitrile

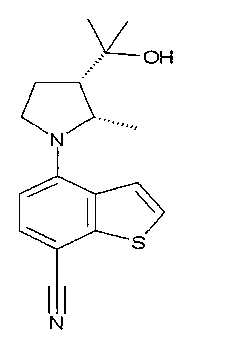

A mixture of 4-fluoro-1-benzothiophene-7-carbonitrile (257 mg), (2S,3S)-3-(1-hydroxy-1-methylethyl)-2-methylpyrrolidine 1/2 oxalic acid salt (300 mg), potassium carbonate (601 mg) and dimethyl sulfoxide (7.0 ml) was stirred at 90°C for 1 hr. The reaction mixture was cooled, and partitioned between water and ethyl acetate. The organic layer was washed with water and dried (sodium sulfate). The residue obtained by concentration was purified by silica gel column chromatography (ethyl acetate-hexane), and crystallized from ethyl acetate to give the object product (228 mg) as colorless crystals. mp 146-147°C
¹H-NMR (CDCl₃) δ: 1.27 (1H, s), 1.30 (3H, d), 1.36 (3H, s), 1.42 (3H, s), 2.11-2.32 (3H, m), 3.66-3.75 (1H, m), 3.84-3.90 (1H, m), 4.47-4.55 (1H, m), 6.41 (1H, d), 7.30 (1H, d), 7.46 (1H, d), 7.71 (1H, d).

### Experimental Example 1 Measurement of organ selective androgen receptor regulating action

A 3-week-old Sprague-Dawley male rat was castrated, and testosterone·propionate (0.5 mg/kg) was administered subcutaneously once a day from the next day. Simultaneously, a test compound was dissolved in 10% ethanol/corn oil, and orally administered twice a day. The next day of a 1 week administration, the weights of the levator ani muscle, prostate and vesicular gland were measured. The dose of the test compound is indicated in a daily amount.
As the test compounds, compound 114, compound 158, compound 117, compound 119 and compound of Reference Example 2 were used. The results are shown in Table 13.

**Table 13**

| compound | concentration (mg/kg/day) | relative % to control | |
|---|---|---|---|
| | | prostate weight | levator ani muscle weight |
| compound 114 | 4.143 | 90.6 | 142.1 |
| | 13.81 | 99.1 | 157.7 |
| compound 158 | 3 | 98.1 | 125.3 |
| | 10 | 89.8 | 171.1 |
| compound 117 | 1.389 | 92.3 | 131.8 |
| | 4.167 | 96.0 | 154.0 |
| compound 119 | 0.4138 | 87.8 | 131.5 |
| | 1.379 | 91.4 | 151.8 |
| Reference Example 2 compound | 3 | 92.0 | 108.0 |
| | 10 | 87.5 | 120.1 |

From the above results, it has been found that the compound of the present invention does not increase the prostate weight with a dose that increases the levator ani muscle weight by 20%.

### Formulation Example 1: Microcapsules containing leuprorelin acetate

5.8 g of leuprorelin acetate is dissolved in 6.7 ml of distilled water. To this is added 138 g of dichloromethane solution containing polylactic acid (weight average molecular weight: 15000) (51.6 g) which has been separately dissolved and filtered, and the mixture is stirred and emulsified with an auto-mini mixer for 9 minutes (rotation number: about 6000 rpm), and adjusted to 15°C. This mixture is added to 13.5 L of aqueous solution of 0.1% polyvinyl alcohol (PVA) which has been previously dissolved, filtered and adjusted to the same temperature, to emulsify it. For emulsification, HOMOMIC LINE FLOW (Tokushu Kika Kogyo Co., Ltd.) is used, and the rotation number of the mixer is about 7,000 rpm. Solvent is removed from this W/O/W emulsion with light stirring for about 3 hours (drying method in water).
The obtained microcapsules are put through a sieve of 74 µm to remove coarse particles, and separated by filtration or centrifugation. Those are washed with distilled water, free drug and PVA are removed, and re-dispersed with small amount of water. 8.7 g of D-mannitol is dissolved therein, and the mixture is sieved and lyophilized. The rack temperature is gradually elevated in the drying process, and the microcapsules are dried finally at 52°C for 69 hours. The microcapsules are sieved and crushed to give microcapsule powders. From this process, 58 g of microcapsule powders containing 15% D-mannitol is obtained.

### Formulation Example 2: Injection containing compound of compound 114

| | | |
|---|---|---|
| (1) | compound 114 | 5.0 mg |
| (2) | sodium chloride | 20.0 mg |
| (3) | distilled water | amount to make total amount 2 ml |

Compound 114 (5.0 mg) and sodium chloride (20.0 mg) are dissolved in distilled water, and water is added thereto to make the total volume 2 ml. The solution is filtered, and filled into a 2 ml ampoule under sterile conditions. The ampoule is sterilized and sealed to give an injectable solution.

### Formulation Example 3: Injection containing compound of compound 158

| | | |
|---|---|---|
| (1) | compound 158 | 5.0 mg |
| (2) | sodium chloride | 20.0 mg |
| (3) | distilled water | amount to make total amount 2 ml |

Compound 158 (5.0 mg) and sodium chloride (20.0 mg) are dissolved in distilled water, and water is added thereto to make the total volume 2 ml. The solution is filtered, and filled into a 2 ml ampoule under sterile conditions. The ampoule is sterilized and sealed to give an injectable solution.

### Formulation Example 4: Injection containing compound of compound 117

| | | |
|---|---|---|
| (1) | compound 117 | 5.0 mg |
| (2) | sodium chloride | 20.0 mg |
| (3) | distilled water | amount to make total amount 2 ml |

Compound 117 (5.0 mg) and sodium chloride (20.0 mg are dissolved in distilled water, and water is added thereto to make the total volume 2 ml. The solution is filtered, and filled into a 2 ml ampoule under sterile conditions. The ampoule is sterilized and sealed to give an injectable solution.

### Formulation Example 5: Injection containing compound of compound 119

| | | |
|---|---|---|
| (1) | compound 119 | 5.0 mg |
| (2) | sodium chloride | 20.0 mg |
| (3) | distilled water | amount to make total amount 2 ml |

Compound 119 (5.0 mg) and sodium chloride (20.0 mg) are dissolved in distilled water, water is added thereto to make the total volume 2 ml. The solution is filtered, and filled into a 2 ml ampoule under sterile conditions. The ampoule is sterilized and sealed to give an injectable solution.

### Formulation Example 6: Injection containing compound of Reference Example 2

| | | |
|---|---|---|
| (1) | Reference Example 2 compound | 5.0 mg |
| (2) | sodium chloride | 20.0 mg |
| (3) | distilled water | amount to make total amount 2 ml |

The compound (5.0 mg) of Reference Example 2 and sodium chloride (20.0 mg) are dissolved in distilled water, water is added thereto to make the total volume 2 ml. The solution is filtered, and filled into a 2 ml ampoule under sterile conditions. The ampoule is sterilized and sealed to give an injectable solution.

### Formulation Example 7: Tablet containing testosterone

| | | |
|---|---|---|
| (1) | Testosterone·propionate | 50 mg |
| (2) | Lactose | 34 mg |
| (3) | Corn starch | 10.6 mg |
| (4) | Corn starch (in paste form) | 5 mg |
| (5) | Magnesium stearate | 0.4 mg |
| (6) | Carboxymethyl cellulose calcium | 20 mg |
| | Total | 120 mg |

In accordance with conventional methods, the above (1) to (6) are mixed and tabletted using a tabletting machine to give a tablet.

### Formulation Example 8

The preparation obtained in Formulation Example 1 and the preparation obtained in Formulation Example 2 are combined.

### Formulation Example 9

The preparation obtained in Formulation Example 1 and the preparation obtained in Formulation Example 3 are combined.

### Formulation Example 10

The preparation obtained in Formulation Example 1 and the preparation obtained in Formulation Example 4 are combined.

### Formulation Example 11

The preparation obtained in Formulation Example 1 and the preparation obtained in Formulation Example 5 are combined.

### Formulation Example 12

The preparation obtained in Formulation Example 1 and the preparation obtained in Formulation Example 6 are combined.

### Formulation Example 13

The preparation obtained in Formulation Example 1, the preparation obtained in Formulation Example 2 and the preparation obtained in Formulation Example 7 are combined.

### Formulation Example 14

The preparation obtained in Formulation Example 1, the preparation obtained in Formulation Example 3 and the preparation obtained in Formulation Example 7 are combined.

### Formulation Example 15

The preparation obtained in Formulation Example 1, the preparation obtained in Formulation Example 4 and the preparation obtained in Formulation Example 7 are combined.

### Formulation Example 16

The preparation obtained in Formulation Example 1, the preparation obtained in Formulation Example 5 and the preparation obtained in Formulation Example 7 are combined.

### Formulation Example 17

The preparation obtained in Formulation Example 1, the preparation obtained in Formulation Example 6 and the preparation obtained in Formulation Example 7 are combined.

### Industrial Applicability

The compound of the present invention has a superior action as an organ selective androgen receptor modulator (particularly agonist), and is useful as a frailty suppressant, a muscular strength enhancer, a muscle increasing agent, a cachexia suppressant, a body weight decrease suppressant, an agent for the prophylaxis or treatment of prostatomegaly, an agent for the prophylaxis or treatment of amyotrophy, an agent for the prophylaxis or treatment of sarcopenia caused by disease, an agent for the prophylaxis or treatment of hypertriglyceridemia (hyperlipidemia), a cholesterol-lowering agent, an agent for the prophylaxis or treatment of metabolic syndrome, a prostate weight-reducing agent and the like.

This application is based on a patent application No. 2006-043141 filed in Japan, the contents of which are incorporated in full herein by this reference.
The present invention presents or describes the preferable embodiments thereof. On the other hand, in the present specification, those of ordinary skill in the art would understand that various changes in embodiments or detail can occur without deviating from the range encompassed in the attached Claims of the invention. All patents, patent publications and other publications indicated or referred to in the present specification are incorporated in full by reference.

## Claims

1. A tissue-selective androgen receptor modulator comprising a compound represented by the formula 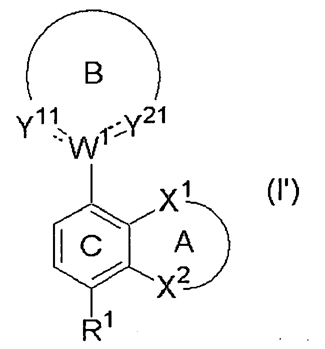 wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), W¹ represents a nitrogen atom, or a group represented by the formula CR^{a} (wherein R^{a} represents a bond, a hydrogen atom, a hydroxy group or an optionally substituted alkoxy group), Y¹¹ represents a group represented by the formula CR²R^{3'} (wherein R² represents a hydrogen atom, a cyano group, a nitro group, an optionally substituted acyl group, an optionally esterified or amidated carboxyl group or an optionally substituted hydrocarbon group, and R^{3'} represents a bond, a hydrogen atom, a cyano group, a nitro group, an optionally substituted acyl group, an optionally esterified or amidated carboxyl group or an optionally substituted hydrocarbon group), and Y²¹ represents a group represented by the formula CR⁴R^{5'} (wherein R⁴ represents a hydrogen atom, a cyano group, a nitro group, an optionally substituted acyl group, an optionally esterified or amidated carboxyl group or an optionally substituted hydrocarbon group, and R^{5'} represents a bond, a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and when Ring B is a bicyclic ring optionally further substituted, CR² for Y¹¹ or CR⁴ or the nitrogen atom for Y²¹ may constitute a part of Ring B, R¹ represents an electron-withdrawing group, and the formula
-̅ -̅ -̅
represents a single bond or a double bond, or a salt thereof, or a prodrug thereof.

2. The modulator of claim 1, which increases prostate weight by not more than about 10% with a dose that increases levator ani muscle weight by about 20%.

3. The modulator of claim 1, which is a frailty suppressant, a muscular strength enhancer, a muscle increasing agent, a cachexia suppressant, a body weight decrease suppressant, an agent for the prophylaxis or treatment of prostate hypertrophy, an agent for the prophylaxis or treatment of amyotrophy, an agent for the prophylaxis or treatment of sarcopenia caused by a disease, an agent for reducing prostate weight, an agent for the prophylaxis or treatment of hypertriglyceridemia (hyperlipidemia), a cholesterol-lowering agent or an agent for the prophylaxis or treatment of metabolic syndrome.

4. A method of tissue-selective modulation of androgen receptor, which comprises administering, to a mammal, an effective amount of a compound represented by the formula 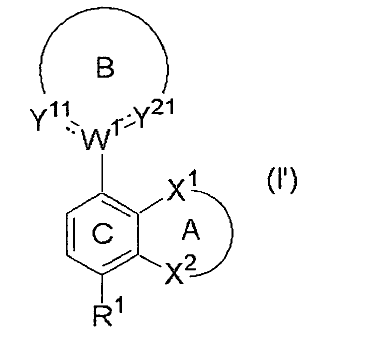 wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), W¹ represents a nitrogen atom, or a group represented by the formula CR^{a} (wherein R^{a} represents a bond, a hydrogen atom, a hydroxy group or an optionally substituted alkoxy group), Y¹¹ represents a group represented by the formula CR²R^{3'} (wherein R² represents a hydrogen atom, a cyano group, a nitro group, an optionally substituted acyl group, an optionally esterified or amidated carboxyl group or an optionally substituted hydrocarbon group, and R^{3'} represents a bond, a hydrogen atom, a cyano group, a nitro group, an optionally substituted acyl group, an optionally esterified or amidated carboxyl group or an optionally substituted hydrocarbon group), and Y²¹ represents a group represented by the formula CR⁴R^{5'} (wherein R⁴ represents a hydrogen atom, a cyano group, a nitro group, an optionally substituted acyl group, an optionally esterified or amidated carboxyl group or an optionally substituted hydrocarbon group, and R^{5'} represents a bond, a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and when Ring B is a bicyclic ring optionally further substituted, CR² for Y¹¹ or CR⁴ or the nitrogen atom for Y²¹ may constitute a part of Ring B, R¹ represents an electron-withdrawing group, and the formula
-̅ -̅ -̅
represents a single bond or a double bond, or a salt thereof, or a prodrug thereof.

5. Use of a compound represented by the formula 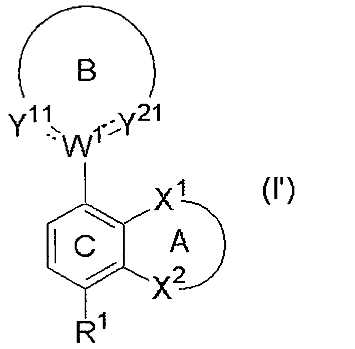 wherein Ring A represents an optionally substituted 5- to 8-membered ring, Ring B represents a 4- to 10-membered ring optionally further substituted, Ring C represents a benzene ring optionally further substituted, X¹ represents an optionally substituted carbon atom, and X² represents an optionally substituted carbon atom, an oxygen atom or a group represented by the formula S(O)ₖ (wherein k represents 0, 1 or 2), W¹ represents a nitrogen atom, or a group represented by the formula CR^{a} (wherein R^{a} represents a bond, a hydrogen atom, a hydroxy group or an optionally substituted alkoxy group), Y¹¹ represents a group represented by the formula CR²R^{3'} (wherein R² represents a hydrogen atom, a cyano group, a nitro group, an optionally substituted acyl group, an optionally esterified or amidated carboxyl group or an optionally substituted hydrocarbon group, and R^{3'} represents a bond, a hydrogen atom, a cyano group, a nitro group, an optionally substituted acyl group, an optionally esterified or amidated carboxyl group or an optionally substituted hydrocarbon group), and Y²¹ represents a group represented by the formula CR⁴R^{5'} (wherein R⁴ represents a hydrogen atom, a cyano group, a nitro group, an optionally substituted acyl group, an optionally esterified or amidated carboxyl group or an optionally substituted hydrocarbon group, and R^{5'} represents a bond, a hydrogen atom, a cyano group, a nitro group, an optionally substituted acyl group, an optionally esterified or amidated carboxyl group or an optionally substituted hydrocarbon group), an optionally substituted nitrogen atom, an oxygen atom or a group represented by the formula S(O)ₘ (wherein m represents 0, 1 or 2), and when Ring B is a bicyclic ring optionally further substituted, CR² for Y¹¹ or CR⁴ or the nitrogen atom for Y²¹ may constitute a part of Ring B, R¹ represents an electron-withdrawing group, the formula
-̅ -̅ -̅
represents a single bond or a double bond, or a salt thereof, or a prodrug thereof for production of a tissue-selective androgen receptor modulator.
